(19) Europäisches Patentamt
European Patent Office
Office européen des brevets

(11) EP 2 935 296 B1

(12) **EUROPEAN PATENT SPECIFICATION**

(45) Date of publication and mention of the grant of the patent:
**15.11.2017 Bulletin 2017/46**

(21) Application number: **13811231.3**

(22) Date of filing: **19.12.2013**

(51) Int Cl.:
***C07F 17/00*** *(2006.01)* ***C07F 7/08*** *(2006.01)*
***C08F 10/06*** *(2006.01)* ***C08F 4/6592*** *(2006.01)*

(86) International application number:
**PCT/EP2013/077531**

(87) International publication number:
**WO 2014/096282 (26.06.2014 Gazette 2014/26)**

(54) **CATALYST**

KATALYSATOR

CATALYSEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2012 EP 12199251**

(43) Date of publication of application:
**28.10.2015 Bulletin 2015/44**

(60) Divisional application:
**17195336.7**

(73) Proprietor: **Borealis AG**
**1220 Vienna (AT)**

(72) Inventors:
- **RESCONI, Luigi**
  **4501 Neuhofen an der Krems (AT)**
- **VIRKKUNEN, Ville**
  **FI-00790 Helsinki (FI)**
- **AJELLAL, Noureddine**
  **00970 Helsinki (FI)**
- **CASTRO, Pascal**
  **FI-00150 Helsinki (FI)**
- **IZMER, Vyatcheslav**
  **Moscow 117186 (RU)**
- **KONONOVICH, Dmitry**
  **Moscow 119234 (RU)**
- **VOSKOBOYNIKOV, Alexander**
  **Moscow 129515 (RU)**

(74) Representative: **Campbell, Neil Boyd**
**Dehns**
**St Bride's House**
**10 Salisbury Square**
**London**
**EC4Y 8JD (GB)**

(56) References cited:
**WO-A1-2007/107448 US-A1- 2007 135 596**

- **ILYA E. NIFANT'EV ET AL: "5-Methoxy-Substituted Zirconium Bis-indenyl ansa -Complexes: Synthesis, Structure, and Catalytic Activity in the Polymerization and Copolymerization of Alkenes", ORGANOMETALLICS, vol. 31, no. 14, 23 July 2012 (2012-07-23) , pages 4962-4970, XP055060553, ISSN: 0276-7333, DOI: 10.1021/om300160v**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 935 296 B1

## Description

### Field of invention

This invention relates to new bisindenyl catalysts, in particular, solid particulate racemic symmetrical metallocene catalysts containing such bisindenyl ligands. The invention also relates to the use of such new bisindenyl metallocene catalysts for the production of polypropylene at excellent catalyst activities to give polypropylene with high molecular weight, and high melting point even at industrially relevant polymerization temperatures.

### Background of Invention

Metallocene catalysts have been used to manufacture polyolefins for many years. Countless academic and patent publications describe the use of these catalysts in olefin polymerisation. Metallocenes are now used industrially and polyethylenes and polypropylenes in particular are often produced using cyclopentadienyl based catalyst systems with different substitution patterns.

The two most important physical properties of isotactic polypropylene (iPP) are its average molecular weight and its melting point (Tm), the latter being mostly determined by the degree of stereoregularity (isotacticity) of the polypropylene chains.

The Ziegler-Natta catalyst systems known in the literature can produce iPP with high molecular weights together with moderate to high isotacticities and melting temperatures (Tm). The Tm (measured by standard DSC methods) of non-nucleated iPPs are in the range of 160 to 165 °C.

In the case of metallocenes, there are very few examples which can produce iPP having both very high molecular weights and high melting points. For example rac-Et(2,4,7-$Me_3$Ind)$_2$Zr$Cl_2$ can produce isotactic polypropylene with a molecular weight of 1,900,000 g/mol and a melting point of 168 °C (Deng Macromolecules, 1996, 29, 6371).

In order to achieve such high values, a polymerization temperature of -30 °C is necessary. When the polymerization temperature is increased to 30 °C, the melting point of the resulting polypropylene decreases to 158 °C. A polymerization temperature of -30 °C is however, far too low for polypropylene manufacturing in commercial plants, which need to be operated above 60 °C. When used at industrially useful polymerization temperatures, this same metallocene yields low molecular weight polypropylenes with relatively low melting point. For example at 70 °C, rac-Et(2,4,7-$Me_3$Ind)$_2$Zr$Cl_2$ /MAO yields a polypropylene of molecular weight of only 30,600 with a melting point of only 145 °C (EP537686).

Higher melting point polymers can be made using dimethylsilyl bis (2-methyl-4-(1-naphthyl)indenyl Zr$Cl_2$ (Spaleck Organometallics, 1994, 13, 954). EP-A-1070729 exemplifies a variety of 2,4-disubstituted metallocenes although melting points are not discussed.

A melting point of 162 °C and of 165 °C have also been reported for the simple $Me_2$Si(2-Me-4-Ph-Indenyl)$_2$ metallocenes, (J. Ewen et al. Macromol. Rapid Commun. 1998, 19, 71).

The metallocenes above have been used in non-supported form however, i.e. typically in solution polymerisation. When used in solid form and in particular when used on a catalyst support such as silica, the melting point of the polymer reduces.

Some supported metallocenes have however been found to give high melting points. In US7,405,261, rac-Et[2,7-$Me_2$-4-(4-tBuPh)Ind]$_2$Zr$Cl_2$ is reported to produce iPP with a melting point of 156 °C, by polymerizing liquid propylene at 65 °C.

WO2009/054831 describes zirconocenes with a 2-methyl-4,7-aryl substitution pattern, such as rac-$Me_2$Si[2-Me-4,7-(4-tBuPh)$_2$Ind]$_2$Zrd$_2$. The melting points of the homopolymers are still quite low, being in all cases below 150 °C despite the relatively low polymerization temperature of 65 °C.

The present applicant has developed an alternative to conventional inorganic supports. In WO03/051934, the inventors proposed an alternative form of catalyst which is provided in solid form but does not require a conventional external carrier material such as silica. The invention is based on the finding that a homogeneous catalyst system containing an organometallic compound of a transition metal can be converted, in a controlled way, to solid, uniform catalyst particles by first forming a liquid/liquid emulsion system, which comprises as the dispersed phase, said solution of the homogeneous catalyst system, and as the continuous phase a solvent immiscible therewith, and then solidifying said dispersed droplets to form solid particles comprising the said catalyst.

The invention described in WO03/051934 enabled the formation of solid spherical catalyst particles of said organotransition metal catalyst without using e.g. external porous carrier particles, such as silica, normally required in the art. Thus, problems relating to catalyst silica residues can be solved by this type of catalyst. Further, it could be seen that catalyst particles having improved morphology, will give, due to the replica effect, polymer particles having improved morphology as well.

Some supported metallocene or metallocenes in solid form made using the techniques of WO03/051934 are

also known. Metallocenes with a 3,5-di-tert-butylphenyl substituent on the 4-position of indene are known. WO02/02576 describes conventionally supported metallocenes such as rac-$Me_2Si[2$-Me-4-(3,5-$tBu_2Ph)Ind]_2Zrd_2$. These metallocene catalysts, activated with MAO or a borate, on a silica support, at a polymerisation temperature of 60 or 70 °C, give iPP with Tm between 156 and 159 °C.

The metallocene rac-9-silafluorenyl-9,9-[2-Me-4-(3,5-$tBu_2Ph)Ind]_2ZrCl_2$ also gives high melting point iPP and are described in WO02/02575.

In EP2532687, a catalyst based on rac-Me2Si[2-Me-4-(3,5-$tBu_2$Ph)-7-OMe-$Ind]_2ZrCl_2$/MAO is described with relatively low activity.

In general however, metallocene catalysts, when used under industrially relevant polymerization conditions, produce iPP having melting points which are lower than the melting points of Ziegler Natta iPP, and even the best metallocene catalysts produce iPP with melting points of less than 160 °C. In addition, few metallocene catalysts can produce iPP having both high melting point and high molecular weight at polymerisation temperatures above 60 °C with good catalyst activity.

In order to overcome this inherent limitation of metallocene catalysts, and in order to produce polypropylenes having both high melting points and high molecular weights with good activity, we have developed a new family of catalysts comprising substituted bis-indenyl complexes.

We preferably employ these complexes in solid particulate, yet unsupported, form to make a new family of catalysts with interesting properties. These metallocenes have been found to produce isotactic polypropylenes with surprisingly high melting points and very high molecular weights at good catalyst activities.

The catalysts of the invention comprise a bridged bisindenyl metallocene complex with an optionally substituted aryl group at the 4-position of an indenyl ligand, a non hydrogen substituent at the 2-position of the ring and a substituent at the 6-position of the ring. The 3, 5 and 7 positions are unsubstituted.

One of the limitations of metallocene catalysts for polypropylene is the relative low melting of the homopolymer. Several families of bridged bisindenyl metallocene catalysts with the proper substitution pattern have been developed to produce high molecular weight, highly isotactic polypropylene. However, often the increased melting point is obtained at the expense of activity, or catalyst cost, or both.

The present inventors sought a new catalyst system capable of producing, *inter alia,* isotactic polypropylene with high melting points, e.g. 152 °C or more, high isotacticity and high molecular weights without compromising catalyst activity at commercially relevant temperatures. In particular, the catalysts claimed herein offer high activities and high molecular weight at high melting points.

**Summary of Invention**

Thus, viewed from one aspect the invention provides a catalyst in solid particulate form free from an external carrier material comprising

(i) a complex of formula (I)

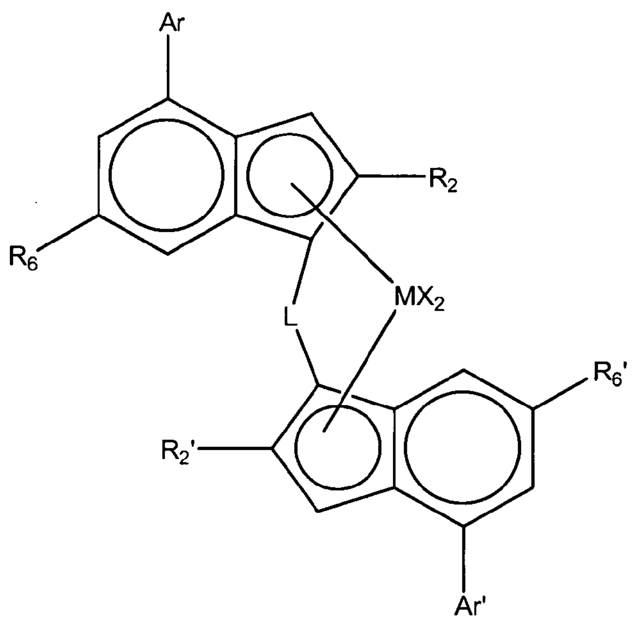

(I)

wherein

M is zirconium or hafnium;
each X is a sigma ligand;
L is a divalent bridge selected from $-R'_2C-$, $-R'_2C-CR'_2-$, $-R'_2Si-$, $-R'_2Si-SiR'_2-$, $-R'_2Ge-$, wherein each R' is independently a hydrogen atom, $C_{1-20}$-alkyl, tri($C_{1-20}$-alkyl)silyl, $C_{6-20}$-aryl, $C_{7-20}$-arylalkyl or $C_{7-20}$-alkylaryl;
$R_2$ is a $C_{1-20}$-hydrocarbyl radical;
$R_{2'}$ is a $C_{1-20}$-hydrocarbyl radical;
$R_6$ is a linear or branched aliphatic $C_{1-20}$-hydrocarbyl group, $SR_9$ or $OR_9$;
$R_{6'}$ is a linear or branched aliphatic $C_{1-20}$-hydrocarbyl group, $SR_{9'}$ or $OR_{9'}$;
with the proviso that, preferably, neither $R_6$ or $R_{6'}$ represents a group having a quaternary carbon atom directly attached to the indenyl ring;
$R_9$ is a $C_{1-20}$-hydrocarbyl group;
$R_{9'}$ is a $C_{1-20}$-hydrocarbyl group;
Ar is a $C_{6-12}$-aryl or $C_{5-12}$-heteroaryl group optionally carrying one or more substituents $R_8$;
Ar' is a $C_{6-12}$-aryl or $C_{5-12}$-heteroaryl group optionally carrying one or more substituents $R_{8'}$;
each $R_8$ is a $C_{1-20}$-hydrocarbyl group;
each $R_{8'}$ is a $C_{1-20}$-hydrocarbyl group;
wherein at least two of $R_2$ and $R_{2'}$; $R_6$ and $R_{6'}$; or Ar and Ar' are the same;
and (ii) a cocatalyst comprising a compound of a group 13 metal, e.g. Al or boron.

Ideally, the catalyst is obtainable by a process in which

(a) a liquid/liquid emulsion system is formed, said liquid/liquid emulsion system comprising a solution of the catalyst components (i) and (ii) dispersed in a solvent so as to form dispersed droplets; and
(b) solid particles are formed by solidifying said dispersed droplets.

Viewed from another aspect the invention provides a process for the manufacture of a catalyst as hereinbefore defined comprising obtaining a complex of formula (I) and a cocatalyst as hereinbefore described; forming a liquid/liquid emulsion system, which comprises a solution of catalyst components (i) and (ii) dispersed in a solvent, and solidifying said dispersed droplets to form solid particles.

Viewed from another aspect the invention provides the use in olefin polymerisation of a catalyst as hereinbefore defined.

Viewed from another aspect the invention provides a process for the polymerisation of at least one olefin, in particular propylene, comprising polymerising said at least one olefin with a catalyst as hereinbefore described.

Viewed from another aspect the invention provides a process for producing an isotactic polypropylene with a melting point of at least 152 °C, ideally at least 155 °C and at a catalyst activity of at least 10.0 kg/g(cat)/h comprising polymerising propylene in the presence of the catalyst as hereinbefore defined.

Viewed from another aspect the invention provides a ligand of formula (I')

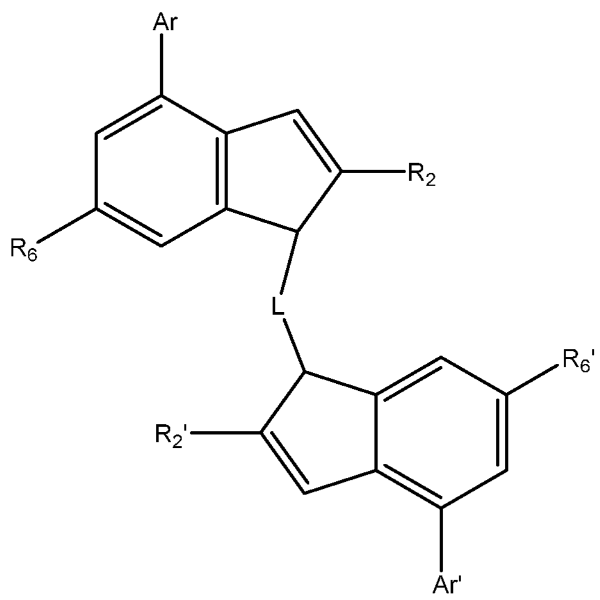

(I')

wherein the substituents are as hereinbefore defined; or a complex of formula (I) as hereinbefore defined.

**Definitions**

Throughout the description the following definitions are employed.

By free from an external carrier is meant that the catalyst does not contain an external support, such as an inorganic support, for example, silica or alumina, or an organic polymeric support material, onto which catalyst components are loaded.

The term $C_{1-20}$-hydrocarbyl group includes $C_{1-20}$-alkyl, $C_{2-20}$-alkenyl, $C_{2-20}$-alkynyl, $C_{3-20}$-cycloalkyl, $C_{3-20}$-cycloalkenyl, $C_{6-20}$-aryl groups, $C_{7-20}$-alkylaryl groups or $C_{7-20}$-arylalkyl groups or of course mixtures of these groups such as cycloalkyl substituted by alkyl.

Unless otherwise stated, preferred $C_{1-20}$-hydrocarbyl groups are $C_{1-20}$-alkyl, $C_{4-20}$-cycloalkyl, $C_{5-20}$-cycloalkyl-alkyl groups, $C_{7-20}$-alkylaryl groups, $C_{7-20}$-arylalkyl groups or $C_{6-20}$-aryl groups, especially $C_{1-10}$-alkyl groups, $C_{6-10}$-aryl groups, or $C_{7-12}$-arylalkyl groups, e.g. $C_{1-8}$-alkyl groups. Most especially preferred hydrocarbyl groups are methyl, ethyl, propyl, isopropyl, tertbutyl, isobutyl, $C_{5-6}$-cycloalkyl, cyclohexylmethyl, phenyl or benzyl.

The term halo includes fluoro, chloro, bromo and iodo groups, especially chloro groups, when relating to the complex definition.

The term heterocyclic group means a preferably monocyclic non aromatic ring structure comprising at least one heteroatom, e.g. piperidinyl or piperazinyl.

The term heteroaryl means a preferably monocyclic aromatic ring structure comprising at least one heteroatom. Preferred heteroaryl groups have 1 to 4 heteroatoms selected from O, S and N. Preferred heteroaryl groups include furanyl, thiophenyl, oxazole, thiazole, isothiazole, isooxazole, triazole and pyridyl.

The oxidation state of the metal ion is governed primarily by the nature of the metal ion in question and the stability of the individual oxidation states of each metal ion.

It will be appreciated that in the complexes of the invention, the metal ion M is coordinated by ligands X so as to satisfy the valency of the metal ion and to fill its available coordination sites. The nature of these $\sigma$-ligands can vary greatly.

Catalyst activity is defined in this application to be the amount of polymer produced(kg)/g catalyst/h. Catalyst metal activity is defined here to be the amount of polymer produced(kg)/g Metal/h. The term productivity is also sometimes used to indicate the catalyst activity although herein it designates the amount of polymer produced per unit weight of catalyst.

At least two of $R_2$ and $R_{2'}$; $R_6$ and $R_{6'}$; or Ar and Ar' are the same. That means that the two ligands in the complex of the invention can vary from each other only in relation to one substituent. If there are differences between the two ligands it is preferred if the variation is between $R_6$ and $R_{6'}$. Thus, $R_2$ and $R_{2'}$ and Ar and Ar' would be the same. Alternatively and preferably, the ligands forming the complex of the invention are the same giving C2 symmetry.

The term symmetrical is used herein to imply that both ligands are identical, i.e. there is C2 symmetry.

The term quaternary carbon atom directly attached to the indenyl ring defines a carbon atom having a bond to

the indenyl ring and also a bond to three other non hydrogen atoms, typically three other carbon atoms. A group such as Ind-C(Me)(Et)(iPr) therefore contains a quaternary carbon atom directly attached to the indenyl ring.

**Detailed Description of invention**

The catalysts of the invention preferably comprise a complex of formula (I)"

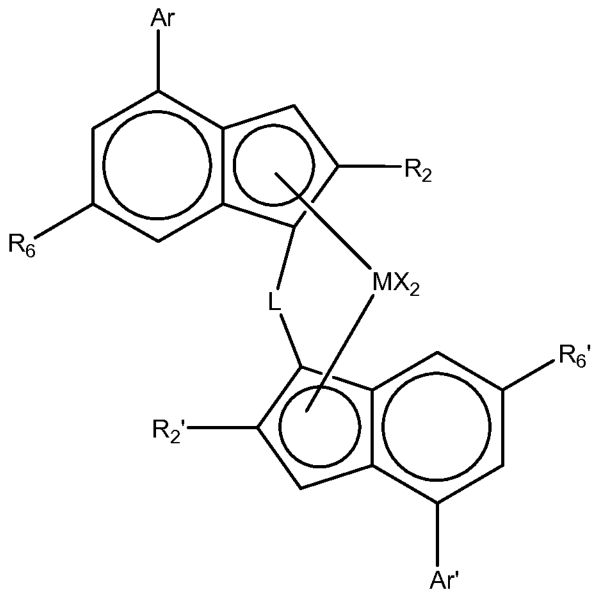

(I)''

wherein

M is zirconium or hafnium;
each X is a sigma ligand;
L is a divalent bridge selected from $-R'_2C-$, $-R'_2C-CR'_2-$, $-R'_2Si-$, $-R'_2Si-SiR'_2-$, $-R'_2Ge-$, wherein each R' is independently a hydrogen atom, $C_{1-20}$-alkyl, tri($C_{1-20}$-alkyl)silyl, $C_{6-20}$-aryl, $C_{7-20}$-arylalkyl or $C_{7-20}$-alkylaryl;
$R_2$ is a $C_{1-20}$-hydrocarbyl radical;
$R_{2'}$ is a $C_{1-20}$-hydrocarbyl radical;
$R_6$ is a linear or branched aliphatic $C_{1-20}$-hydrocarbyl group, $SR_9$ or $OR_9$;
$R_{6'}$ is a linear or branched aliphatic $C_{1-20}$-hydrocarbyl group, $SR_9'$ or $OR_{9'}$;
with the proviso that neither $R_6$ or $R_{6'}$ represents a group having a quaternary carbon atom attached directly to the indenyl ring;
$R_9$ is a $C_{1-20}$-hydrocarbyl group;
$R_{9'}$ is a $C_{1-20}$-hydrocarbyl group;
Ar is a $C_{6-12}$-aryl or $C_{5-12}$-heteroaryl group optionally carrying one or more substituents $R^8$;
Ar' is a $C_{6-12}$-aryl or $C_{5-12}$-heteroaryl group optionally carrying one or more substituents $R^{8'}$;
each $R_8$ is a $C_{1-20}$-hydrocarbyl group;
each $R_{8'}$ is a $C_{1-20}$-hydrocarbyl group;

wherein at least two of $R_2$ and $R_{2'}$, $R_6$ and $R_{6'}$ or Ar and Ar' are the same.

Complexes of formula (I) form a still yet further aspect of the invention along with their corresponding ligands in which the $MX_2$ group is absent and the indenyl ring is an indene ring, i.e. formula (I') as herein defined.

The catalyst of the invention is preferably not in supported form but is in solid particulate form. The term solid implies that the catalyst is solid at room temperature. The term particulate implies that the catalyst is a free flowing powder like material.

The two multicyclic ligands making up the complexes of the invention are preferably identical and hence the complexes of the invention may be symmetrical. The complexes of the invention are preferably in their *racemic* form. It is a feature of the invention, that the process described in detail below for the formation of the complexes of the invention gives rise to complexes predominantly in their *rac* form. There is low or very low *meso* form of the complexes formed, e.g. less than 20 wt% thereof.

M is preferably Zr.

Each X, which may be the same or different, is preferably a hydrogen atom, a halogen atom, a R, OR, $OSO_2CF_3$, OCOR, SR, $NR_2$ or $PR_2$ group wherein R is a linear or branched, cyclic or acyclic, $C_{1-20}$-alkyl, $C_{2-20}$-alkenyl, $C_{2-20}$-alkynyl, $C_{6-20}$-aryl, $C_{7-20}$-alkylaryl or $C_{7-20}$-arylalkyl radical; optionally containing heteroatoms belonging to groups 14-16. R is preferably a $C_{1-6}$-alkyl, phenyl or benzyl group.

Most preferably each X is independently a hydrogen atom, a halogen atom, $C_{1-6}$-alkoxy group or an R group, e.g. preferably a $C_{1-6}$-alkyl, phenyl or benzyl group. Most preferably X is chlorine or a methyl radical. Preferably both X groups are the same.

L is preferably a bridge comprising a heteroatom, such as silicon or, germanium, e.g. $-SiR^7{}_2-$, wherein each $R^7$ is independently $C_{1-20}$-alkyl, $C_{5-10}$-cycloalkyl, $C_{6-20}$-aryl or tri($C_{1-20}$-alkyl)silyl-residue, such as trimethylsilyl. More preferably $R^7$ is $C_{1-6}$-alkyl, especially methyl. Most preferably, L is a dimethylsilyl or diethylsilyl bridge. It may also be an ethylene or methylene bridge.

$R_2$ is preferably a $C_{1-10}$-hydrocarbyl group such as $C_{1-6}$-hydrocarbyl group. More preferably it is a linear or branched $C_{1-20}$-alkyl group. More preferably it is a linear or branched $C_{1-6}$-alkyl group, especially linear $C_{1-6}$-alkyl group such as methyl or ethyl. Ideally $R_2$ is methyl.

$R_{2'}$ is preferably a $C_{1-10}$-hydrocarbyl group such as $C_{1-6}$-hydrocarbyl group. More preferably it is a linear or branched $C_{1-20}$-alkyl group. More preferably it is a linear or branched $C_{1-6}$-alkyl group, especially linear $C_{1-6}$-alkyl group such as methyl or ethyl. Ideally $R_{2'}$ is methyl.

It is preferred if $R_2$ and $R_{2'}$ are the same. Ideally, both are methyl.

Ar and Ar' are both preferably $C_{6-10}$-aryl, especially Ph. Both groups preferably carry at least one group $R_8$ or $R_{8'}$ respectively. It is preferred if Ar and Ar' are the same.

At least one $R_8$ or $R_{8'}$ group is preferably present on the Ar, preferably Ph rings. It is preferred if all $R_8$ or $R_{8'}$ groups are the same. It is preferred however, if 2 such groups are present, i.e. n is 2. In particular, those groups should be positioned at the 3 and 5 positions of the Ph ring bound to the indenyl ligand.

$R_8$ is preferably a $C_{1-20}$-hydrocarbyl group, such as a $C_{1-20}$-alkyl group or $C_{6-10}$-aryl group. $R_8$ groups can be the same or different, preferably the same. More preferably, $R_8$ is a $C_{2-10}$-alkyl group such as $C_{3-8}$-alkyl group. Highly preferred groups are tert butyl groups. It is preferred if the group $R_8$ is bulky, i.e. is branched. Branching might be alpha or beta to the Ph ring. Branched $C_{3-8}$-alkyl groups are also favoured therefore. The use of a 3,5-disubstituted Ph group is preferred.

$R_{8'}$ is preferably as defined for $R_8$. As Ar is preferably the same as Ar', it is thus preferred if $R_8=R_{8'}$ as well.

It is preferred if $R_2$ and $R_{2'}$ are the same and Ar and Ar' are the same.

$R_6$ and $R_{6'}$ are preferably a linear or branched $C_{1-10}$-alkyl group, such as methyl, ethyl, isopropyl or a group $CH_2$-$C_{1-9}$-alkyl. $R_6$ and $R_{6'}$ are preferably not tert butyl but may be -CH($C_{1-4}$-alkyl)$_2$. Alternatively, $R_6$ and $R_{6'}$ may be $SC_{1-6}$-alkyl or $OC_{1-6}$-alkyl. It is preferred that the carbon atom directly attached to the indenyl ring at $R_6$ and $R_{6'}$ is not quaternary. The carbon atom directly attached to the indenyl ring should therefore carry at least one H atom, such as two H atoms.

Where one of the substituents in the complexes of the invention is different between the two ligands, it is preferred if the differing substituent is $R_6$ and $R_{6'}$ however, it is again preferred if $R_6 = R_{6'}$.

Preferably, the complex of the invention is a symmetrical complex of formula (II)

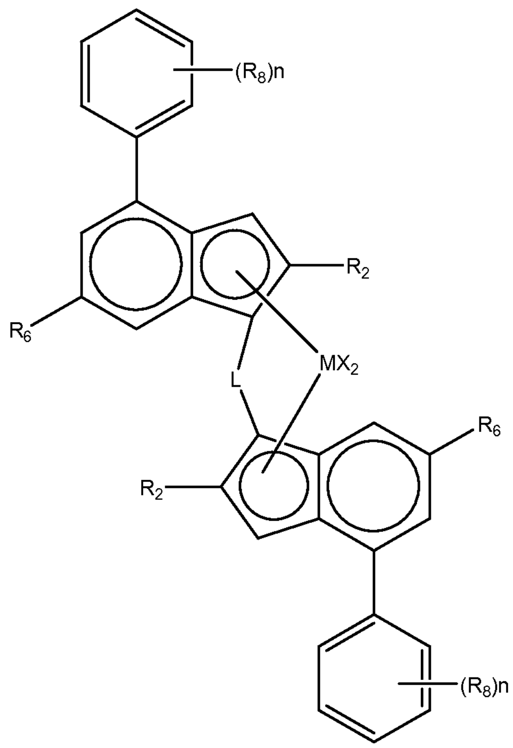

(II)

wherein

M is zirconium or hafnium;
each X is a sigma ligand;
L is a divalent bridge selected from $-R'_2C-$, $-R'_2C-CR'_2-$, $-R'_2Si-$, $-R'_2Si-SiR'_2-$, $-R'_2Ge-$, wherein each R' is independently a hydrogen atom, $C_{1-20}$-alkyl, tri($C_{1-20}$-alkyl)silyl, $C_{6-20}$-aryl, $C_{7-20}$-arylalkyl or $C_{7-20}$-alkylaryl;
$R_2$ is a $C_{1-20}$-hydrocarbyl radical;
$R_6$ is a linear or branched aliphatic $C_{1-20}$-hydrocarbyl group, $SR_9$ or $OR_9$;
with the proviso that $R_6$ preferably does not represent a group having a quaternary carbon atom directly attached to the indenyl ring;
$R_9$ is a $C_{1-20}$-hydrocarbyl group;
n is independently 1, 2 or 3; and
each $R_8$ is a $C_{1-20}$-hydrocarbyl group.

Further preferred complexes of the invention are therefore symmetrical complexes of formula (III)

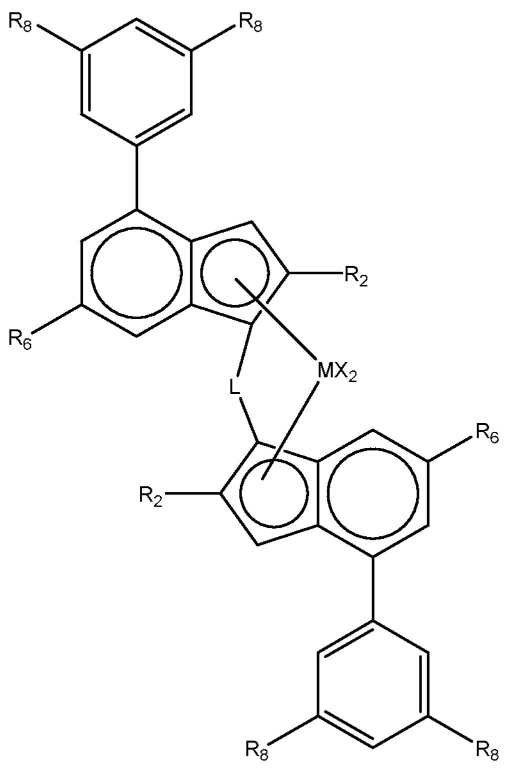

(III)

wherein

M is zirconium or hafnium;
each X is a sigma ligand, preferably each X is independently a hydrogen atom, a halogen atom, $C_{1-6}$-alkoxy group, $C_{1-6}$-alkyl, phenyl or benzyl group;
L is a divalent bridge selected from $-R'_2C-$, $-R'_2C-CR'_2-$, $-R'_2Si-$, $-R'_2Si-SiR'_2-$, $-R'_2Ge-$, wherein each R' is independently a hydrogen atom, $C_{1-20}$-alkyl, tri($C_{1-20}$-alkyl)silyl, $C_{6-20}$-aryl, $C_{7-20}$-arylalkyl or $C_{7-20}$-alkylaryl; preferably dimethylsilyl, methylene or ethylene;
$R_2$ is a $C_{1-10}$-alkyl group;
$R_6$ is a $C_{1-10}$-alkyl group with the proviso that $R_6$ does not represent a group having a quaternary carbon atom directly attached to the indenyl ring;
and each $R_8$ is a $C_{1-20}$-hydrocarbyl group, such as $C_{1-10}$-alkyl group.

Still more preferred complexes of the invention are symmetrical complexes of formula (IV):

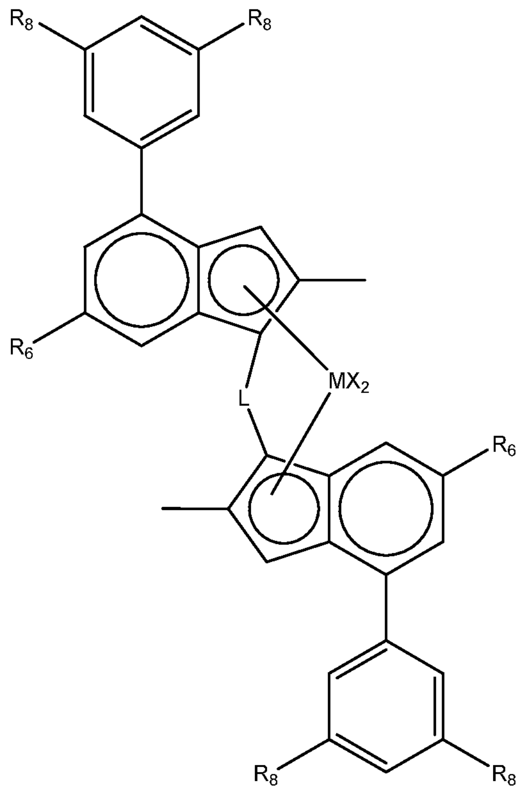

(IV)

wherein

M is zirconium or hafnium;
each X is a sigma ligand, preferably each X is independently a hydrogen atom, a halogen atom, $C_{1-6}$-alkoxy group, $C_{1-6}$-alkyl, phenyl or benzyl group;
L is a divalent bridge selected from $-R'_2C-$, $-R'_2C-CR'_2-$, $-R'_2Si-$, $-R'_2Si-SiR'_2-$, $-R'_2Ge-$, wherein each R' is independently a hydrogen atom, $C_{1-20}$-hydrocarbyl, tri($C_{1-20}$-alkyl)silyl, $C_{6-20}$-aryl, $C_{7-20}$-arylalkyl or $C_{7-20}$-alkylaryl; preferably dimethylsilyl;
$R_6$ is a linear $C_{1-10}$-alkyl group, $-CH(C_{1-4}\text{-alkyl})_2$ group or $CH_2(C_{1-9}\text{-alkyl})$ group, where said $C_{1-4}$-alkyl and $C_{1-9}$-alkyl groups can be linear or branched;
and each $R^8$ is a $C_{1-10}$-alkyl group.

Still more preferred complexes of the invention are symmetrical complexes of formula (V)

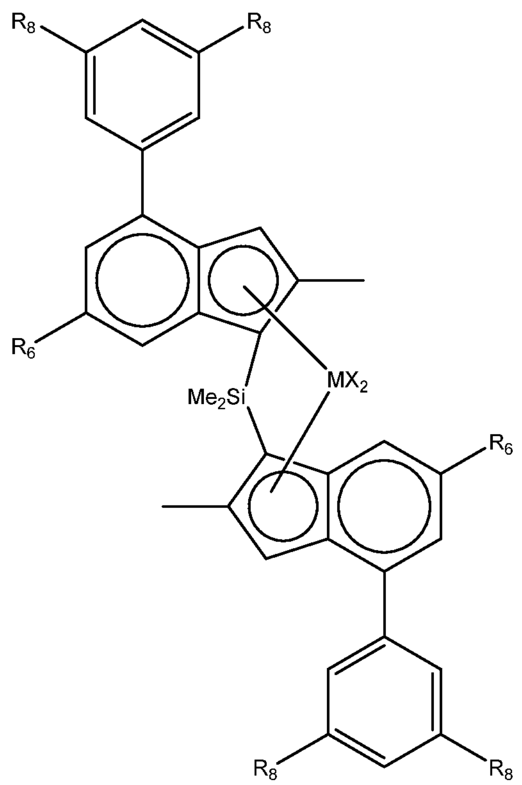

(V)

wherein M is Zr/Hf;
X is a hydrogen atom, a halogen atom, $C_{1-6}$-alkoxy group, $C_{1-6}$-alkyl, phenyl or benzyl group;
$R_6$ is linear $C_{1-6}$-alkyl, $-CH(C_{1-4}\text{-alkyl})_2$ or $-CH_2(C_{1-6}\text{-alkyl})$, where said $C_{1-4}$-alkyl and $C_{1-6}$-alkyl groups can be linear or branched; and
$R_8$ is $C_{3-8}$-alkyl, such as branched $C_{3-8}$-alkyl.

Further preferred complexes are symmetrical complexes of formula (VI):

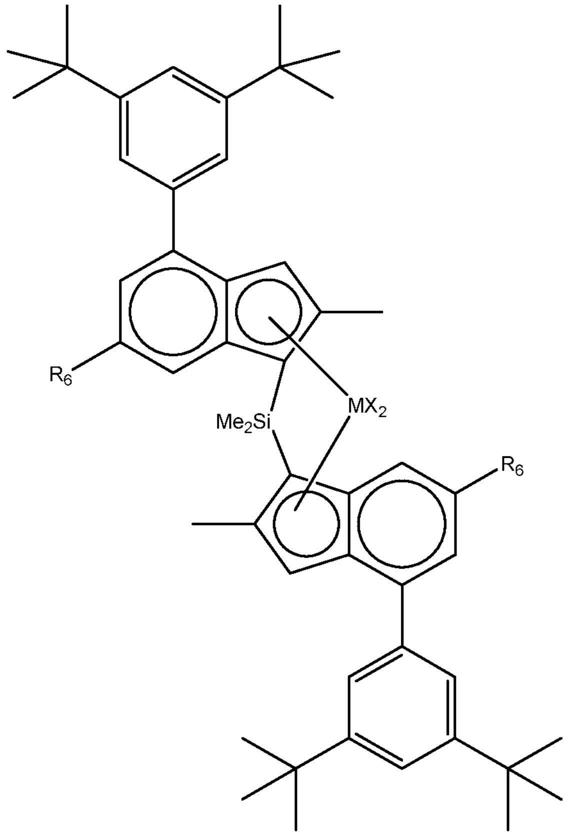

(VI)

wherein M is Zr/Hf
X is a hydrogen atom, a halogen atom, $C_{1-6}$-alkoxy group, $C_{1-6}$-alkyl, phenyl or benzyl group;
$R_6$ is linear $C_{1-6}$-alkyl, $-CH(C_{1-4}\text{-alkyl})_2$ or $-CH_2(C_{1-6}\text{-alkyl})$ where said $C_{1-4}$-alkyl and $C_{1-6}$-alkyl groups can be linear or branched.

Highly preferred complexes of the invention are:

*Rac*-dimethylsilanediylbis[2,6-dimethyl-4-(3,5-di-*tert*-butylphenyl)-inden-1-yl]zirconium dichloride (I-mc1)

*Rac*-dimethylsilanediylbis[2-methyl-4-(3,5-di-*tert*-butylphenyl)-6-ethyl-inden-1-yl]zirconium dichloride (I-mc2)

*Rac*-dimethylsilanediylbis[2-methyl-4-(3,5-di-*tert*-butylphenyl)-6-isopropyl-inden-1-yl]zirconium dichloride (I-mc3)

*Rac*-dimethylsilanediylbis[2-methyl-4-(3,5-di-*tert*-butylphenyl)-6-neopentyl-inden-1-yl]zirconium dichloride (I-mc4)

or their Hf analogues.

For the avoidance of doubt, any narrower definition of a substituent offered above can be combined with any other broad or narrowed definition of any other substituent.

Throughout the disclosure above, where a narrower definition of a substituent is presented, that narrower definition is deemed disclosed in conjunction with all broader and narrower definitions of other substituents in the application.

**Synthesis**

The ligands required to form the complexes and hence catalysts of the invention can be synthesised by any process and the skilled organic chemist would be able to devise various synthetic protocols for the manufacture of the necessary ligand materials. In particular WO02/02576 describes suitable synthetic protocols.

Ideally, the Ph group at the 4-position should carry at least two substituents, in particular substituents such as methyl, iso-propyl, neopentyl, tert-butyl or phenyl. Ideally, such bulky substituents are in the 3,5-positions of the 4-substituent. Ideally they are tert-butyl groups.

A conventional synthesis for ligands of formula (I) is given in WO02/02576. The key indene ligand precursor is shown in Scheme 1 below for the most preferred ligand uses herein:

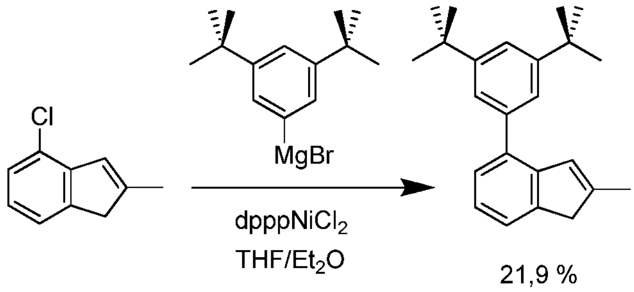


Scheme 1: synthesis of 4-(3',5'-di-tertbutylphenyl)-2-methyl-indene

The present inventors have devised a new procedure for the formation of this key intermediate which forms a further aspect of the invention.

The new procedure is shown in Scheme 2:

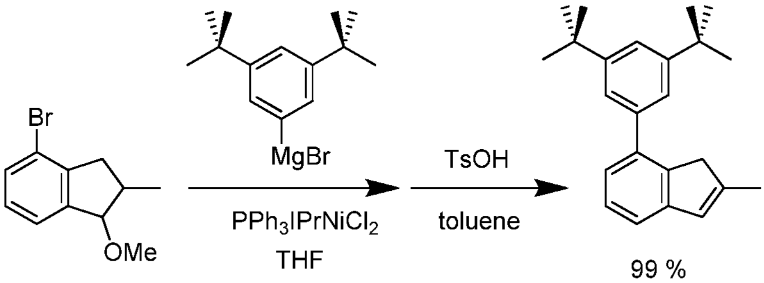


Scheme 2: synthesis of 7-(3',5'-di-tertbutylphenyl)-2-methyl-indene

The first step of this "one-pot" sequence is a Ni-catalyzed Kumada coupling, where the bromine atom in the indene 6-membered ring gets substituted with a di(tert-butyl)phenyl moiety). In order to obtain an indene i.e. formally eliminate MeOH and form a carbon-carbon double bond, an acid-catalyzed elimination using a dean-stark apparatus is used. TsOH can be used as an acid catalyst and toluene can be employed to remove water/methanol azeotropically.The Examples section contains further protocols which the skilled person can adapt to make compounds of the invention.

**Cocatalyst**

To form an active catalytic species it is normally necessary to employ a cocatalyst as is well known in the art. Cocatalysts comprising an organometallic compound of Group 13 metal, like organoaluminium compounds used to activate metallocene catalysts are suitable for use in this invention.

The olefin polymerisation catalyst system of the invention therefore comprises (i) a complex of the invention; and normally (ii) an aluminium alkyl compound (or other appropriate cocatalyst), or the reaction product thereof. Thus the cocatalyst is preferably an alumoxane, like MAO or an alumoxane other than MAO.

Alternatively, however, the catalysts of the invention may be used with other cocatalysts, e.g. boron compounds. It will be appreciated by the skilled man that where boron based cocatalysts are employed, it is normal to preactivate the complex by reaction thereof with an aluminium alkyl compound, such as TIBA. This procedure is well known and any suitable aluminium alkyl, e.g. $Al(C_{1-6}\text{-alkyl})_3$. can be used.

Boron based cocatalysts of interest include those of formula

$$BY_3$$

wherein Y is the same or different and is an aryl group of from 6 to about 15 carbon atoms, alkylaryl, arylalkyl, haloalkyl or haloaryl each having from 1 to 10 carbon atoms in the alkyl radical and from 6-20 carbon atoms in the aryl radical or fluorine, chlorine, bromine or iodine. Preferred examples for Y are haloaryl like p-fluorophenyl, 3,5-difluorophenyl, pentachlorophenyl, pentafluorophenyl, 3,4,5-trifluorophenyl and 3,5-di(trifluoromethyl)phenyl. Preferred options are trifluoroborane, triphenylborane, tris(4-fluorophenyl)borane, tris(3,5-difluorophenyl)borane, tris(4-fluoromethylphenyl)borane, tris(2,4,6-trifluorophenyl)borane, tris(penta-fluorophenyl)borane, tris(tolyl)borane, tris(3,5-dimethyl-phenyl)borane, tris(3,5-difluorophenyl)borane and/or tris (3,4,5-trifluorophenyl)borane.

Particular preference is given to tris(pentafluorophenyl)borane.

It is preferred however if borates are used, i.e. compounds containing a borate 3+ ion. Such ionic cocatalysts preferably contain a non-coordinating anion such as tetrakis(pentafluorophenyl)borate and tetraphenylborate. Suitable counterions are protonated amine or aniline derivatives such as methylammonium, anilinium, dimethylammonium, diethylammonium, N-methylanilinium, diphenylammonium, N,N-dimethylanilinium, trimethylammonium, triethylammonium, tri-n-butylammonium, methyldiphenylammonium, pyridinium, p-bromo-N,N-dimethylanilinium or p-nitro-N,N-dimethylanilinium.

Preferred ionic compounds which can be used according to the present invention include: tributylammoniumtetra(pentafluorophenyl)borate, tributylammoniumtetra(trifluoromethylphenyl)borate, tributylammoniumtetra(4-fluorophenyl)borate, N,N-dimethylcyclohexylammoniumtetrakis(pentafluorophenyl)borate, N,N-dimethylbenzylammoniumtetrakis(pentafluorophenyl)borate, N,N-dimethylaniliniumtetrakis(pentafluorophenyl)borate, N,N-di(propyl)ammoniumtetrakis(pentafluorophenyl)borate, di(cyclohexyl)ammoniumtetrakist(pentafluorophenyl)borate, triphenylcarbeniumtetrakis(pentafluorophenyl)borate, or ferroceniumtetrakis(pentafluorophenyl)borate. Preference is given to triphenylcarbeniumtetrakis(pentafluorophenyl) borate, N,N-dimethylcyclohexylammoniumtetrakis(pentafluorophenyl)borate or NSN-dimethylbenzylammoniumtetrakis(pentafluorophenyl)borate.

The use of $B(C_6F_5)_3$, $C_6H_5N(CH_3)_2H{:}B(C_6F_5)_4$, $(C_6H_5)_3C{:}B(C_6F_5)_4$ or $Ni(CN)_4[B(C_6F_5)_3]_4^{2-}$ is especially preferred.

Suitable amounts of borate cocatalyst will be well known to the skilled man.

The use of aluminoxanes, especially MAO, is highly preferred.

Suitable amounts of aluminoxane cocatalyst will be well known to the skilled man. Typically Al to M molar ratios are from 1:1 to 1000:1 mol/mol. Preferably when an aluminium alkyl is used as a cocatalyst, the molar ratio of the aluminium in the activator to the transition metal in the complex is from 1 to 500 mol/mol, preferably from 10 to 400 mol/mol and in particular from 50 to 400 mol/mol.

**Manufacture**

The metallocene complex of the present invention can be used in combination with a suitable cocatalyst as a catalyst for the polymerization of olefins, e.g. in a solvent such as toluene or an aliphatic hydrocarbon, (i.e. for polymerization in solution), as it is well known in the art. Preferably, polymerization of olefins, especially propylene, takes place in the condensed phase or in gas phase.

The catalyst of the invention is preferably in solid particulate form but unsupported, i.e. no external carrier is used. In order to provide the catalyst of the invention in solid form but without using an external carrier, it is preferred if a liquid liquid emulsion system is used. The process involves forming dispersing catalyst components (i) and (ii) in a solvent, and solidifying said dispersed droplets to form solid particles.

In particular, the method involves preparing a solution of one or more catalyst components; dispersing said solution in an solvent to form an emulsion in which said one or more catalyst components are present in the droplets of the dispersed phase; immobilising the catalyst components in the dispersed droplets, in the absence of an external particulate porous support, to form solid particles comprising the said catalyst, and optionally recovering said particles.

This process enables the manufacture of active catalyst particles with improved morphology, e.g. with a predetermined spherical shape and particle size and without using any added external porous support material, such as an inorganic oxide, e.g. silica. Also desirable surface properties can be obtained.

By the term "preparing a solution of one or more catalyst components" is meant that the catalyst forming compounds may be combined in one solution which is dispersed to the immiscible solvent, or, alternatively, at least two separate catalyst solutions for each part of the catalyst forming compounds may be prepared, which are then dispersed successively to the solvent.

In a preferred method for forming the catalyst at least two separate solutions for each or part of said catalyst may be prepared, which are then dispersed successively to the immiscible solvent.

More preferably, a solution of the complex comprising the transition metal compound and the cocatalyst is combined with the solvent to form an emulsion wherein that inert solvent forms the continuous liquid phase and the solution comprising the catalyst components forms the dispersed phase (discontinuous phase) in the form of dispersed droplets. The droplets are then solidified to form solid catalyst particles, and the solid particles are separated from the liquid and optionally washed and/or dried. The solvent forming the continuous phase may be immiscible to the catalyst solution at least at the conditions (e.g. temperatures) used during the dispersing step.

The term "immiscible with the catalyst solution" means that the solvent (continuous phase) is fully immiscible or partly immiscible i.e. not fully miscible with the dispersed phase solution.

Preferably said solvent is inert in relation to the compounds of the catalyst system to be produced. Full disclosure of the necessary process can be found in WO03/051934 which is herein incorporated by reference.

The inert solvent must be chemically inert at least at the conditions (e.g. temperature) used during the dispersing step. Preferably, the solvent of said continuous phase does not contain dissolved therein any significant amounts of catalyst forming compounds. Thus, the solid particles of the catalyst are formed in the droplets from the compounds which originate from the dispersed phase (i.e. are provided to the emulsion in a solution dispersed into the continuous phase).

The terms "immobilisation" and "solidification" are used herein interchangeably for the same purpose, i.e. for forming free flowing solid catalyst particles in the absence of an external porous particulate carrier, such as silica. The solidification happens thus within the droplets. Said step can be effected in various ways as disclosed in said WO03/051934 Preferably solidification is caused by an external stimulus to the emulsion system such as a temperature change to cause the solidification. Thus in said step the catalyst component(s) remain "fixed" within the formed solid particles. It is also possible that one or more of the catalyst components may take part in the solidification/immobilisation reaction.

Accordingly, solid, compositionally uniform particles having a predetermined particle size range can be obtained.

Furthermore, the particle size of the catalyst particles of the invention can be controlled by the size of the droplets in the solution, and spherical particles with a uniform particle size distribution can be obtained.

The invention is also industrially advantageous, since it enables the preparation of the solid particles to be carried out as a one-pot procedure. Continuous or semicontinuous processes are also possible for producing the catalyst.

**Dispersed Phase**

The principles for preparing two phase emulsion systems are known in the chemical field. Thus, in order to form the two phase liquid system, the solution of the catalyst component(s) and the solvent used as the continuous liquid phase have to be essentially immiscible at least during the dispersing step. This can be achieved in a known manner e.g. by choosing said two liquids and/or the temperature of the dispersing step/solidifying step accordingly.

A solvent may be employed to form the solution of the catalyst component(s). Said solvent is chosen so that it dissolves said catalyst component(s). The solvent can be preferably an organic solvent such as used in the field, comprising an optionally substituted hydrocarbon such as linear or branched aliphatic, alicyclic or aromatic hydrocarbon, such as a linear or cyclic alkane, an aromatic hydrocarbon and/or a halogen containing hydrocarbon.

Examples of aromatic hydrocarbons are toluene, benzene, ethylbenzene, propylbenzene, butylbenzene and xylene. Toluene is a preferred solvent. The solution may comprise one or more solvents. Such a solvent can thus be used to facilitate the emulsion formation, and usually does not form part of the solidified particles, but e.g. is removed after the solidification step together with the continuous phase.

Alternatively, a solvent may take part in the solidification, e.g. an inert hydrocarbon having a high melting point (waxes), such as above 40 °C, suitably above 70 °C, e.g. above 80 °C or 90 °C, may be used as solvents of the dispersed phase to immobilise the catalyst compounds within the formed droplets.

In another embodiment, the solvent consists partly or completely of a liquid monomer, e.g. liquid olefin monomer designed to be polymerised in a "prepolymerisation" immobilisation step.

**Continuous Phase**

The solvent used to form the continuous liquid phase is a single solvent or a mixture of different solvents and may be immiscible with the solution of the catalyst components at least at the conditions (e.g. temperatures) used during the dispersing step. Preferably said solvent is inert in relation to said compounds.

The term "inert in relation to said compounds" means herein that the solvent of the continuous phase is chemically inert, i.e. undergoes no chemical reaction with any catalyst forming component. Thus, the solid particles of the catalyst are formed in the droplets from the compounds which originate from the dispersed phase, i.e. are provided to the emulsion in a solution dispersed into the continuous phase.

It is preferred that the catalyst components used for forming the solid catalyst will not be soluble in the solvent of the continuous liquid phase. Preferably, said catalyst components are essentially insoluble in said continuous phase forming solvent.

Solidification takes place essentially after the droplets are formed, i.e. the solidification is effected within the droplets e.g. by causing a solidifying reaction among the compounds present in the droplets. Furthermore, even if some solidifying agent is added to the system separately, it reacts within the droplet phase and no catalyst forming components go into the continuous phase.

The term "emulsion" used herein covers both bi-and multiphasic systems.

In a preferred embodiment said solvent forming the continuous phase is an inert solvent including a halogenated

organic solvent or mixtures thereof, preferably fluorinated organic solvents and particularly semi, highly or perfluorinated organic solvents and functionalised derivatives thereof. Examples of the above-mentioned solvents are semi, highly or perfluorinated hydrocarbons, such as alkanes, alkenes and cycloalkanes, ethers, e.g. perfluorinated ethers and amines, particularly tertiary amines, and functionalised derivatives thereof. Preferred are semi, highly or perfluorinated, particularly perfluorinated hydrocarbons, e.g. perfluorohydrocarbons of e.g. C3-C30, such as C4-C10. Specific examples of suitable perfluoroalkanes and perfluorocycloalkanes include perfluoro-hexane, -heptane, -octane and - (methylcyclohexane). Semi fluorinated hydrocarbons relates particularly to semifluorinated n-alkanes, such as perfluoroalkyl-alkane.

"Semi fluorinated" hydrocarbons also include such hydrocarbons wherein blocks of -C-F and -C-H alternate. "Highly fluorinated" means that the majority of the -C-H units are replaced with -C-F units. "Perfluorinated" means that all -C-H units are replaced with -C-F units. See the articles of A. Enders and G. Maas in "Chemie in unserer Zeit", 34. Jahrg. 2000, Nr.6, and of Pierandrea Lo Nostro in "Advances in Colloid and Interface Science", 56 (1995) 245-287, Elsevier Science.

**Dispersing step**

The emulsion can be formed by any means known in the art: by mixing, such as by stirring said solution vigorously to said solvent forming the continuous phase or by means of mixing mills, or by means of ultra-sonic wave, or by using a so called phase change method for preparing the emulsion by first forming a homogeneous system which is then transferred by changing the temperature of the system to a biphasic system so that droplets will be formed.

The two phase state is maintained during the emulsion formation step and the solidification step, as, for example, by appropriate stirring.

Additionally, emulsifying agents/emulsion stabilisers can be used, preferably in a manner known in the art, for facilitating the formation and/or stability of the emulsion. For the said purposes e.g. surfactants, e.g. a class based on hydrocarbons (including polymeric hydrocarbons with a molecular weight e.g. up to 10 000 and optionally interrupted with a heteroatom(s)), preferably halogenated hydrocarbons, such as semi- or highly fluorinated hydrocarbons optionally having a functional group selected e.g. from -OH, -SH, $NH_2$, $NR''_2$. -COOH, $-COONH_2$, oxides of alkenes, - $CR''=CH_2$, where R" is hydrogen, or $C_{1-20}$-alk-yl, $C_{2-20}$-alkenyl or $C_{2-20}$-alkynyl group, oxo-groups, cyclic ethers and/or any reactive derivative of these groups, like alkoxy, or carboxylic acid alkyl ester groups, or, preferably semi-, highly- or perfluorinated hydrocarbons having a functionalised terminal, can be used. The surfactants can be added to the catalyst solution, which forms the dispersed phase of the emulsion, to facilitate the forming of the emulsion and to stabilize the emulsion.

Alternatively, an emulsifying and/or emulsion stabilising aid can also be formed by reacting a surfactant precursor bearing at least one functional group with a compound reactive with said functional group and present in the catalyst solution or in the solvent forming the continuous phase. The obtained reaction product acts as the actual emulsifying aid and or stabiliser in the formed emulsion system.

Examples of the surfactant precursors usable for forming said reaction product include e.g. known surfactants which bear at least one functional group selected e.g. from -OH, -SH, $NH_2$, $NR''_2$. -COOH, $-COONH_2$, oxides of alkenes, $-CR''=CH_2$, where R" is hydrogen, or $C_{1-20}$-alkyl, $C_{2-20}$-alkenyl or $C_{2-20}$-alkynyl group, oxo-groups, cyclic ethers with 3 to 5 ring atoms, and/or any reactive derivative of these groups, like alkoxy or carboxylic acid alkyl ester groups; e.g. semi-, highly or perfluorinated hydrocarbons bearing one or more of said functional groups. Preferably, the surfactant precursor has a terminal functionality as defined above.

The compound reacting with such surfactant precursor is preferably contained in the catalyst solution and may be a further additive or one or more of the catalyst forming compounds. Such compound is e.g. a compound of group 13 (e.g. MAO and/or an aluminium alkyl compound and/or a transition metal compound).

If a surfactant precursor is used, it is preferably first reacted with a compound of the catalyst solution before the addition of the transition metal compound. In one embodiment e.g. a highly fluorinated $C_{1-n}$- (suitably $C_{4-30}$- or $C_{5-15}$-) alcohol (e.g. highly fluorinated heptanol, octanol or nonanol), oxide (e.g. propenoxide) or acrylate ester is reacted with a cocatalyst to form the "actual" surfactant. Then, an additional amount of cocatalyst and the transition metal compound is added to said solution and the obtained solution is dispersed to the solvent forming the continuous phase. The "actual" surfactant solution may be prepared before the dispersing step or in the dispersed system. If said solution is made before the dispersing step, then the prepared "actual" surfactant solution and the transition metal solution may be dispersed successively (e.g. the surfactant solution first) to the immiscible solvent, or be combined together before the dispersing step.

**Solidification**

The solidification of the catalyst component(s) in the dispersed droplets can be effected in various ways, e.g. by causing or accelerating the formation of said solid catalyst forming reaction products of the compounds present in the droplets. This can be effected, depending on the used compounds and/or the desired solidification rate, with or

without an external stimulus, such as a temperature change of the system.

In a particularly preferred embodiment, the solidification is effected after the emulsion system is formed by subjecting the system to an external stimulus, such as a temperature change. Temperature differences are of e.g. 5 to 100 °C, such as 10 to 100 °C, or 20 to 90 °C, such as 50 to 90 °C.

The emulsion system may be subjected to a rapid temperature change to cause a fast solidification in the dispersed system. The dispersed phase may e.g. be subjected to an immediate (within milliseconds to few seconds) temperature change in order to achieve an instant solidification of the component(s) within the droplets. The appropriate temperature change, i. e. an increase or a decrease in the temperature of an emulsion system, required for the desired solidification rate of the components cannot be limited to any specific range, but naturally depends on the emulsion system, i. a. on the used compounds and the concentrations/ratios thereof, as well as on the used solvents, and is chosen accordingly. It is also evident that any techniques may be used to provide sufficient heating or cooling effect to the dispersed system to cause the desired solidification.

In one embodiment the heating or cooling effect is obtained by bringing the emulsion system with a certain temperature to an inert receiving medium with significantly different temperature, e.g. as stated above, whereby said temperature change of the emulsion system is sufficient to cause the rapid solidification of the droplets. The receiving medium can be gaseous, e.g. air, or a liquid, preferably a solvent, or a mixture of two or more solvents, wherein the catalyst component(s) is (are) immiscible and which is inert in relation to the catalyst component(s). For instance, the receiving medium comprises the same immiscible solvent used as the continuous phase in the first emulsion formation step.

Said solvents can be used alone or as a mixture with other solvents, such as aliphatic or aromatic hydrocarbons, such as alkanes. Preferably a fluorinated solvent as the receiving medium is used, which may be the same as the continuous phase in the emulsion formation, e.g. perfluorinated hydrocarbon.

Alternatively, the temperature difference may be effected by gradual heating of the emulsion system, e.g. up to 10 °C per minute, preferably 0.5 to 6 °C per minute and more preferably in 1 to 5 °C per minute.

In case a melt of e.g. a hydrocarbon solvent is used for forming the dispersed phase, the solidification of the droplets may be effected by cooling the system using the temperature difference stated above.

Preferably, the "one phase" change as usable for forming an emulsion can also be utilised for solidifying the catalytically active contents within the droplets of an emulsion system by, again, effecting a temperature change in the dispersed system, whereby the solvent used in the droplets becomes miscible with the continuous phase, preferably a fluorous continuous phase as defined above, so that the droplets become impoverished of the solvent and the solidifying components remaining in the "droplets" start to solidify. Thus the immisciblity can be adjusted with respect to the solvents and conditions (temperature) to control the solidification step.

The miscibility of e.g. organic solvents with fluorous solvents can be found from the literature and be chosen accordingly by a skilled person. Also the critical temperatures needed for the phase change are available from the literature or can be determined using methods known in the art, e.g. the Hildebrand-Scatchard-Theorie. Reference is also made to the articles of A. Enders and G. and of Pierandrea Lo Nostro cited above.

Thus according to the invention, the entire or only part of the droplet may be converted to a solid form. The size of the "solidified" droplet may be smaller or greater than that of the original droplet, e.g. if the amount of the monomer used for the prepolymerisation is relatively large.

The solid catalyst particles recovered can be used, after an optional washing step, in a polymerisation process of an olefin. Alternatively, the separated and optionally washed solid particles can be dried to remove any solvent present in the particles before use in the polymerisation step. The separation and optional washing steps can be effected in a known manner, e.g. by filtration and subsequent washing of the solids with a suitable solvent.

The droplet shape of the particles may be substantially maintained. The formed particles may have a mean size range of 1 to 500 μm, e.g. 5 to 500 μm, advantageously 5 to 200 μm or 10 to 150 μm. Even a mean size rangeof 5 to 60 μm is possible. The size may be chosen depending on the polymerisation the catalyst is used for. Advantageously, the mean particle size of the ready particulate catalysts of the invention are in the range of 2 to 150 μm, preferably 5 to 120 μm, more preferably 5 to 90 μm and especially in the range 10 to 70 μm. The particles are essentially spherical in shape, they have a low porosity and a low surface area.

The formation of solution can be effected at a temperature of 0-100 °C, e.g. at 20-80 °C. The dispersion step may be effected at -20 °C-100 °C, e.g. at about -10-70 °C, such as at -5 to 30 °C, e.g. around 0 °C.

To the obtained dispersion an emulsifying agent as defined above, may be added to improve/stabilise the droplet formation. The solidification of the catalyst component in the droplets is preferably effected by raising the temperature of the mixture, e.g. from 0 °C temperature up to 100 °C, e.g. up to 60-90 °C, gradually. E.g. in 1 to 180 minutes, e.g. 1-90 or 5-30 minutes, or as a rapid heat change. Heating time is dependent on the size of the reactor.

During the solidification step, which is preferably carried out at about 60 to 100 °C, preferably at about 75 to 95 °C, (below the boiling point of the solvents) the solvents may preferably be removed and optionally the solids are washed with a wash solution, which can be any solvent or mixture of solvents such as those defined above and/or used

in the art, preferably a hydrocarbon, such as pentane, hexane or heptane, suitably heptane. The washed catalyst can be dried or it can be slurried into an oil and used as a catalyst-oil slurry in polymerisation process.

All or part of the preparation steps can be done in a continuous manner. Reference is made to WO2006/069733 describing principles of such a continuous or semicontinuous preparation methods of the solid catalyst types, prepared via emulsion/solidification method.

**Polymerisation**

The olefin polymerized using the catalyst of the invention is preferably propylene or a higher alpha-olefin or a mixture of ethylene and an $\alpha$-olefin or a mixture of alpha olefins, for example $C_{2-20}$ olefins, e.g. ethylene, propylene, 1-butene, 1-hexene, 4-methyl-1-pentene, 1-octene etc. The olefins polymerized in the method of the invention may include any compound which includes unsaturated polymerizable groups. Thus, for example unsaturated compounds, such as $C_{6-20}$ olefins (including cyclic and polycyclic olefins (e.g. norbornene)), and polyenes, especially $C_{4-20}$ dienes, may be included in a comonomer mixture with lower olefins, e.g. $C_{2-5}$ $\alpha$-olefins. Diolefins (i.e. dienes) are suitably used for introducing long chain branching into the resultant polymer. Examples of such dienes include $\alpha,\omega$ linear dienes such as 1,5-hexadiene, 1,6-heptadiene, 1,8-nonadiene, 1,9-decadiene, etc.

The catalysts of the present invention are particularly suited for use in the manufacture of polypropylene polymers, especially isotactic polypropylene.

Polymerization in the method of the invention may be effected in one or more, e.g. 1, 2 or 3, polymerization reactors, using conventional polymerization techniques, e.g. gas phase, solution phase, slurry or bulk polymerization.

In general, a combination of slurry (or bulk) and at least one gas phase reactor is often preferred, particularly with the reactor order being slurry (or bulk) then one or more gas phase reactors.

In case of propylene polymerisation for slurry reactors, the reaction temperature will generally be in the range 60 to 110°C (e.g. 60-90°C), the reactor pressure will generally be in the range 5 to 80 bar (e.g. 20-60 bar), and the residence time will generally be in the range 0.1 to 5 hours (e.g. 0.3 to 2 hours). The monomer is usually used as reaction medium.

For gas phase reactors, the reaction temperature used will generally be in the range 60 to 115°C (e.g. 70 to 110°C), the reactor pressure will generally be in the range 10 to 25 bar, and the residence time will generally be 0,5 to 8 hours (e.g. 0,5 to 4 hours)The gas used will be the monomer optionally as mixture with a non-reactive gas such as nitrogen or propane. In addition to actual polymerisation steps and reactors, the process can contain any additional polymerisation steps, like prepolymerisation step, and any further after reactor handling steps as known in the art.

Generally the quantity of catalyst used will depend upon the nature of the catalyst, the reactor types and conditions and the properties desired for the polymer product. As is well known in the art hydrogen can be used for controlling the molecular weight of the polymer. It is particularly notable that the catalyst of the present invention performs exceptionally well over a wide range of hydrogen concentration used during the polymerisation process, which makes the catalyst beneficial to be used for productions of a wide range of polymers The catalysts are useful at higher hydrogen concentrations as well with lower hydrogen concentrations to get polymer with higher molecular weight. The activity of the catalysts of the invention is also very high and the polymer productivity levels are excellent.

It is a feature of the invention that the claimed catalysts enable the formation of polymers with remarkably high melting temperatures, Tm and with remarkably high molecular weight. These features can be achieved at commercially interesting polymerisation temperatures, e.g. 60 °C or more. It is a preferred feature of the invention that the catalysts of the invention are used to polymerise propylene at a temperature of at least 60 °C, preferably at least 65 °C, such as at least 70 °C. It is also notable that catalysts of the present invention produce polymers with high melting temperatures, such as above 156 °C with clearly higher activity of the catalyst compared to catalysts of the prior art.

Catalyst activities may be of the order of 10.0 kg/g(cat)/h or more, such as 12 kg/g(cat)/h or more.

The catalysts of the invention enable the formation of high molecular weight polypropylene which also possess high isotacticity. Isotacticity is measured by 13C NMR or also by DSC. Thus, in the case of polypropylene homopolymers, isotacticity can be higher than 99.1 % mm when measured by 13C NMR. When measured by standard DSC, the high isotacticity of the polypropylene homopolymers means a melting point (Tm) higher than 150 °C, preferably higher than 152 °C, even more preferably higher than 155 °C.

The molecular weight of the polypropylene can be at least 300,000, preferably at least 400,000, especially at least 500,000. However, the molecular weight of the formed polymer is dependent on the amount of hydrogen employed, as is well known in the art.

Polypropylenes made by the metallocene catalysts of the invention can be made with $MFR_2$ values in the whole range of interest, that is from very high (as high as 2000, for example 1000 or 500) to very low, that is fractional values (<1). Hydrogen can be used to manipulate MFR as is well known.

The polymers made by the catalysts of the invention are useful in all kinds of end articles such as pipes, films (cast, blown and BOPP films), fibers, moulded articles (e.g. injection moulded, blow moulded, rotomoulded articles),

extrusion coatings and so on. Film applications, such as those requiring BOPP (bi-oriented polypropylene) film, especially for capacitors are favoured.

The invention will now be illustrated by reference to the following nonlimiting Examples.

Chemicals

All the chemicals and chemical reactions were handled under an inert gas atmosphere using Schlenk and glovebox techniques, with oven-dried glassware, syringes, needles or cannulas.

MAO was purchased from Albermarle and used as a 30 wt-% solution in toluene.

The mixture of perfluoroalkylethyl acrylate esters (CAS 65605-70-1) used as the surfactant was purchased from the Cytonix corporation, dried over activated molecular sieves (2 times) and degassed by argon bubbling prior to use.

Perfluoro-1,3-dimethylcyclohexane (PFC, CAS 335-27-3) was dried over activated molecular sieves (2 times) and degassed by argon bubbling prior to use.

Triethylaluminum was purchased from Crompton and used in pure form. Hydrogen is provided by AGA and purified before use.

Propylene is provided by Borealis and adequately purified before use.

2 M HCl, 12 M HCl (Reachim, Russia), silica gel 60 (40-63 um, Merck), $K_2CO_3$ (Merck), $ZrCl_4(THF)_2$ magnesium turnings (Acros), TsOH (Aldrich), nBuLi (Chemetall), n-hexane (Merck), methyl iodide (Acros), 2-Bromo-4-chloro-toluene (Aldrich), 1-bromo-3,5-di-*tert*-butylbenzene (Aldrich), (3,5-di-tert-butylphenyl)boronic acid (Aldrich), Pd(OAc)2 (Strem), PPh3 (Acros), and DME (Merck) were used as received. Toluene (Merck), THF (Merck), dichloromethane (Merck), were kept and distilled over Na/K alloy. Dichlorodimethylsilane (Merck) was distilled before use. $CDCl_3$, DMSO-$d_6$ and $CD_2Cl_2$ (Deutero GmbH) for NMR experiments were dried and kept over $CaH_2$.

Bis(2,6-diisopropylphenyl)imidazolium chloride, i.e. IPr(HCl), and (IPr)$NiCl_2(PPh_3)$ were synthesized as described in [Hintermann, L. Beilstein J. Org. Chem. 2007, 3, 1] and [Matsubara, K.; Ueno, K.; Shibata, Y. Organometallics 2006, 25, 3422], respectively.

4-Bromo-1-methoxy-2-methylindane was obtained as described in [Izmer, V.V.; Lebedev, A.Y.; Nikulin, M.V.; Ryabov, A.N.; Asachenko, A.F.; Lygin, A.V.; Sorokin, D.A.; Voskoboynikov, A.Z. Organometallics 2006, 25, 1217].

Test methods

**ICP analysis**

The elemental analysis of a catalyst was performed by taking a solid sample of mass, M, cooling over dry ice. Samples were diluted up to a known volume, V, by dissolving in nitric acid (HNO3, 65 %, 5 % of V) and freshly deionised (DI) water (5 % of V). The solution was then added to hydrofluoric acid (HF, 40 %, 3% of V), diluted with DI water up to the final volume, V, and left to stabilise for two hours.

The analysis was run at room temperature using a Thermo Elemental iCAP 6300 Inductively Coupled Plasma - Optical Emmision Spectrometer (ICP-OES) which was calibrated using a blank (a solution of 5 % HNO3, 3 % HF in DI water), and 6 standards of 0.5 ppm, 1 ppm, 10 ppm, 50 ppm, 100 ppm and 300 ppm of Al, with 0.5 ppm, 1 ppm, 5 ppm, 20 ppm, 50 ppm and 100 ppm of Hf and Zr in solutions of 5 % HNO3, 3 % HF in DI water.

Immediately before analysis the calibration is 'resloped' using the blank and 100 ppm Al, 50 ppm Hf, Zr standard, a quality control sample (20 ppm Al, 5 ppm Hf, Zr in a solution of 5 % HNO3, 3 % HF in DI water) is run to confirm the reslope. The QC sample is also run after every 5th sample and at the end of a scheduled analysis set.

The content of hafnium was monitored using the 282.022 nm and 339.980 nm lines and the content for zirconium using 339.198 nm line. The content of aluminium was monitored via the 167.079 nm line, when Al concentration in ICP sample was between 0-10 ppm (calibrated only to 100 ppm) and via the 396.152 nm line for Al concentrations above 10 ppm.

The reported values are an average of three successive aliquots taken from the same sample and are related back to the original catalyst by inputting the original mass of sample and the dilution volume into the software.

**DSC analysis**

Melting temperature $T_m$ and crystallization temperature $T_c$ were measured on approx. 5 mg samples with a Mettler-Toledo 822e differential scanning calorimeter (DSC), according to ISO11357-3 in a heat/cool/heat cycle with a scan rate of 10 °C/min in the temperature range of +23 to +225 °C under a nitrogen flow rate of 50 ml $min^{-1}$. Melting and crystallization temperatures were taken as the endotherm and exotherm peaks, respectively in the second heating and in the cooling step. Calibration of the instrument was performed with $H_20$, Lead, Tin, Indium, according to ISO 11357-1.

**Melt Flow Rate**

The melt flow rate (MFR) is determined according to ISO 1133 and is indicated in g/10 min. The MFR is an indication of the flowability, and hence the processability, of the molten polymer. The higher the melt flow rate, the lower the viscosity of the polymer. The MFR is determined at 230 °C and may be determined at different loadings such as 2.16 kg ($MFR_2$) or 21.6 kg ($MFR_{21}$).

**Molecular weight averages, molecular weight distribution (Mn, Mw, Mz, MWD)**

Molecular weight averages (Mz, Mw and Mn), Molecular weight distribution (MWD) and its broadness, described by polydispersity index, PDI= Mw/Mn (wherein Mn is the number average molecular weight and Mw is the weight average molecular weight) were determined by Gel Permeation Chromatography (GPC) according to ISO 16014-1:2003, ISO 16014-2:2003, ISO 16014-4:2003 and ASTM D 6474-12 using the following formulas:

$$M_n = \frac{\sum_{i=1}^{N} A_i}{\sum_{i=1}^{N}(A_i/M_i)} \quad (1)$$

$$M_w = \frac{\sum_{i=1}^{N}(A_i x\, M_i)}{\sum_{i=1}^{N} A_i} \quad (2)$$

$$M_z = \frac{\sum_{i=1}^{N}(A_i\, x\, M_i^2)}{\sum_{i=1}^{N}(A_i/M_i)} \quad (3)$$

For a constant elution volume interval $\Delta V_i$, where $A_i$, and $M_i$ are the chromatographic peak slice area and polyolefin molecular weight (MW), respectively associated with the elution volume, $V_i$, where N is equal to the number of data points obtained from the chromatogram between the integration limits.

A high temperature GPC instrument, equipped with either infrared (IR) detector (IR4 or IR5 from PolymerChar (Valencia, Spain) or differential refractometer (RI) from Agilent Technologies, equipped with 3 x Agilent-PLgel Olexis and 1x Agilent-PLgel Olexis Guard columns was used. As the solvent and mobile phase 1,2,4-trichlorobenzene (TCB) stabilized with 250 mg/L 2,6-Di tert butyl-4-methyl-phenol) was used. The chromatographic system was operated at 160 °C and at a constant flow rate of 1 mL/min. 200 μL of sample solution was injected per analysis. Data collection was performed using either Agilent Cirrus software version 3.3 or PolymerChar GPC-IR control software.

The column set was calibrated using universal calibration (according to ISO 16014-2:2003) with 19 narrow MWD polystyrene (PS) standards in the range of 0,5 kg/mol to 11 500 kg/mol. The PS standards were dissolved at room temperature over several hours. The conversion of the polystyrene peak molecular weight to polyolefin molecular weights is accomplished by using the Mark Houwink equation and the following Mark Houwink constants:

$$K_{PS} = 19 \text{ x } 10^{-3} \text{ mL/g}, \alpha_{PS} = 0.655$$

$$K_{PE} = 39 \text{ x } 10^{-3} \text{ mL/g}, \qquad \alpha_{PE} = 0.725$$

$$K_{PP} = 19 \text{ x } 10^{-3} \text{ mL/g}, \alpha_{PP} = 0.725$$

A third order polynomial fit was used to fit the calibration data.

All samples were prepared in the concentration range of 0,5 -1 mg/ml and dissolved at 160 °C for 2.5 hours for PP or 3 hours for PE under continuous gentle shaking.

**Quantification of polypropylene homopolymer microstructure by NMR spectroscopy**

Quantitative nuclear-magnetic resonance (NMR) spectroscopy was used to quantify the isotacticity and content of regio-defects of the polypropylene homopolymers. Quantitative $^{13}C\{^1H\}$ NMR spectra recorded in the solution-state

using a Bruker Advance III 400 NMR spectrometer operating at 400.15 and 100.62 MHz for $^{1}H$ and $^{13}C$ respectively. All spectra were recorded using a $^{13}C$ optimised 10 mm selective excitation probehead at 125 °C using nitrogen gas for all pneumatics. Approximately 200 mg of material was dissolved in 1,2-tetrachloroethane-$d_2$ (TCE-$d_2$). This setup was chosen primarily for the high resolution needed for tacticity distribution quantification (Busico, V., Cipullo, R., Prog. Polym. Sci. 26 (2001) 443; Busico, V.; Cipullo, R., Monaco, G., Vacatello, M., Segre, A.L., Macromolecules 30 (1997) 6251). Standard single-pulse excitation was employed utilising the NOE and bi-level WALTZ16 decoupling scheme (Zhou, Z., Kuemmerle, R., Qiu, X., Redwine, D., Cong, R., Taha, A., Baugh, D. Winniford, B., J. Mag. Reson. 187 (2007) 225; Busico, V., Carbonniere, P., Cipullo, R., Pellecchia, R., Severn, J., Talarico, G., Macromol. Rapid Commun. 2007, 28, 11289). A total of 8192 (8k) transients were acquired per spectra. Quantitative $^{13}C\{^{1}H\}$ NMR spectra were processed, integrated and relevant quantitative properties determined from the integrals using proprietary computer programs. All chemical shifts are internally referenced to the methyl signal of the isotactic pentad mmmm at 21.85 ppm.

The tacticity distribution was quantified through integration of the methyl region between 23.6 and 19.7 ppm correcting for any sites not related to the stereo sequences of interest (Busico, V., Cipullo, R., Prog. Polym. Sci. 26 (2001) 443; Busico, V., Cipullo, R., Monaco, G., Vacatello, M., Segre, A.L., Macromolecules 30 (1997) 6251). The pentad isotacticity was determined through direct integration of the methyl region and reported as either the mole fraction or percentage of isotactic pentad mmmm with respect to all steric pentads i.e. [mmmm] = mmmm / sum of all steric pentads. When appropriate integrals were corrected for the presence of sites not directly associated with steric pentads.

Characteristic signals corresponding to regio irregular propene insertion were observed (Resconi, L., Cavallo, L., Fait, A., Piemontesi, F., Chem. Rev. 2000, 100, 1253). The presence of secondary inserted propene in the form of 2,1 erythro regio defects was indicated by the presence of the two methyl signals at 17.7 and 17.2 ppm and confirmed by the presence of other characteristic signals. The amount of 2,1 erythro regio defects was quantified using the average integral (e) of the e6 and e8 sites observed at 17.7 and 17.2 ppm respectively, i.e. e = 0.5 * (e6 + e8). Characteristic signals corresponding to other types of regio irregularity were not observed (Resconi, L., Cavallo, L., Fait, A., Piemontesi, F., Chem. Rev. 2000, 100, 1253). The amount of primary inserted propene (p) was quantified based on the integral of all signals in the methyl region (CH3) from 23.6 to 19.7 ppm paying attention to correct for other species included in the integral not related to primary insertion and for primary insertion signals excluded from this region such that p = CH3 + 2*e. The relative content of a specific type of regio defect was reported as the mole fraction or percentage of said regio defect with respect all observed forms of propene insertion i.e. sum of all primary (1,2), secondary (2,1) and tertiary (3,1) inserted propene units, e.g. [21e]=e/(p+e+t+i). The total amount of secondary inserted propene in the form of 2,1-erythro or 2,1-threo regio defects was quantified as sum of all said regio irregular units, i.e. [21] = [21e] + [21t].

*Synthesis of the complexes*

**Comparative complexes:**

**C-mc1:**

The metallocene rac-dimethylsilylenebis(2-methyl-4-(3,5-di-tert-butylphenyl)-inden-1-yl)zirconium dichloride has been synthesized as described in European Patent Application No. 12199251.5.

**C-mc2:**

The metallocene rac-dimethylsilylenebis(2-methyl-4-(3,5-di-tert-butylphenyl)-7-methoxy-inden-1-yl)zirconium dichloride has been synthesized as described in EP-A-2532687.

**Inventive complexes:**

**I-mc1:**

**2-Chloro-4-methylbenzaldehyde**

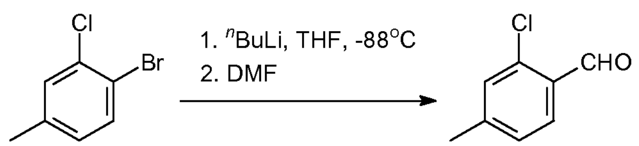

165 ml (413 mmol) of 2.5 M nBuLi in hexanes was added dropwise over 1 h to a solution of 82.2 g (400 mmol) of 4-bromo-3-chlorotoluene in 400 ml of THF cooled to -88 °C. The resulting mixture was stirred for 30 min at this temperature, and then 44.0 g (602 mmol) of DMF was added dropwise by vigorous stirring for 10 min. The reaction mixture was stirred overnight at room temperature, and then 100 ml of water and 400 ml of 3 N HCl were added at 0 °C. The organic layer was separated, the aqueous layer was extracted with 2 x 125 ml of dichloromethane. The combined organic extract was dried over K2CO3 and then passed through a short layer of silica gel 60 (40-63 μm). The silica gel layer was additionally washed by 50 ml of dichloromethane. The combined organic eluate was evaporated to dryness to give a slightly orange liquid which was then distilled under vacuum to give 58.0 g (94%) of the title product (b.p. 99-102 °C/11 mm Hg,) as a colorless liquid crystallized overnight at room temperature.

Anal. calc. for C8H7ClO: C, 62.15; H, 4.56. Found: C, 62.24; H, 4.45.
1HNMR (CDCl3): δ 10.4 (s, 1H, CHO), 7.80 (d, J = 7.8 Hz, 1H, 6-H), 7.25 (s, 1H, 3-H), 7.17 (d, J = 7.8 Hz, 1H, 5-H), 2.40 (s, 3H, 4-Me).

**(2-Chloro-4-methylphenyl)methanol**

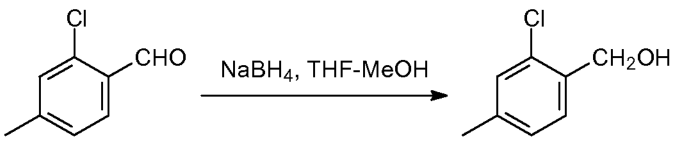


375 ml of methanol was added dropwise with vigorous stirring over 5 h to a mixture of 116 g (0.75 mol) of 2-chloro-4-methylbenzaldehyde and 43.0 g (1.14 mol) of NaBH4 in 750 ml of THF at 0-5 °C. This mixture was stirred overnight at room temperature, and then evaporated to dryness. The resulting oily mass was acidified with 1200 ml of 2 M HCl to pH~1, and the formed product was extracted consequently with 3 x 400 ml of dichloromethane. The combined organic extract was dried over Na2SO4 and evaporated to dryness. This product was used without further purification.
1H NMR (CDCl3): δ 7.29 (d, J = 7.8 Hz, 1H, 5-H), 7.15 (s, 1H, 3-H), 7.04 (d, J = 7.8 Hz, 1H, 6-H), 4.67 (s, 2H, CH2OH), 2.59 (br.s, 1H, CH2OH), 2.30 (s, 3H, 4-Me). 13C{1H} NMR (CDCl3): δ 138.9, 135.0, 132.4, 129.7, 128.6, 127.6, 62.5, 20.7.

**2-Chloro-1-(chloromethyl)-4-methylbenzene**

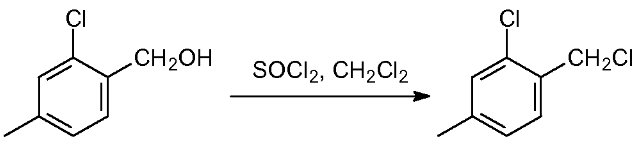


The above-obtained 2-chloro-4-methylbenzyl alcohol dissolved in 750 ml of dichloromethane was added dropwise to 55 ml (754 mmol) of thionyl chloride at +5°C. The resulting solution was stirred overnight at room temperature and then evaporated to dryness. The residue was dissolved in 500 ml dichloromethane, and the formed solution was washed with 250 ml of water. The organic layer was separated and the aqueous layer was extracted with 2 x 150 ml of dichloromethane. The combined organic extract was dried over Na2SO4, passed through a short pad of silica gel 60 (40-63 μm), and then evaporated to dryness. Crude product was distilled under vacuum to give 114 g (87%) of the title product as a colorless liquid, b.p. 92-95 °C/5 mm Hg.

Anal. calc. for C8H8Cl2: C, 54.89; H, 4.61. Found: C, 54.80; H, 4.65.
1H NMR (CDCl3): δ 7.30 (d, J = 7.8 Hz, 1H, 5-H), 7.19 (s, 1H, 3-H), 7.04 (d, J = 7.8 Hz, 1H, 6-H), 4.64 (s, 2H, CH2Cl), 2.30 (s, 3H, Me). 13C{1H} NMR (CDCl3): δ 140.3, 133.7, 131.9, 130.6, 130.2, 127.9, 43.5, 20.8.

**3-(2-Chloro-4-methylphenyl)-2-methylpropanoyl chloride**

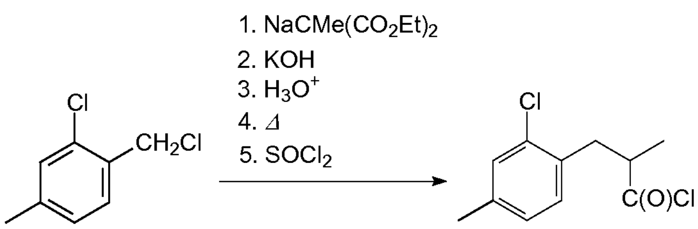


119 g (0.68 mol) of diethyl methylmalonate was added to a solution of sodium ethoxide obtained from 17.0 g (0.74 mol) of sodium metal and 600 ml of dry ethanol. The formed mixture was stirred for 15 min, and then 114 g (0.651 mol) of 2-chloro-1-(chloromethyl)-4-methylbenzene was added by vigorous stirring at such a rate to maintain a gentle reflux. The resulting mixture was refluxed for 2 h and then cooled to room temperature. A solution of 135 g of KOH in 550 ml of water was added. This mixture was refluxed for 4 h to saponificate the ester formed. Ethanol and water were distilled off until vapor temperature reached 95oC, and 3000 ml of water and then 12 M HCl (to pH~1) were added to the residue. The precipitated substituted methylmalonic acid was filtered off and washed with water. This diacid was dearboxylated at 160-180 °C to form a slightly orange oil crystallized at room temperature. A mixture of the formed acid and 166 ml of thionyl chloride was stirred for 24 h at room temperature. After evaporation of an excess of thionyl chloride, the residue was distilled under vacuum to give 123 g (82%) of the title product, b.p. 105-117oC/5 mm Hg.

Anal. calc. for C11H12Cl2O: C, 57.16; H, 5.23. Found: C, 57.36; H, 5.38.
1H NMR (CDCl3): δ 7.19 (s, 1H, 3-H), 7.10 (d, J = 7.7 Hz, 1H, 5-H), 7.00 (d, J = 7.7 Hz, 1H, 6-H), 3.20-3.32 (m, 2H, CHH' and CHMe), 2.82 (dd, J = 12.8 Hz, J = 6.4 Hz, 1H, CHH'), 2.30 (s, 3H, 4-Me), 1.30 (d, J = 6.8 Hz, 3H, CHMe). 13C{1H} NMR (CDCl3): δ 177.1, 138.6, 133.8, 132.1, 131.2, 130.2, 127.7, 51.4, 36.5, 20.7, 16.7.

**4-Chloro-2,6-dimethylindan-1-one**

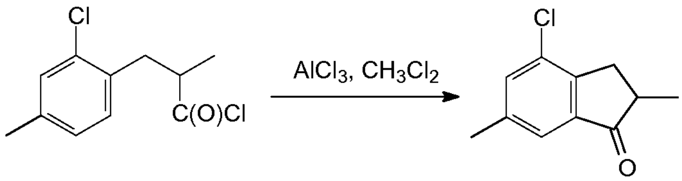


A solution of 123 g (531 mmol) of 3-(2-chloro-4-methylphenyl)-2-methylpropanoyl chloride in 100 ml of dichloromethane was added dropwise at 5°C to a stirred suspension of 85.0 g (638 mmol) of AlCl3 in 500 ml of dichloromethane This mixture was stirred overnight at room temperature and then poured on 500 g of crushed ice. The organic layer was separated, and the aqueous layer was extracted with 3 x 100 ml of dichloromethane. The combined organic extract was washed with aqueous K2CO3, dried over K2CO3, passed through a short pad of silica gel 60 (40-63 μm), and then evaporated to dryness. Crude product was distilled under vacuum to give 98.4 g (95%) of a colorless liquid, b.p. 131-132 °C/8 mm Hg.

Anal. calc. for C11H11ClO: C, 67.87; H, 5.70. Found: C, 68.01; H, 5.69.
1HNMR (CDCl3): δ 7.42 (s, 1H, 7-H), 7.38 (s, 1H, 5-H), 3.32 (dd, J = 17.3 Hz, J = 7.8 Hz, 1H, 3-CHH'), 2.68-2.76 (m, 1H, 2-H), 2.62 (dd, 1H, J = 17.3 Hz, J = 3.6 Hz, 3-CHH'), 2.38 (s, 3H, 6-Me), 1.31 (d, J = 7.5 Hz, 3H, 2-Me). 13C{1H} NMR (CDCl3): δ 208.2, 148.0, 139.3, 138.1, 135.0, 132.1, 122.2, 42.0, 33.3, 20.7, 16.1.

**4-Chloro-1-methoxy-2,6-dimethylindane**

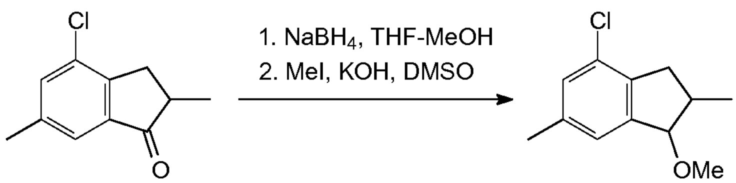

205 ml of methanol was added dropwise by vigorous stirring over 5 h at 0-5 °C to a mixture of 98.4 g (0.505 mol) of 4-chloro-2,6-dimethylindan-1-one and 29.0 g (0.767 mol) of NaBH4 in 510 ml of THF. This mixture was stirred overnight at room temperature and then evaporated to dryness. The residue was acidified by 2 M HCl to pH 5-6, and the formed 4-chloro-2,6-dimethylindan-1-ol was extracted with 3 x 300 ml of dichloromethane. The combined organic extract was dried over Na2SO4 and evaporated to dryness. The obtained white solid was dissolved in 800 ml of DMSO and 132 g (2.35 mol) of KOH and 163 g (1.15 mol) of MeI were added to it. This mixture was stirred for 5 h at ambient temperature. The solution was decanted from an excess of KOH, the latter was additionally washed with 3 x 350 ml of dichloromethane. The combined organic extract was washed with 3000 ml of water. The organic layer was separated, and the aqueous layer was extracted with 3 x 300 ml of dichloromethane. The combined organic extract was washed with 7 x 1500 ml of water, dried over Na2SO4, and then evaporated to dryness. The residue was distilled under vacuum to give 99.9 g (94%) of the title product consisting of two diastereomers, b.p. 104-105oC/8 mm Hg.

Anal. calc. for C12H15ClO: C, 68.40; H, 7.18. Found: C, 68.58; H, 7.25.

Syn-isomer. 1H NMR (CDCl3): δ 7.05 (s, 2H, 5-H and 7-H), 4.51 (d, J = 5.7 Hz, 1H, 1-H), 3.41 (s, 3H, OMe), 2.92 (dd, J = 15.3 Hz, J = 6.4 Hz, 1H, 3-CHH'), 2.68-2.59 (m, 2H, 3-CHH' and 2-H), 2.32 (s, 3H, 6-Me), 1.07 (d, J = 6.8 Hz, 3H, 2-Me). 13C{1H} NMR (CDCl3): δ 144.6, 138.3, 137.8, 130.7, 128.7, 124.1, 86.4, 57.0, 38.2, 36.9,21.0, 13.5.

Anti-isomer. 1H NMR (CDCl3): δ 7.05 (s, 1H, 7-H), 7.07 (s, 1H, 5-H), 4.37 (d, J = 3.9 Hz, 1H, 1-H), 3.45 (s, 3H, OMe), 3.19 (dd, J = 16.2 Hz, J = 7.6 Hz, 1H, 3-CHH'), 2.50 (m, 1H, 2-H), 2.42 (dd, J = 16.2 Hz, J = 5.0 Hz, 1H, 3-CHH'), 2.32 (s, 3H, 6-Me), 1.16 (d, J = 6.8 Hz, 3H, 2-Me). 13C{1H} NMR (CDCl3): δ 144.2, 138.1 (two resonances), 130.7, 128.9, 124.2, 91.8, 56.6, 39.4, 37.2, 21.0, 19.3.

**4-(3,5-Di-tert-butylphenyl)-1-methoxy-2,6-dimethylindane**

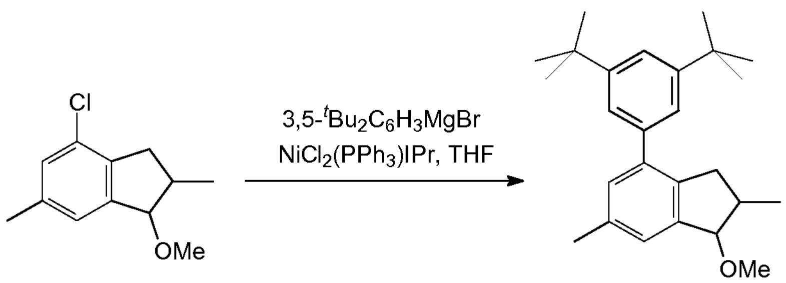


400 ml (200 mmol) of 0.5 M 3,5-di-tert-butylphenylmagnesium bromide in THF was added at room temperature to a mixture of 2.00 g (2.56 mmol, 1.74 mol.%) of NiCl2(PPh3)IPr and 31.1 g (148 mmol) of 4-chloro-1-methoxy-2,6-dimethylindane. The resulting mixture was refluxed for 3.5 h, then cooled to room temperature, followed by the addition of 100 ml of water. The major part of THF was distilled off on rotary evaporator. To the residue 400 ml of dichloromethane and 1000 ml of 1 M HCl were added. The organic layer was separated, the aqueous layer was extracted with 2 x 100 ml of dichloromethane. The combined organic extract was evaporated to dryness to give a slightly yellowish oil. The product was isolated by flash-chromatography on silica gel 60 (40-63 μm; eluent: hexanes-dichloromethane = 2:1, vol., then 1:1, vol.). This procedure gave a colorless thick oil containing two stereoisomers.

Anal. calc. for C26H36O: C, 85.66; H, 9.95. Found: C, 85.89; H, 10.13.

Syn-isomer. 1H NMR (CDCl3): δ 7.39 (t, J = 1.4 Hz, 1H, 4-H in 3,5-tBu2C6H3), 7.28 (d, J = 1.4 Hz, 2H, 2,6-H in 3,5-tBu2C6H3), 7.18 (s, 1H, 5-H in indane), 7.16 (s, 1H, 7-H in indane), 4.51 (d, J = 5.5 Hz, 1H, 1-H in indane), 3.47 (s, 3H, OMe), 2.91 (dd, J = 15.5 Hz, J = 7.1 Hz, 1H, 3-CHH' in indane), 2.79 (dd, J = 15.5 Hz, J = 6.8 Hz, 1H, 3-CHH' in indane), 2.45 (m, 1H, 2-H in indane), 2.41 (s, 3H, 6-Me in indane), 1.36 (s, 18H, 3,5-tBu2C6H3), 1.08 (d, J = 6.8 Hz, 3H, 2-Me in indane). 13C{1H} NMR (CDCl3): δ 150.4, 143.6, 140.0, 139.5, 138.3, 136.1, 129.2, 124.6, 123.0, 120.7, 86.3, 56.9, 39.2, 38.0, 34.9, 31.5, 21.3, 13.6.

Anti-isomer. 1H NMR (CDCl3): δ 7.39 (t, J = 1.6 Hz, 1H, 4-H in 3,5-tBu2C6H3), 7.28 (d, 2H, J = 1.6 Hz, 2,6-H in 3,5-tBu2C6H3), 7.19 (s, 1H, 5-H in indane), 7.17 (s, 1H, 7-H in indane), 4.39 (d, J = 3.6 Hz, 1H, 1-H in indane), 3.50 (s, 3H, OMe), 3.31 (dd, J = 15.3 Hz, J = 6.8 Hz, 1H, 3-CHH' in indane), 2.50 (m, 1H, 2-H in indane), 2.45 (dd, J = 15.3 Hz, J = 5.0 Hz, 1H, 3-CHH' in indane), 2.41 (s, 3H, 6-Me in indane), 1.36 (s, 18H, 3,5-tBu2C6H3), 1.10 (d, J = 6.8 Hz, 3H, 2-Me in indane). 13C{1H} NMR (CDCl3): δ 150.4, 142.9, 140.0, 139.6, 137.8, 136.4, 129.6, 124.7, 122.9, 120.7, 91.5, 56.6, 40.0, 38.1, 34.9, 31.5, 21.3, 19.1.

**7-(3,5-Di-tert-butylphenyl)-2,5-dimethyl-1H-indene**

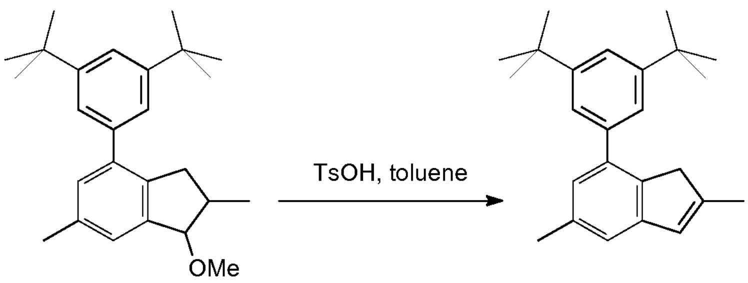


1.8 g of TsOH was added to a solution of 4-(3,5-di-tert-butylphenyl)-1-methoxy-2,6-dimethylindane (prepared above) in 500 ml of toluene, and the resulting solution was refluxed using Dean-Stark head for 15 min. After cooling to room temperature the reaction mixture was washed by 200 ml of 10% aqueous NaHCO3. The organic layer was separated, and the aqueous layer was additionally extracted with 2 x 100 ml of dichloromethane. The combined organic extract was evaporated to dryness, and the product was isolated by flash-chromatography on silica gel 60 (40-63 μm; eluent: hexanes-dichloromethane = 10:1, vol.). This procedure gave 47.4 g (97%) of white crystalline material.

Anal. calc. for C25H32: C, 90.30; H, 9.70. Found: C, 90.44; H, 9.51.

1H NMR (CDCl3): δ 7.40 (t, J = 1.8 Hz, 1H, 4-H in 3,5-tBu2C6H3), 7.36 (d, J = 1.8 Hz, 2H, 2,6-H in 3,5-tBu2C6H3), 7.07 (s, 1H, 6-H in indene), 6.97 (s, 1H, 4-H in indene), 6.48 (m, 1H, 3-H in indene), 3.34 (s, 2H, CH2 in indene), 2.42 (s, 3H, 5-Me in indene), 2.12 (s, 3H, 2-Me in indene), 1.37 (s, 18H, 3,5-tBu2C6H3). 13C{1H} NMR (CDCl3): δ 150.5, 146.7, 146.5, 140.6, 138.2, 138.0, 136.5, 127.1, 125.1, 122.8, 120.8, 119.5, 42.5, 34.9, 31.6, 21.5, 16.8.

**Bis[2,6-dimethyl-4-(3,5-di-tert-butylphenyl)-1H-inden-1-yl](dimethyl)silane**

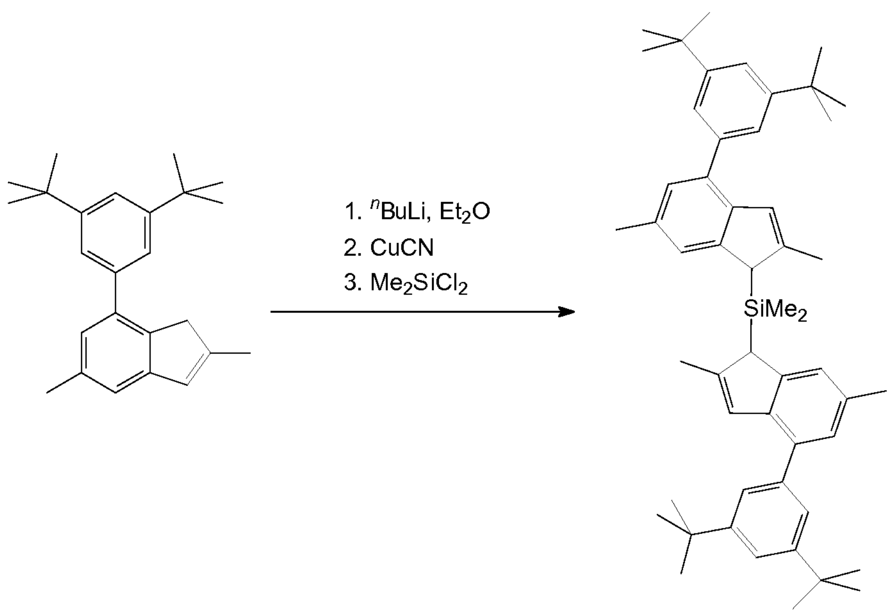


20.0 ml (50.0 mmol) of 2.5 M$^{n}$BuLi in hexanes was added in one portion to a solution of 16.6 g (50.0 mmol) of 7-(3,5-di-*tert*-butylphenyl)-2,5-dimethyl-1*H*-indene in 250 ml of ether at -50 °C. This mixture was stirred overnight at room temperature, then the resulting yellow suspension was cooled to -40 °C, and 200 mg of CuCN was added. The resulting mixture was stirred for 30 min at -25 °C, and 3.23 g (25.03 mmol) of dichlorodimethylsilane was added in one portion. Then this mixture was stirred overnight at ambient temperature. This solution was filtered through a pad of silica gel 60 (40-63 μm) which was additionally washed by 2 x 50 ml of dichloromethane. The combined filtrate was evaporated under vacuum, and the residue was dried in vacuo at elevated temperature. This procedure gave 18.4 g (~100%) of the

title product of >90% purity (ca. 1:1 mixture of stereoisomers based on NMR analysis) as a yellowish glass which was used without further purification.
$^1$H NMR ($CDCl_3$): $\delta$ 7.53 (m), 7.48-7.52 (m), 7.37 (m), 7.31 (m), 7.25 (m), 7.23 (m), 6.91 (m), 6.89 (m), 3.90 (s), 2.543 (s), 2.538 (s), 2.37 (s), 2.34 (s), 1.51 (s), - 0.05 (s), -0.09 (2s).

***Rac*-dimethylsilanediylbis[2,6-dimethyl-4-(3,5-di-*tert*-butylphenyl)-inden-1-yl]zirconium dichloride (I-mc1)**

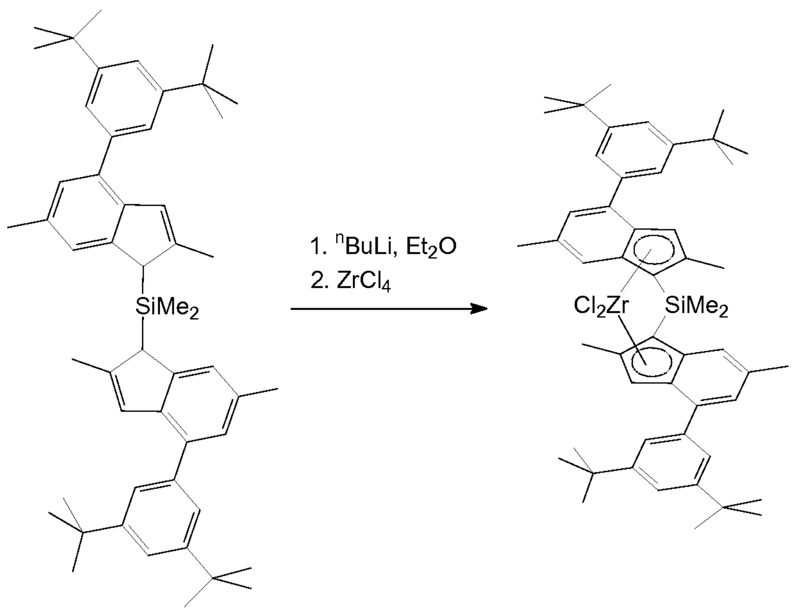


20.0 ml (50.0 mmol) of 2.5 M $^n$BuLi in hexanes was added in one portion to a solution of 18.4 g (25.0 mmol) of bis[4-(3,5-di-*tert*-butylphenyl)-2,6-dimethyl-1*H*-inden-1-yl](dimethyl)silane (as prepared above) in 250 ml of ether cooled to -30°°C. This mixture was stirred overnight at room temperature. The resulting light-orange solution was cooled to -50 °C, and 5.83 g (25.0 mmol) of $ZrCl_4$ $ZrCl_4$ was added. The reaction mixture was stirred for 24 h and then evaporated to dryness. The residue was poured into 200 ml of hot toluene. The obtained hot suspension was filtered through glass frit (G4). On the evidence of NMR spectroscopy, the filtrate included a ca. 1 to 1 mixture of *rac*- and *meso*-zirconocenes. This filtrate was evaporated to 110 ml and heated to dissolve a precipitate. Orange crystals precipitated from this solution overnight at room temperature were collected and dried in vacuum. This procedure gave a ca. 2 to 3 mixture of *rac*- and *meso*-zirconocenes. The mother liquor was evaporated to ca. 50 ml. Again, crystals precipitated from this solution at room temperature were collected and dried in vacuum. This procedure gave 1.72 g of pure *rac*-zirconocene. The mother liquor was evaporated to ca. 25 ml, heated almost to the boiling point, and 25 ml of n-hexane was added. Crystals precipitated from this solution at room temperature were collected and dried in vacuum. This procedure gave 1.68 g of a ca. 3 to 2 mixture of *rac*- and *meso*-zirconocenes. The above-described ca. 2 to 3 mixture of *rac*- and *meso*-complexes was re-crystallized from 80 ml of toluene. Orange crystals precipitated from this solution overnight at room temperature were collected and dried in vacuum. This procedure gave 0.98 g of pure *meso*-zirconocene. The mother liquor was evaporated to ca. 60 ml. Crystals precipitated from this solution overnight at room temperature were collected and dried in vacuo. This procedure gave 7.10 g of a ca. 7 to 3 mixture of *meso*- and *rac*-zirconocenes. Again, the mother liquor was evaporated to ca. 40 ml. Crystals precipitated from this solution overnight at room temperature were collected and dried in vacuum. This procedure gave 2.10 g of pure *rac*-zirconocene. Thus, the total yield of *rac*- and *meso*-zirconocenes isolated in this reaction was 13.6 g (62%).

*Rac*-complex (I-mc1).

Anal. calc. for $C_{52}H_{66}Cl_2SiZr$: C, 70.87; H, 7.55. Found: C, 71.05; H, 7.69.
$^1$H NMR ($CDCl_3$): $\delta$ 7.52 (m, 4H), 7.39 (m, 4H), 7.24 (m, 2H), 6.91 (m, 2H), 2.38 (s, 6H), 2.25 (s, 6H), 1.33 (s, 6H), 1.32 (s, 36H). $^{13}C\{^1H\}$ NMR ($CDCl_3$): $\delta$ 151.0, 139.4, 138.6, 135.7, 134.8, 130.6, 128.9, 128.4, 123.3, 122.8, 122.5, 121.4, 82.2, 3 5.1, 31.5, 22.2, 18.4, 2.8.

*Meso*-complex (meso- I-mc1).

Anal. found: C, 71.16; H, 7.72.
$^1$H NMR ($CDCl_3$): $\delta$ 7.46 (m, 4H), 7.39 (m, 2H), 7.37 (m, 2H), 6.96 (m, 2H), 6.73 (m, 2H), 2.42 (s, 6H), 2.26 (s, 6H), 1.48

(s, 3H), 1.33 (s, 36H), 1.22 (s, 3H). $^{13}C\{^1H\}$ NMR ($CDCl_3$): $\delta$ 150.6, 138.7, 138.2, 136.1, 134.2, 133.1, 129.2, 128.1, 123.8, 123.2, 121.3, 120.6, 83.6, 35.0, 31.5, 22.0, 18.8, 3.0.

**Synthesis of I-mc2 : 3-Bromo-4-methylacetophenone**

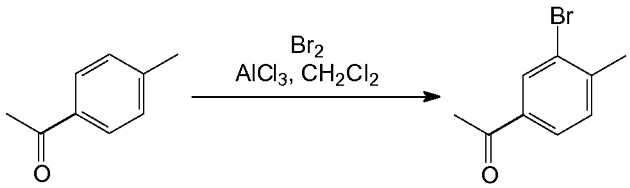


A solution of 97 g (723 mol) of 4-methylacetophenone in 100 ml of dichloromethane was added by vigorous stirring over 30 min to a suspension of 237 g (1.78 mol) of $AlCl_3$ in 700 ml of dichloromethane at 0 °C. The reaction mixture was then stirred for 10 min at this temperature, then 40.0 ml (125 g, 0.781 mol, 8% excess) of bromine was added dropwise over 1 h. The resulting mixture was stirred overnight at room temperature and then poured onto 2000 $cm^3$ of ice. The organic layer was separated, and the aqueous layer was extracted with 3 x 150 ml of dichloromethane. The combined organic extract was washed with aqueous $K_2CO_3$, dried over $K_2CO_3$, passed through a short pad of silica gel 60 (40-63 $\mu$m), and then evaporated to dryness. Crude product was distilled under vacuum to give 143 g (93%) of a colorless liquid (b.p. 125-132 °C/8 mm Hg) which completely solidified on standing at room temperature.

Anal. calc. for $C_9H_9BrO$: C, 50.73; H, 4.26. Found: C, 50.89; H, 4.12.
$^1$HNMR($CDCl_3$): $\delta$ 8.09(s, 1H, 2-H), 7.77 (d, *J* = 7.8 Hz, 1H, 6-H), 7.31 (d, *J* = 7.8 Hz, 1H, 5-H), 2.56 (s, 3H, COMe), 2.44 (s, 3H, 4-Me).

**1-[3-Bromo-4-(bromomethyl)phenyl]ethanone**

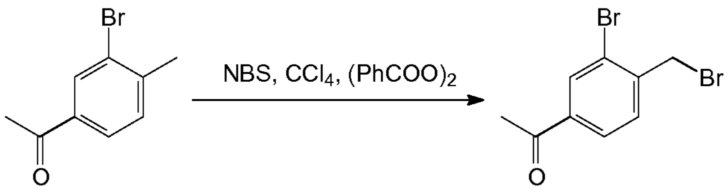


222 g (1.25 mol) of N-bromosuccinimide and 2 g of benzoyl peroxide were added to a solution of 265 g (1.24 mol) of 3-bromo-4-methylacetophenone in 2000 ml of tetrachloromethane. The formed mixture was refluxed for 2 h, then filtered through glass frit (G3), and the filtrate was evaporated to dryness. The residue was dried in vacuo to yield crude 4-acetyl-2-bromobenzyl bromide as red oil (purity ~70%) which was used without further purification.
$^1$H NMR ($CDCl_3$): $\delta$ 8.12 (s, 1H, 2-H), 7.85 (d, *J* = 7.8 Hz, 1H, 6-H), 7.54 (d, *J* = 7.8 Hz, 1H, 5-H), 4.59 (s, 2H, $CH_2Br$), 2.58 (s, 3H, COMe).

**Diethyl (4-acetyl-2-bromobenzyl)(methyl)malonate**

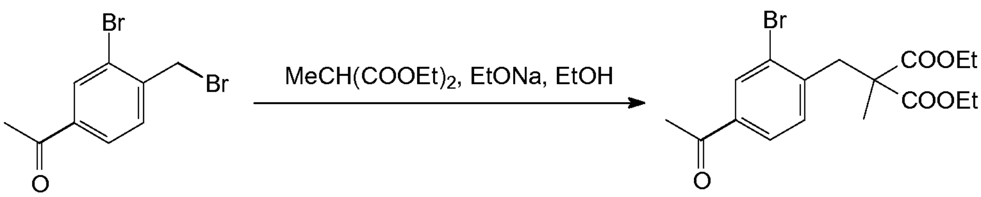

225 g (1.29 mol) of diethyl methylmalonate was added to a solution of sodium ethoxide prepared from 27.4 g (1.19 mol) of sodium metal and 1000 ml of dry ethanol. This mixture was stirred for 15 min; then, 4-acetyl-2-bromobenzyl bromide (prepared above) was added at such a rate as to maintain a gentle reflux. The obtained mixture was refluxed for 3 h, and then ethanol was distilled off at atmospheric pressure. The reaction mixture was cooled to room temperature, 2500 ml of water was added, and crude product was extracted with 3 x 200 ml of ether. The combined organic extract was dried over $Na_2SO_4$, passed through a short layer of silica gel 60 (40-63 $\mu$m), and then evaporated to dryness. The residue was distilled under vacuum to give 217 g (45%) of diethyl (4-acetyl-2-bromobenzyl)(methyl)malonate (b.p. 180-200 °C/5 mm Hg) as a slightly yellowish oil which completely solidified on standing at room temperature.

Anal. calc. for $C_{17}H_{21}BrO_5$: C, 53.00; H, 5.49. Found: C, 53.22; H, 5.25.
$^1$H NMR ($CDCl_3$): $\delta$ 8.13 (s, 1H, 3-H), 7.78 (d, *J*= 7.9 Hz, 1H, 5-H), 7.30 (d, *J* = 7.9 Hz, 1H, 6-H), 4.23 (m, 4H, OC*H*$_2$Me), 3.54 (s, 2H, ArC*H*$_2$), 2.57 (s, 3H, C(O)Me), 1.39 (s, 3H, *Me*C(COOEt)$_2$), 1.26 (t, *J* = 7.1 Hz, 6H, OCH$_2$*Me*). $^{13}$C{$^1$H} NMR ($CDCl_3$): $\delta$ 195.9, 171.2, 141.6, 136.9, 132.6, 131.3, 126.7, 126.4, 61.4, 54.6, 39.2, 26.3, 19.3, 13.7.

**3-(2-Bromo-4-ethylphenyl)-2-methylpropanoic acid**

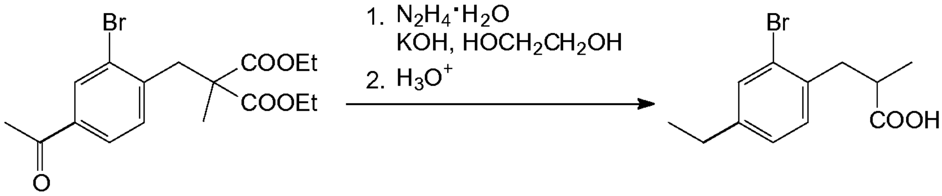


125 g of KOH was added in small portions by vigorous stirring over 30 min to a warm turbid mixture of 141 g (366 mmol) of diethyl (4-acetyl-2-bromobenzyl)(methyl)malonate, 63 ml of hydrazine hydrate and 1000 ml of ethylene glycol. The resulting slightly yellowish solution was refluxed for 5 h. Further on, the reflux condenser was replaced by a Claisen distillation head with condenser, and a mixture of water, hydrazine, and ethylene glycol was distilled off until the distillation temperature reached 195°C (eventually this reaction mixture was thus evaporated to about half its initial volume in 1 h). The residue was cooled to ca. 100 °C and then poured into 2500 ml of cold water. This mixture was acidified by 12 M HCl to pH~1, and crude product was extracted with 500 ml of dichloromethane. The aqueous layer was additionally extracted with 2 x 250 ml of dichloromethane. The combined organic extract was washed with 1000 ml of water and then evaporated to dryness. The residue was distilled under reduced pressure to give 60.2 g (61%) of 3-(2-bromo-4-ethylphenyl)-2-methylpropanoic acid (b.p. 144-172 °C/5 mm Hg) as a colorless oily liquid.

Anal. calc. for $C_{12}H_{15}BrO_2$: C, 53.15; H, 5.58. Found: C, 53.28; H, 5.74.
$^1$H NMR ($CDCl_3$): $\delta$ 11.99 (s, 1H, $CO_2$H), 7.38 (s, 1H, 3-H), 7.12 (d, *J* = 7.8 Hz, 1H, 5-H), 7.04 (d, *J* = 7.8 Hz, 1H, 6-H), 3.15 (dd, *J* = 13.5 Hz, *J* = 6.8 Hz, 1H, ArC*H*H'), 2.85-2.94 (m, 1H, C*H*(Me)$CO_2$H), 2.76 (dd, *J*= 13.5 Hz, *J* = 7.7 Hz, 1H, ArCH*H'*), 2.58 (q, *J* = 7.5 Hz, 2H, C*H*$_2$Me), 1.19-1.22 (m, 6H, CH*Me* and CH$_2$*Me*). $^{13}$C{$^1$H} NMR ($CDCl_3$): $\delta$ 182.6, 144.5, 135.4, 132.2, 131.1, 126.9, 124.5, 39.5, 38.8, 28.0, 16.6, 15.2.

**3-(2-Bromo-4-ethylphenyl)-2-methylpropanoyl chloride**

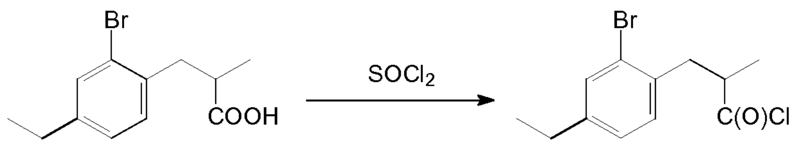


A mixture of 60.2 g (0.222 mol) of 3-(2-bromo-4-ethylphenyl)-2-methylpropanoic acid and 57 ml (0,781 mol) of thionyl chloride was stirred at room temperature for 24 h. Then the excess of thionyl chloride was distilled off, and the residue was distilled under vacuum to give 53.1 g (83%) of the title product, b.p. 132-133°C/5 mm Hg.

Anal. calc. for $C_{12}H_{14}BrClO$: C, 49.77; H, 4.87. Found: C, 49.85; H, 5.00.
$^1$H NMR ($CDCl_3$): $\delta$ 7.39 (s, 1H, 3-H), 7.13 (d, *J* = 7.8 Hz, 1H, 5-H), 7.07 (d, *J* = 7.8 Hz, 1H, 6-H), 3.19-3.36 (m, 2H, ArC*H*H' and C*H*(COCl)), 2.81 (dd, *J* = 13.2 Hz, *J*= 6.8 Hz, 1H, ArCH*H'*), 2.60 (q, *J*= 7.6 Hz, 2H, C*H*$_2$Me), 1.31 (d, *J* = 6.8 Hz, 3H, CH*Me),* 1.21 (t, *J* = 7.6 Hz, 3H, CH$_2$*Me*). $^{13}$C{$^1$H} NMR ($CDCl_3$): $\delta$ 177.0, 145.1, 134.0, 132.4, 131.3, 127.1,

124.4, 51.4, 38.9, 28.0, 16.7, 15.2.

**4-Bromo-6-ethyl-2-methylindan-1-one**

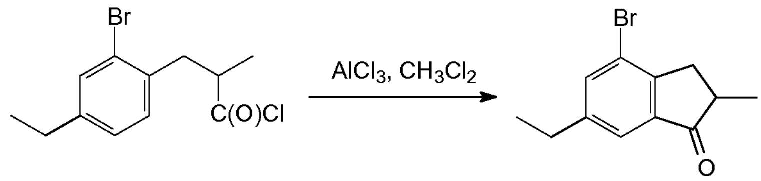


*A solution of 53.1 g (183 mmol)* of 3-(2-bromo-4-ethylphenyl)-2-methylpropanoyl chloride in 50 ml of dichloromethane was added dropwise to a stirred suspension of 30.0 g (225 mmol, 1.2 eq.) of $AlCl_3$ in 200 ml of dichloromethane at 5°C. This mixture was stirred overnight at room temperature and then poured on 500 g of crushed ice. The organic layer was separated, and the aqueous layer was extracted with 2 x 100 ml of dichloromethane. The combined organic extract was washed by aqueous $K_2CO_3$, dried over $K_2CO_3$, and then evaporated to dryness. The product was isolated by flash-chromatography on silica gel 60 (40-63 μm; eluent: hexanes-dichloromethane = 1:1, vol.). This procedure gave 43.8 g (95%) of a yellowish solid.

Anal. calc. for $C_{12}H_{13}BrO$: C, 56.94; H, 5.18. Found: C, 57.19; H, 5.36.
$^1H$ NMR ($CDCl_3$): $\delta$ 7.61 (s, 1H, 7-H), 7.53 (s, 1H, 5-H), 3.30 (dd, *J* = 17.4 Hz, *J* = 7.8 Hz, 1H, 3-C*H*H'), 2.65-2.80 (m, 3H, 2-H and C*H*$_2$Me), 2.61 (dd, *J* = 17.4 Hz, *J* = 3.6 Hz, 1H, 3-CH*H'*), 1.32 (d, *J* = 7.5 Hz, 3H, 2-Me), 1.25 (t, *J* = 7.5 Hz, 3H, $CH_2$*Me*). $^{13}C\{^1H\}$ NMR ($CDCl_3$): $\delta$ 208.6, 150.5, 146.0, 138.3, 137.3, 121.7, 121.7, 42.3, 35.5, 28.2, 16.2, 15.3.

**4-Bromo-6-ethyl-1-methoxy-2-methylindane**

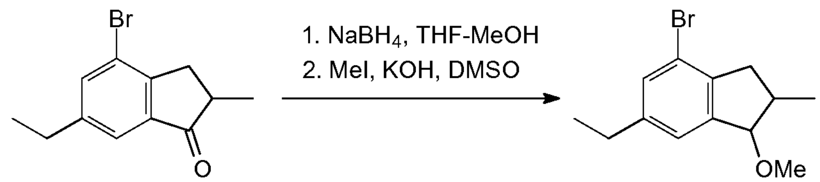


150 ml of methanol was added dropwise by vigorous stirring over 5 h to a mixture of 43.8 g (173 mmol) of 4-bromo-6-ethyl-2-methylindan-1-one and 9.8 g (259 mmol) of $NaBH_4$ in 300 ml of THF at 0-5 °C. This mixture was stirred overnight at room temperature and then evaporated under vacuum. 500 ml of dichloromethane and 500 ml of water were added to the obtained white mass, and the resulting mixture was acidified by 2 M HCl to pH~4. The organic layer was separated, and the aqueous layer was extracted with 2 x 200 ml of dichloromethane. The combined organic extract was dried over $Na_2SO_4$ and then evaporated to dryness. The resulting white solid was immediately dissolved in 370 ml of DMSO, and 45 g (802 mmol, 4.64 eq.) of KOH and 55.5 g (391 mmol, 2.26 eq.) of MeI were added. The formed mixture was stirred overnight at room temperature, then the solution was decanted from an excess of KOH, the latter was washed with 3 x 150 ml of dichloromethane, and 1500 ml of water was added to the combined extract. The organic layer was separated, and the aqueous layer was extracted with 2 x 150 ml of dichloromethane. The combined organic extract was washed with 3 x 1000 ml of water, dried over $K_2CO_3$, and evaporated to dryness. The product was isolated by flash-chromatography on silica gel 60 (40-63 μm; eluent: hexanes-dichloromethane = 2:1, vol., then 1:1, vol.). This procedure gave 40.5 g (87%) of a colorless thick oil composed of a mixture of two diastereoisomers.

Anal. calc. for $C_{13}H_{17}BrO$: C, 58.01; H, 6.37. Found: C, 58.22; H, 6.49.

*Syn*-isomer: $^1H$ NMR ($CDCl_3$): $\delta$ 7.24 (s, 1H, 5-H), 7.12 (s, 1H, 7-H), 4.55 (d, *J* = 5.7 Hz, 1H, 1-H), 3.41 (s, 3H, OMe), 2.90 (dd, *J* = 17.6 Hz, *J* = 8.9 Hz, 1H, 3-C*H*H'), 2.56-2.70 (m, 4H, 3-CHH', 2-H and C*H*$_2$Me), 1.22 (t, *J* = 7.5 Hz, 3H, C*H*$_2$Me), 1.07 (d, *J* = 6.6 Hz, 3H, 2-Me). $^{13}C\{^1H\}$ NMR ($CDCl_3$): $\delta$ 144.6, 144.5, 140.7, 130.6, 123.5, 120.1, 86.7, 57.0, 39.0, 37.9, 28.4, 15.7, 13.5.

*Anti*-isomer: $^1H$ NMR ($CDCl_3$): $\delta$ 7.26 (s, 1H, 5-H), 7.13 (s, 1H, 7-H), 4.42 (d, *J* = 4.3 Hz, 1H, 1-H), 3.45 (s, 3H, OMe), 3.18 (dd, *J*= 16.2 Hz, *J*= 7.5 Hz, 1H, 3-C*H*H'), 2.62 (q, *J* = 7.5 Hz, 2H, C*H*$_2$Me), 2.44-2.56 (m, 1H, 2-H), 2.40 (dd, *J* = 16.2 Hz, *J* = 5.2 Hz, 1H, 3-C*H*H'), 1.22 (t, *J*= 7.5 Hz, 3H, C*H*$_2$Me), 1.17 (d, *J*= 7.1 Hz, 3H, 2-Me). $^{13}C\{^1H\}$ NMR ($CDCl_3$): $\delta$ 144.9, 144.1, 140.5, 130.9, 123.6, 120.1, 92.0, 56.6, 39.3, 39.2, 28.4, 19.4, 15.6.

**4-(3,5-Di-*tert*-butylphenyl)-6-ethyl-1-methoxy-2-methylindane**

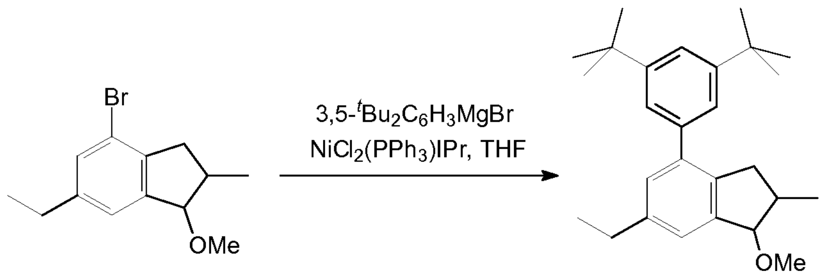


400 ml (200 mmol) of 0.5 M 3,5-di-*tert*-butylphenylmagnesium bromide in THF was added to a mixture of 1.0 g (1.28 mmol, 0.85 mol.%) of $NiCl_2(PPh_3)IPr$ and 40.5 g (151 mmol) of 4-bromo-6-ethyl-1-methoxy-2-methylindane, at such a rate as to maintain a gentle reflux. The resulting solution was refluxed for 1 h, then cooled to room temperature, and 150 ml of water was added. The major part of THF was distilled off using rotary evaporator, then 500 ml of dichloromethane and 1000 ml of 1 M HCl were added to the residue. The organic layer was separated, and the aqueous layer was extracted with 150 ml of dichloromethane. The combined organic extract was evaporated to dryness to give ca. 68 g of a slightly greenish oil. The product was isolated by flash-chromatography on silica gel 60 (40-63 μm; eluent: hexanes-dichloromethane = 2:1 vol., then 1:1 vol.). This procedure gave a colourless thick oil composed of a mixture of two diastereoisomers.

Anal. calc. for $C_{27}H_{38}O$: C, 85.66; H, 10.12. Found: C, 85.92; H, 10.37.

*Syn*-isomer: $^1H$ NMR ($CDCl_3$): $\delta$ 7.39 (t, *J* = 1.6 Hz, 1H, 4-H in 3,5-$^tBu_2C_6H_3$), 7.28 (d, *J* = 1.6 Hz, 2H, 2,6-H in 3,5-$^tBu_2C_6H_3$), 7.20 (s, 1H, 5-H in indane), 7.18 (s, 1H, 7-H in indane), 4.53 (d, *J* = 5.5 Hz, 1H, 1-H in indane), 3.48 (s, 3H, OMe), 2.92 (dd, *J*= 15.5 Hz, *J*= 7.1 Hz, 1H, 3-C*H*H' in indane), 2.79 (dd, *J*= 15.5 Hz, *J*= 6.6 Hz, 1H, 3-CHH' in indane), 2.71 (q, *J* = 7.5 Hz, 2H, C*H*$_2$Me), 2.50-2.63 (m, 1H, 2-H in indane), 1.36 (s, 18H, 3,5-$^t$*Bu*$_2C_6H_3$), 1.29 (t, *J*= 7.5 Hz, 3H, C*H*$_2$Me), 1.08 (d, *J*= 6.8 Hz, 3H, 2-Me in indane). $^{13}C\{^1H\}$ NMR ($CDCl_3$): $\delta$ 150.4, 143.57, 142.7, 140.1, 139.6, 138.6, 128.2, 123.3, 123.0, 120.7, 86.4, 56.9, 39.2, 38.0, 34.9, 31.5, 28.8, 15.9, 13.6.

*Anti*-isomer: $^1H$ NMR ($CDCl_3$): $\delta$ 7.39 (t, *J* = 1.8 Hz, 1H, 4-H in 3,5-$^tBu_2C_6H_3$), 7.28 d, *J*= 1.8 Hz, 2H, 2,6-H in 3,5-$^tBu_2C_6H_3$), 7.21 (s, 1H, 5-H in indane), 7.18 (s, 1H, 7-H in indane), 4.41 (d, *J*= 3.9 Hz, 1H, 1-H in indane), 3.51 (s, 3H, OMe), 3.30 (dd, *J*= 15.3 Hz, *J*= 6.8 Hz, 1H, 3-C*H*H' in indane), 2.71 (q, *J*= 7.5 Hz, 2H, C*H*$_2$Me), 2.40-2.55 (m, 2H, 2-Me and 3-CHH' in indane), 1.36 (s, 18H, 3,5-$^t$*Bu*$_2C_6H_3$), 1.28 (t, *J* = 7.5 Hz, 3H, C*H*$_2$Me), 1.12 (d, *J* = 6.8 Hz, 3H, 2-Me in indane). $^{13}C\{^1H\}$ NMR ($CDCl_3$): $\delta$ 150.4, 143.0 (two resonances), 140.1, 139.6, 138.1, 128.5, 123.4, 122.9, 120.7, 91.5, 56.7, 40.0, 38.1, 34.9, 31.5, 28.8, 19.2, 15.9.

**2-methylMethyl-5-ethyl-7-(3,5-Di-*tert*-butylphenyl)-1*H*-indene**

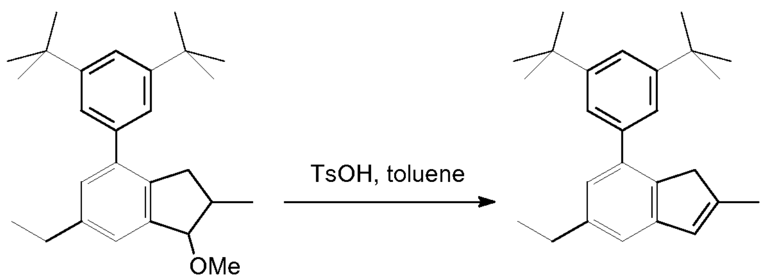


0.75 g of TsOH was added to a solution of 4-(3,5-di-*tert*-butylphenyl)-6-ethyl-1-methoxy-2-methylindane (as prepared above) in 450 ml of toluene. The resulting solution was refluxed using Dean-Stark head for 15 min, then cooled to room temperature, and finally washed with 200 ml of 10% aqueous $NaHCO_3$. The organic layer was separated, and the aqueous layer was extracted with 150 ml of dichloromethane. The combined organic extract was evaporated to dryness, and the product was isolated by flash-chromatography on silica gel 60 (40-63 μm; eluent: hexanes-dichloromethane = 10:1, vol.). This procedure gave 52.0 g (99%) of crude 7-(3,5-di-*tert*-butylphenyl)-5-ethyl-2-methyl-1*H*-indene as a white crystalline solid. This material was re-crystallized from 100 ml of n-hexane to give 43.8 g of a white

crystalline product. The following crystallization of the evaporated mother liquor from 10 ml of n-hexane gave the second crop (6.30 g) of the analytically pure product.

Anal. calc. for $C_{26}H_{34}$: C, 90.11; H, 9.89. Found: C, 90.35; H, 10.05.

$^1$H NMR ($CDCl_3$): $\delta$ 7.41 (t, *J* = 1.8 Hz, 1H, 4-H in 3,5-$^t$Bu$_2$C$_6$H$_3$), 7.37 (d, *J* = 1.8 Hz, 2H, 2,6-H in 3,5-$^t$Bu$_2$C$_6$H$_3$), 7.10 (s, 1H, 6-H in indene), 7.00 (s, 1H, 4-H in indene), 6.50 (q, *J*= 1.3 Hz, 1H, 3-H in indene), 3.34 (s, 2H, $CH_2$ in indene), 2.72 (q, *J*= 7.6 Hz, 2H, C*H*$_2$Me), 2.13 (s, 3H, 2-Me in indene), 1.38 (s, 18H, 3,5-$^t$*Bu*$_2$C$_6$H$_3$), 1.29 (t, *J* = 7.6 Hz, 3H, CH$_2$*Me*). $^{13}$C{$^1$H} NMR ($CDCl_3$): $\delta$ 150.5, 146.7, 146.5, 143.2, 140.7, 138.3, 138.3, 127.2, 124.1, 122.8, 120.8, 118.3, 42.5, 35.0, 31.6, 29.0, 16.8, 16.1.

**Bis[2-methyl-4-(3,5-di-tert-butylphenyl)-6-ethyl-1H-inden-1-yl](dimethyl)silane**

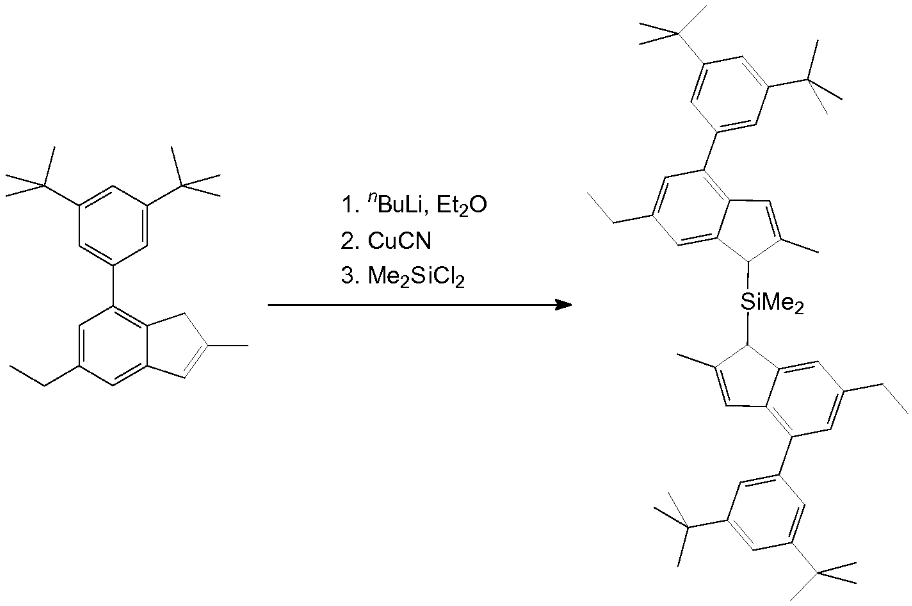


20.0 ml (50.0 mmol) of 2.5 M $^n$BuLi in hexanes was added in one portion to a solution of 17.3 g (50.0 mmol) of 2-methyl-7-(3,5-di-*tert*-butylphenyl)-5-ethyl-1*H*-indene in 250 ml of ether at -50 °C. This mixture was stirred overnight at room temperature, then the resulting yellow solution was cooled to -40 °C, and 200 mg of CuCN was added. The obtained mixture was stirred for 30 min at -25 °C, and then 3.23 g (25.0 mmol) of dichlorodimethylsilane was added in one portion. Then this mixture was stirred overnight at ambient temperature, then filtered through a pad of silica gel 60 (40-63 µm) which was additionally washed by 2 x 50 ml of dichloromethane. The combined filtrate was evaporated under vacuum, and the residue was dried in vacuo at elevated temperature. This procedure gave 18.9 g (~100%) of the title compound of >90% purity. On the evidence ofNMR spectroscopy this product is a ca. 1:1 mixture of the stereoisomers. The obtained yellowish glassy product was used without further purification.

$^1$H NMR ($CDCl_3$): $\delta$ 7.44-7.53 (m), 7.39 (m), 7.31 (m), 7.23 (m), 7.22 (m), 6.88 (m), 6.87 (m), 3.87 (s), 2.81 (q, *J* = 7.4 Hz), 2.33 (s), 2.32 (s), 1.48 (s), 1.36 (t, *J* = 7.4 Hz), -0.07 (s), -0.12 (s).

***Rac*-dimethylsilanediylbis[2-methyl-4-(3,5-di-*tert*-butylphenyl)-6-ethyl-inden-1-yl]zirconium dichloride (I-mc2)**

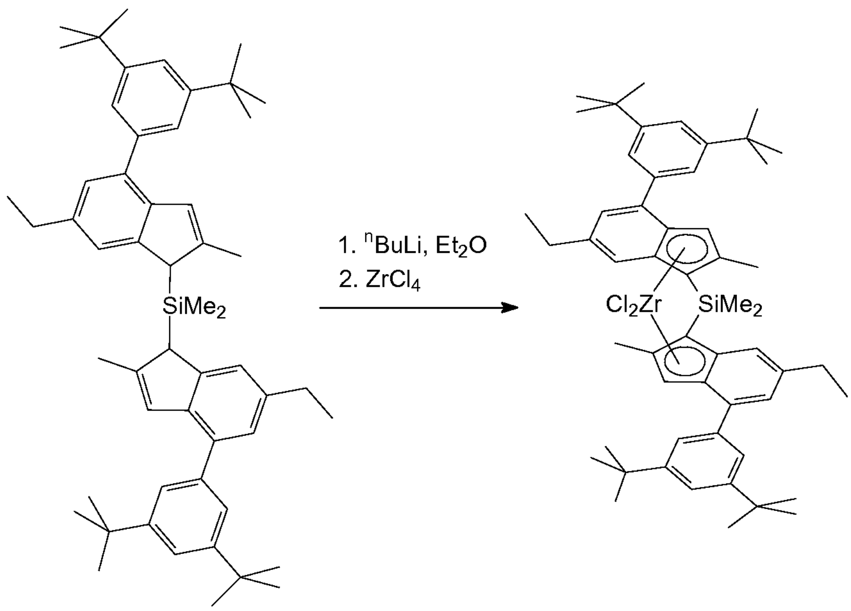


20.0 ml (50.0 mmol) of 2.5 M$^{n}$BuLi in hexanes was added in one portion to a solution of 18.9 g (25.0 mmol) of bis[2-methyl-4-(3,5-di-*tert*-butylphenyl)-6-ethyl-1*H*-inden-1-yl](dimethyl)silane (as prepared above) in 250 ml of ether cooled to -30 °C. This mixture was stirred overnight at room temperature. The resulting light-orange solution was cooled to -50 °C, and 5.83 g (25.0 mmol) of $ZrCl_4$ was added. The reaction mixture was stirred for 24 h giving yellow-orange suspension which was then evaporated to dryness. The residue was poured into 200 ml of hot toluene. The obtained hot mixture was filtered through glass frit (G4). On the evidence of NMR spectroscopy the obtained filtrate included a ca. 1 to 1 mixture of *rac*- and *meso*-zirconocenes. Yellow crystals precipitated from this filtrate overnight at room temperature were collected and dried in vacuum. This procedure gave 2.80 g of pure *rac*-zirconocene. The mother liquor was evaporated to ca. 75 ml. Crystals precipitated from this filtrate overnight at room temperature were collected and dried in vacuo. This procedure gave 9.07 g of a ca. 55 to 45 mixture of *rac*- and *meso*-zirconocenes. The mother liquor was evaporated to ca. 15 ml. Crystals precipitated from this filtrate overnight at room temperature were collected and dried in vacuo. This procedure gave 1.73 g of *meso*-zirconocene contaminated with ca. 1% of *rac*-isomer. The mother liquor was evaporated to dryness. To the residue ca. 25 ml of n-hexane was added. Crystals precipitated from this solution for 2 h at room temperature were collected and dried in vacuo. This procedure gave 3.36 g of a ca. 3 to 7 mixture of *rac*- and *meso*-zirconocenes. Thus, the overall yield of *rac*- and *meso*-zirconocenes isolated in this synthesis was 17.0 g (75%).

*Rac*-zirconocene (I-mc2).

Anal. calc. for $C_{54}H_{70}Cl_2SiZr$: C, 71.32; H, 7.76. Found: C, 71.58; H, 7.90.
$^{1}$H NMR ($CDCl_3$): $\delta$ 7.52 (d, *J* = 1.8 Hz, 4H), 7.42 (m, 2H), 7.39 (t, *J* = 1.8 Hz, 2H), 7.27 (m, 2H), 6.91 (m, 2H), 2.69 (q, *J* = 7.4 Hz, 4H), 2.26 (s, 6H), 1.34 (s, 6H), 1.32 (s, 36H), 1.24 (t, *J* = 7.4 Hz, 6H). $^{13}C\{^{1}H\}$ NMR ($CDCl_3$): $\delta$ 150.94, 141.94, 139.46, 138.66, 134.74, 130.86, 128.45, 128.21, 123.29, 122.71, 121.44, 121.23, 82.24, 35.05, 31.55, 29.43, 18.50, 15.07, 2.88.

*Meso*-zirconocene (meso I-mc2).

Anal. found: C, 71.43; H, 7.89.
$^{1}$H NMR ($CDCl_3$): $\delta$ 7.48 (m, 4H), 7.39 (m, 4H), 7.00 (m, 2H), 6.75 (m, 2H), 2.55 (m, 4H), 2.43 (s, 6H), 1.48 (s, 3H), 1.33 (s, 36H), 1.22 (s, 3H), 1.17 (t, *J* = 7.4 Hz, 6H). $^{13}C\{^{1}H\}$ NMR ($CDCl_3$): $\delta$ 150.68, 140.68, 138.74, 138.43, 135.81, 133.20, 129.15, 127.06, 123.22, 122.08, 121.38, 121.30, 83.95, 35.01, 31.52, 29.40, 18.67, 14.99, 3.04, 2.94.

**I-mc3:**

**1-(Chloromethyl)-4-isopropylbenzene**

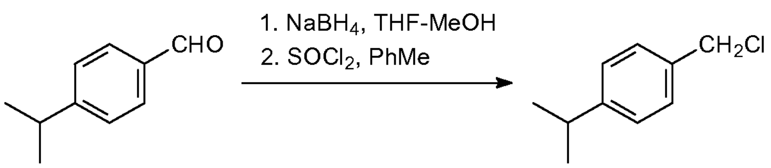


500 ml of methanol was added dropwise by vigorous stirring over 5 h to a mixture of 148 g (1.0 mol) 4-isopropylbenzaldehyde and 37.8 g (1.0 mol) of $NaBH_4$ in 1000 ml of THF at 0-5 °C. This mixture was stirred overnight at room temperature and then evaporated under vacuum. The residue was acidified with 1200 ml of 2 M HCl to pH~1, and the formed (4-isopropylphenyl)methanol was extracted with 3 x 400 ml of dichloromethane. The combined organic extract was dried over $Na_2SO_4$ and evaporated to dryness. To the residue dissolved in 1000 ml of dichloromethane 73 ml (1.0 mol) of thionyl chloride was added dropwise at +5°C. The resulting solution was stirred at room temperature overnight, evaporated to dryness, and then the residue was dissolved in 750 ml dichloromethane. The formed solution was washed by 250 ml of water. The organic layer was separated, the aqueous layer was extracted with 2 x 150 ml of dichloromethane. The combined organic extract was dried over $Na_2SO_4$, passed through a short pad of silica gel 60 (40-63 μm), and evaporated to dryness. Crude product was distilled under vacuum to give 142 g (84%) of a colorless liquid, b.p. 107-112 °C/15 mm Hg.

Anal. calc. for $C_{10}H_{13}Cl$: C, 71.21; H, 7.77. Found: C, 71.25; H, 7.63.
$^1$H NMR ($CDCl_3$): $\delta$7.31 (d, $J$ = 8.1 Hz, 2H), 7.21 (d, $J$ = 8.1 Hz, 2H), 4.56 (s, 2H), 2.96-2.85 (m, 1H), 1.24 (d, $J$ = 6.8 Hz, 6H).

**3-(4-Isopropylphenyl)-2-methylpropanoyl chloride**

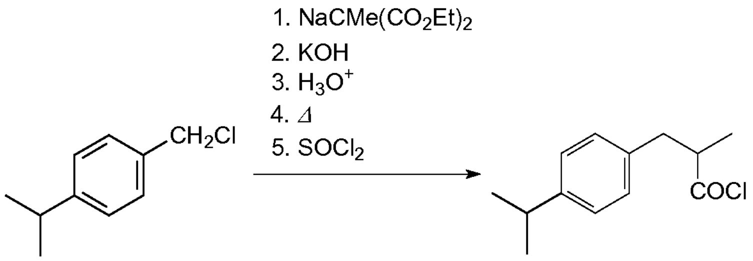


120 g (0.691 mol) of diethyl methylmalonate was added to a solution of sodium ethoxide prepared from 19.0 g (0.827 mol) of sodium metal and 500 ml of anhydrous ethanol. This mixture was stirred for 15 min, then 117 g (0.691 mmol) of 1-(chloromethyl)-4-isopropylbenzene was added at such a rate as to maintain a gentle reflux. This mixture was refluxed for 3 h and then cooled to room temperature. A solution of 138 g of KOH in 370 ml of water was added. The obtained mixture was refluxed for 4 h to saponificate the ester formed. Ethanol and water were distilled off until the temperature reached 95 °C, and 2000 ml of water and then 12 M HCl (to pH~1) were added to the residue. The precipitated substituted methylmalonic acid was filtered off, washed with water, and partially dried on the filter. Crude 3-(4-isopropylphenyl)-2-methylpropanoic acid was obtained after decarboxylation of this substituted methylmalonic acid at 180°C. A mixture of this acid and 175 ml of thionyl chloride was stirred at room temperature for 24 h, and then an excess of thionyl chloride was distilled off. The residue was distilled under vacuum to give 135 g (*87*%) of the title compound, b.p. 114-119°C/5 mm Hg.

Anal. calc. for $C_{13}H_{17}ClO$: C, 69.48; H, 7.62. Found: C, 69.63; H, 7.80.
$^1$H NMR ($CDCl_3$): $\delta$7.14-7.17 (m, 2H, 2,6-H), 7.08-7.11 (m, 2H, 3,5-H), 3.09-3.17 (m, 2H, ArC*H*H' and C*H*C(O)Cl), 2.87 (sep, $J$ = 7.0 Hz, 1H, C*H*Me$_2$), 2.68-2.74 (m, 1H, ArCH*H'*), 1.26 (d, $J$ = 7.0 Hz, 3H, CH*Me*), 1.23 (d, $J$ = 7.0 Hz, 6H, CH*Me$_2$*). $^{13}$C{$^1$H} NMR ($CDCl_3$): $\delta$ 177.1, 147.4, 134.8, 128.9, 126.6, 53.3, 38.8, 33.7, 23.9, 16.6.

**6-Isopropyl-2-methylindan-1-one**

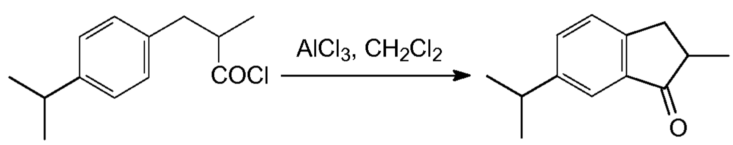


To a suspension of 192 g (1.44 mol, 1.2 eq.) of $AlCl_3$ in 1000 ml of dichloromethane a solution of *270 g (1.2 mol)* of 3-(4-isopropylphenyl)-2-methylpropanoyl chloride in 200 ml of dichloromethane was added dropwise by vigorous stirring at 5°C. The obtained mixture was stirred overnight at room temperature and then poured on 1500 g of the crushed ice. The organic layer was separated, and the aqueous layer was extracted with 3 x 100 ml of dichloromethane. The combined organic extract was washed by aqueous $K_2CO_3$, dried over $K_2CO_3$, passed through a short pad of silica gel 60 (40-63 μm), and then evaporated to dryness. Crude product was distilled under vacuum to give 200 g (89%) of as a colorless liquid, b.p. 125-131 °C/5 mm Hg.

Anal. calc. for $C_{13}H_{16}O$: C, 82.94; H, 8.57. Found: C, 82.92; H, 8.66.
$^1$H NMR ($CDCl_3$): $\delta$ 7.63 (d, *J* = 1.1 Hz, 1H, 7-H), 7.46 (dd, *J* = 7.9 Hz, *J* = 1.8 Hz, 1H, 5-H), 7.36 (d, *J* = 7.9 Hz, 1H, 5-H), 3.35 (dd, *J* = 17.0 Hz, *J* = 7.9 Hz, 1H, 3-C*H*H'), 2.97 (sep, *J* = 7.0 Hz, 1H, C*H*Me$_2$), 2.65-2.74 (m, 2H, 3-CH*H'* and 2-H), 1.30 (d, *J* = 7.3 Hz, 3H, CH*Me*), 1.26 (d, *J* = 7.0 Hz, 6H, CH*Me$_2$*). $^{13}$C{$^1$H} NMR ($CDCl_3$): $\delta$ 209.5, 151.2, 148.4, 136.4, 133.7, 126.2, 121.0, 42.2, 34.5, 33.8, 23.9, 16.2.

**4,7-Dibromo-6-isopropyl-2-methylindan-1-one**

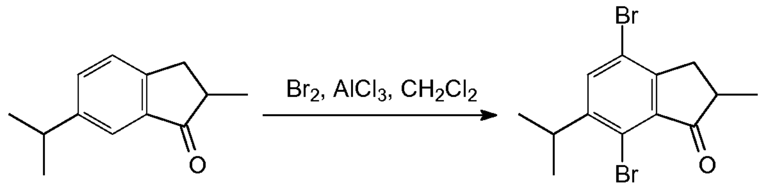


a A solution of 230 g (1.22 mol) of 6-isopropyl-2-methylindan-1-one in 700 ml of dichloromethane was added dropwise with vigorous stirring over 1 h to a suspension of 416 g (3.12 mol, 2.55 eq.) of $AlCl_3$ in 1100 ml of dichloromethane at 0 °C. The reaction mixture was stirred for 10 min at this temperature, then 2 g of iron powder was added. Further on, 402 g (2.52 mol, 2.07 eq.) of bromine was added dropwise for 1 h. The resulting mixture was stirred overnight at room temperature and then poured onto 4000 cm$^3$ of the crushed ice. The organic layer was separated, and the aqueous layer was extracted with 3 x 400 ml of dichloromethane. The combined organic extract was washed with aqueous $K_2CO_3$, dried over $K_2CO_3$, passed through a short pad of silica gel 60 (40-63 μm), and then evaporated to dryness. The resultant dark-red oily liquid was distilled under vacuum: a broad-boiling fraction with b.p. 140-200 °C/6 mm Hg was collected to give 357 g of crude product. This crude product was dissolved in 1200 ml of hot hexane, and the formed solution was kept in a -30 °C freezer over 2 days. A yellowish solid precipitate formed, which was collected and dried in vacuo. This procedure gave 275 g (65%) of 4,7-dibromo-6-isopropyl-2-methylindan-1-one. The mother liquor was evaporated to dryness to give 97.9 g of a mixture (~80% purity) of 4-bromo-6-isopropyl-2-methylindan-1-one and 4,7-dibromo-6-isopropyl-2-methylindan-1-one in ratio equal to ca. 1 to 0.43, i.e. it contains ca. 0.235 mol of 4-bromo-6-isopropyl-2-methylindan-1-one and 0.101 mol of 4,7-dibromo-6-isopropyl-2-methylindan-1-one. This mixture was dissolved in 130 ml of dichloromethane and then brominated via the above-described protocol using 78.5 g of $AlCl_3$ in 200 ml of dichloromethane, 1 g of iron powder and 40.0 g of bromine. This procedure gave 59.3 g of 4,7-dibromo-6-isopropyl-2-methylindan-1-one. Thus, 4,7-dibromo-6-isopropyl-2-methylindan-1-one was obtained in 79% total yield.

Anal. calc. for $C_{13}H_{14}Br_2O$: C, 45.12; H, 4.08. Found: C, 45.42; H, 4.31.
$^1$H NMR ($CDCl_3$): $\delta$ 7.64 (s, 1H, 5-H), 3.59 (sep, *J* = 6.9 Hz, 1H, C*H*Me$_2$), 3.25 (dd, *J* = 17.4 Hz, *J* = 8.0 Hz, 1H, 3-C*H*H'), 2.69-2.82 (m, 1H, 2-H), 2.57 (dd, *J* = 17.4 Hz, *J* = 4.1 Hz, 1H, 3-CH*H'*), 1.33 (d, *J* = 7.2 Hz, 3H, 2-Me), 1.25 (d, *J* = 6.9 Hz, 6H, CH*Me$_2$*). $^{13}$C{$^1$H} NMR ($CDCl_3$): $\delta$ 205.7, 153.0, 149.9, 135.6, 135.1, 121.2, 119.9, 42.8, 34.6, 31.4, 22.7, 16.2.

**4,7-Dibromo-6-isopropyl-1-methoxy-2-methylindane**

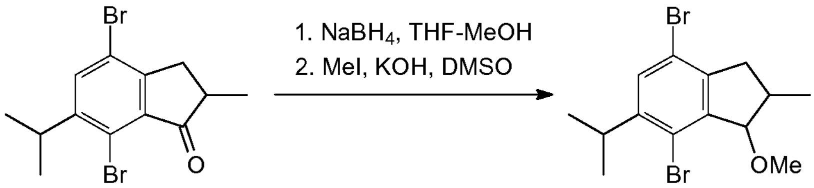


300 ml of methanol was added dropwise over 5 h with vigorous stirring to a mixture of 167 g (0.483 mol) of 4,7-dibromo-6-isopropyl-2-methylindan-1-one and 27.7 g (0.732 mol) of $NaBH_4$ in 600 ml of THF at 0-5 °C. This mixture was stirred overnight at room temperature and then evaporated under vacuum. The residue was acidified by 2 M HCl to pH~5, and the formed 4-bromo-6-isopropyl-2-methylindan-1-ol was extracted with 3 x 300 ml of dichloromethane. The combined organic extract was dried over $Na_2SO_4$ and evaporated to dryness. The residue was immediately dissolved in 1050 ml of DMSO, then 127 g (2.26 mol, 4.7 eq.) of KOH and 157 g (1.1 mol, 2.28 eq.) of iodomethane were added. This mixture was stirred for 5 h at ambient temperature and then poured into 3000 ml of water. Crude product was extracted with 3 x 500 ml of dichloromethane. The combined organic extract was washed with 7 x 1000 ml of water, dried over $Na_2SO_4$, and then evaporated to dryness. Crude product was distilled under vacuum to give 174 g (99%) of a colorless liquid (b.p. 148-162 °C/5 mm Hg) containing a mixture of two stereoisomers.

Anal. calc. for $C_{14}H_{18}Br_2O$: C, 46.44; H, 5.01. Found: C, 46.63; H, 5.18.

*Syn*-isomer: $^1$H NMR ($CDCl_3$): $\delta$ 7.29 (s, 1H, 5-H), 4.66 (d, *J* = 5.5 Hz, 1H, 1-H), 3.55 (s, 3H, OMe), 3.36 (sep, *J* = 6.9 Hz, 1H, C*H*Me$_2$), 2.95 (dd, *J* = 16.0 Hz, *J* = 7.8 Hz, 1H, 3-C*H*H'), 2.75 (dd, *J* = 16.0 Hz, *J* = 9.6 Hz, 1H, 3-CH*H'*), 2.38-2.53 (m, 1H, 2-H), 1.25 (d, *J* = 7.1 Hz, 3H, 2-Me), 1.22 (d, *J* = 6.9 Hz, 3H, CH*Me*Me'), 1.20 (d, *J* = 6.9 Hz, 3H, CHMe*Me'*). $^{13}$C{$^1$H} NMR ($CDCl_3$): $\delta$147.7, 145.6, 143.8, 129.7, 121.4, 119.1, 88.0, 59.5, 40.7, 38.7, 32.5, 22.9, 22.8, 13.5.

Anti-isomer: $^1$H NMR ($CDCl_3$): $\delta$ 7.32 (s, 1H, 5-H), 4.48 (s, 1H, 1-H), 3.46 (s, 3H, OMe), 3.25-3.43 (m, 2H, C*H*Me$_2$ and 3-C*H*H'), 2.52-2.63 (m, 1H, 2-H), 2.49 (dd, *J* = 16.7 Hz, *J* = 1.6 Hz, 1H, 3-CH*H'*), 1.22 (d, 6H, *J* = 6.9 Hz, CH*Me$_2$*), 1.05 (d, *J* = 7.3 Hz, 3H, 2-Me). $^{13}$C{$^1$H} NMR ($CDCl_3$): $\delta$ 148.0, 143.2, 143.1, 130.1, 122.0, 119.7, 93.4, 57.1, 40.7, 36.1, 32.4, 22.9, 22.8, 19.9.

**1-methoxy-2-methyl-4-bromo-6-isopropylindane**

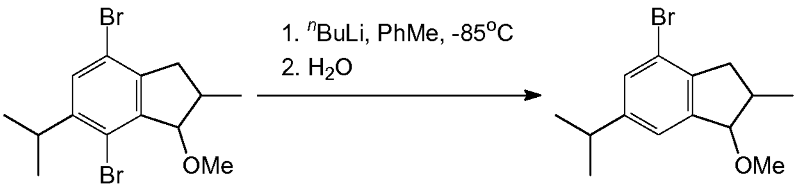


46.0 ml (0.115 mol) of 2.5 M $^n$BuLi in hexanes was added dropwise over 30 min with vigorous stirring to a solution of 41.4 g (0.114 mol) of 4,7-dibromo-6-isopropyl-1-methoxy-2-methylindane in 250 ml of toluene at -85 °C. The resulting mixture was allowed to warm to

-30 °C/-35 °C and stirred for 30 min at this temperature. The reaction was quenched by addition of 150 ml of water. The organic layer was separated, and the aqueous layer was extracted with 100 ml of dichloromethane. The combined organic extract was dried over $K_2CO_3$ and then passed through a short pad of silica gel 60 (40-63 µm). The silica gel layer was additionally washed with 50 ml of dichloromethane. The combined organic eluate was evaporated to dryness to give 34.6 g (~100% yield, the only impurity is toluene) of 4-bromo-6-isopropyl-1-methoxy-2-methylindane as a slightly yellowish liquid. The $^1$H and $^{13}$C NMR spectra were identical to those reported above.

Anal. calc. for $C_{14}H_{19}BrO$: C, 59.37; H, 6.76. Found: C, 59.13; H, 6.88.

*Syn*-isomer: $^1$H NMR ($CDCl_3$): $\delta$ 7.26 (s, 1H, 5-H), 7.15 (s, 1H, 7-H), 4.56 (d, *J* = 5.5 Hz, 1H, 1-H), 3.41 (s, 3H, OMe), 2.81-2.97 (m, 2H, 3-CH*H'* and CH*Me$_2$*), 2.57-2.70 (m, 2H, 3-CH*H'* and 2-H), 1.23 (d, *J* = 6.8 Hz, 6H, CH*Me$_2$*), 1.07 (d, *J* = 6.6 Hz, 3H, 2-Me). $^{13}$C{$^1$H} NMR ($CDCl_3$): $\delta$ 149.4, 144.5, 140.8, 129.3, 122.0, 120.2, 86.8, 57.0, 39.0, 37.9, 33.9, 24.1, 24.0, 13.5.

*Anti*-isomer: $^1$H NMR ($CDCl_3$): $\delta$ 7.28 (s, 1H, 5-H), 7.16 (s, 1H, 7-H), 4.42 (d, *J* = 4.3 Hz, 1H, 1-H), 3.46 (s, 3H, OMe), 3.18 (dd, *J* = 16.2 Hz, *J* = 7.8 Hz, 1H, 3-C*H*H'), 2.88 (sep, *J* = 6.9 Hz, 1H, CH*Me$_2$*), 2.44-2.57 (m, 1H, 2-H), 2.40 (dd, *J* = 16.2 Hz, *J* = 5.5 Hz, 1H, 3-C*H*H'), 1.23 (d, *J* = 6.9 Hz, 6H, CH*Me$_2$*), 1.18 (d, *J* = 6.8 Hz, 3H, 2-Me). $^{13}$C{$^1$H} NMR ($CDCl_3$): $\delta$ 149.7, 144.1, 140.6, 129.5, 122.2, 120.1, 92.1, 56.6, 39.33, 39.25, 33.9, 24.1, 23.9, 19.4.

**1-methoxy-2-methyl-4-(3,5-Di-*tert*-butylphenyl)-6-isopropylindane**

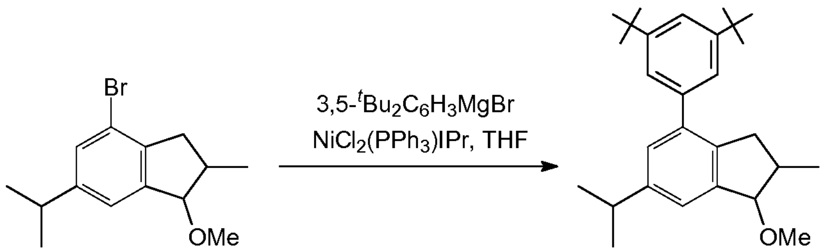


400 ml (200 mmol) of 0.5 M 3,5-di-*tert*-butylphenylmagnesium bromide in THF was added to a mixture of 1.0 g (1.28 mmol) of $NiCl_2(PPh_3)$IPr and 37.6 g (133 mmol) of 4-bromo-6-isopropyl-1-methoxy-2-methylindane at such a rate as to maintain a gentle reflux. The resulting solution was refluxed for 1 h, then cooled to room temperature, and 150 ml of water was added. The major part of THF was distilled off on rotary evaporator, then 500 ml of dichloromethane and 1000 ml of 1 M HCl were added to the residue. The organic layer was separated, and the aqueous layer was extracted with 150 ml of dichloromethane. The combined organic extract was evaporated to dryness to give a slightly greenish oil. The product was isolated by flash chromatography on silica gel 60 (40-63 μm; eluent: hexanes-dichloromethane = 2:1 vol., then 1:1 vol.). This procedure gave 4-(3,5-di-*tert*-butylphenyl)-6-isopropyl-1-methoxy-2-methylindane as colorless thick oil which slowly crystallized at room temperature. The product is a mixture of two stereoisomers.

Anal. calc. for $C_{28}H_{40}O$: C, 85.66; H, 10.27. Found: C, 85.86; H, 10.43.

*Syn*-isomer: $^1$H NMR ($CDCl_3$): $\delta$ 7.39 (t, *J* = 1.8 Hz, 1H, 4-H in 3,5-$^t$Bu$_2$C$_6$H$_3$), 7.29 (d, *J* = 1.8 Hz, 2H, 2,6-H in 3,5-$^t$Bu$_2$C$_6$H$_3$), 7.23 (m, 1H, 5-H in indane), 7.20 (m, 1H, 7-H in indane), 4.53 (d, *J* = 5.5 Hz, 1H, 1-H), 3.48 (s, 3H, OMe), 2.98 (sep, *J* = 6.9 Hz, 1H, C*H*Me$_2$), 2.92 (dd, *J* = 15.5 Hz, *J* = 7.1 Hz, 1H, 3-C*H*H'), 2.79 (dd, *J* = 15.5 Hz, *J* = 6.7 Hz, 1H, 3-CH*H'*), 2.49-2.65 (m, 1H, 2-H), 1.37 (s, 18H, 3,5-$^t$*Bu*$_2$C$_6$H$_3$), 1.30 (d, *J* = 6.9 Hz, 6H, CH*Me*$_2$), 1.09 (d, *J* = 6.8 Hz, 3H, 2-Me in indane). $^{13}$C{$^1$H} NMR ($CDCl_3$): $\delta$ 150.4, 147.4, 143.5, 140.2, 139.5, 138.7, 126.9, 123.0, 121.8, 120.7, 86.4, 56.9, 39.1, 38.0, 34.9, 34.1, 31.5, 24.3, 24.2, 13.6.

*Anti*-isomer: $^1$H NMR ($CDCl_3$): $\delta$ 7.39 (t, *J* = 1.8 Hz, 1H, 4-H in 3,5-$^t$Bu$_2$C$_6$H$_3$), 7.28 (d, *J* = 1.8 Hz, 2H, 2,6-H in 3,5-$^t$Bu$_2$C$_6$H$_3$), 7.24 (m, 1H, 5-H in indane), 7.20 (m, 1H, 7-H in indane), 4.53 (d, *J* = 3.9 Hz, 1H, 1-H), 3.52 (s, 3H, OMe), 3.29 (dd, *J* = 15.3 Hz, *J* = 7.1 Hz, 1H, 3-C*H*H'), 2.98 (sep, *J* = 6.9 Hz, 1H, C*H*Me$_2$), 2.40-2.56 (m, 2H, 3-CH*H'* and 2-H), 1.37 (s, 18H, 3,5-$^t$Bu$_2$C$_6$H$_3$), 1.30 (d, *J* = 6.9 Hz, 6H, CH*Me*$_2$), 1.13 (d, *J* = 6.8 Hz, 3H, 2-Me in indane). $^{13}$C{$^1$H} NMR ($CDCl_3$): $\delta$ 150.4, 147.7, 142.9, 140.2, 139.6, 138.2, 127.2, 122.9, 121.8, 120.7, 91.6, 56.6, 40.0, 38.1, 34.9, 34.1, 31.5, 24.4, 24.1, 19.2.

**2-methyl-5-isopropyl-7-(3,5-Di-*tert*-butylphenyl)-1*H*-indene**

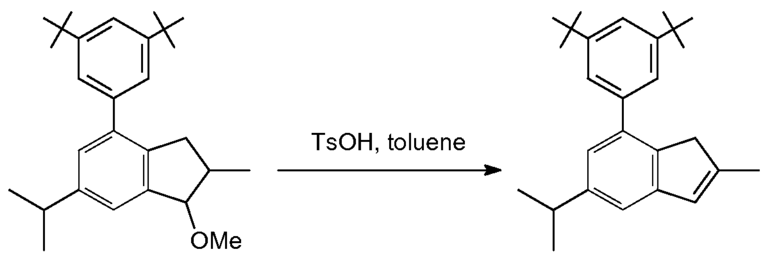


1.0 g of TsOH was added to a solution of 4-(3,5-di-*tert*-butylphenyl)-6-isopropyl-1-methoxy-2-methylindane (as prepared above) in 450 ml of toluene, and the resulting solution was refluxed using Dean-Stark head for 15 min. After that it was cooled to room temperature and washed by 200 ml of 10% aqueous $NaHCO_3$. The organic layer was separated, and the aqueous layer was extracted with 200 ml of dichloromethane. The combined organic extract was evaporated to dryness, and the product was isolated by flash chromatography on silica gel 60 (40-63 μm; eluent: hexanes-dichloromethane = 10:1, vol.). This procedure gave 46.7 g (98%) of 7-(3,5-di-*tert*-butylphenyl)-5-isopropyl-2-methyl-1*H*-indene as a yellowish glass which slowly crystallized at room temperature.

Anal. calc. for $C_{27}H_{36}$: C, 89.94; H, 10.06. Found: C, 90.24; H, 10.31.

$^1H$ NMR ($CDCl_3$): $\delta$ 7.41 (t, $J$ = 1.4 Hz, 1H, 4-H in 3,5-$^tBu_2C_6H_3$), 7.38 (d, $J$ = 1.4 Hz, 2H, 2,6-H in 3,5-$^tBu_2C_6H_3$), 7.14 (s, 1H, 6-H in indene), 7.02 (s, 1H, 4-H in indene), 6.51 (d, $J$ = 1.5 Hz, 1H, 3-H in indene), 3.34 (s, 2H, $CH_2$ in indene), 2.99 (sep, $J$ = 6.9 Hz, 1H, C*H*Me$_2$), 2.12 (s, 3H, 2-Me in indene), 1.38 (s, 18H, 3,5-$^tBu_2C_6H_3$), 1.31 (d, 6H, $J$ = 6.9 Hz, CH*Me$_2$*). $^{13}C\{^1H\}$ NMR ($CDCl_3$): $\delta$ 150.5 (two resonances), 147.9, 146.7, 146.5, 140.8, 138.5, 138.3, 127.3, 122.8, 120.8, 116.7, 42.5, 35.0, 34.3, 31.6, 24.4, 16.8.

**Bis[2-methyl-4-(3,5-di-*tert*-butylphenyl)-6-isopropyl-1*H*-inden-1-yl] (dimethyl)silane**

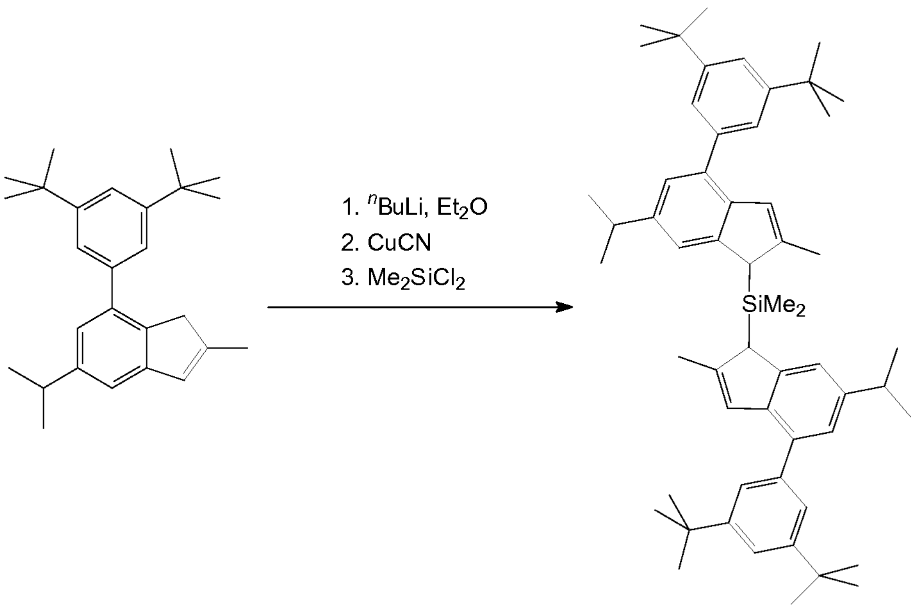


20.0 ml (50.0 mmol) of 2.5 M $^nBuLi$ in hexanes was added in one portion to a solution of 18.0 g (50.0 mmol) of 7-(3,5-di-*tert*-butylphenyl)-5-isopropyl-2-methyl-1*H*-indene in 250 ml of ether at -50 °C. This mixture was stirred overnight at room temperature, then the resulting yellow-orange solution was cooled to -40 °C, and 200 mg of CuCN was added. The obtained mixture was stirred for 30 min at -25 °C, and 3.23 g (25.0 mmol) of dichlorodimethylsilane was added in one portion. Further on, this mixture was stirred overnight at ambient temperature, then filtered through a pad of silica gel 60 (40-63 μm) which was additionally washed by 2 x 50 ml of dichloromethane. The combined filtrate was evaporated under vacuum, and the residue was dried in vacuo at elevated temperature. This procedure gave 19.5 g (~100%) of the title product of >90% purity. On the evidence of NMR spectroscopy it was a ca. 3:2 mixture of the stereoisomers. This yellowish glassy product was used without further purification.
$^1H$ NMR ($CDCl_3$): $\delta$ 7.48-7.56 (m), 7.38 (m), 7.25-7.30 (m), 6.90 (m), 3.90 (s), 3.88 (s), 3.11 (sept, $J$ = 6.9 Hz), 2.36 (s), 1.52 (s), 1.38-1.49 (m), -0.02 (s), -0.08 (s), - 0.09 (s).

***Rac*-dimethylsilanediylbis[2-methyl-4-(3,5-di-*tert*-butylphenyl)-6-isopropyl-inden-1-yl]zirconium dichloride (I-mc3)**

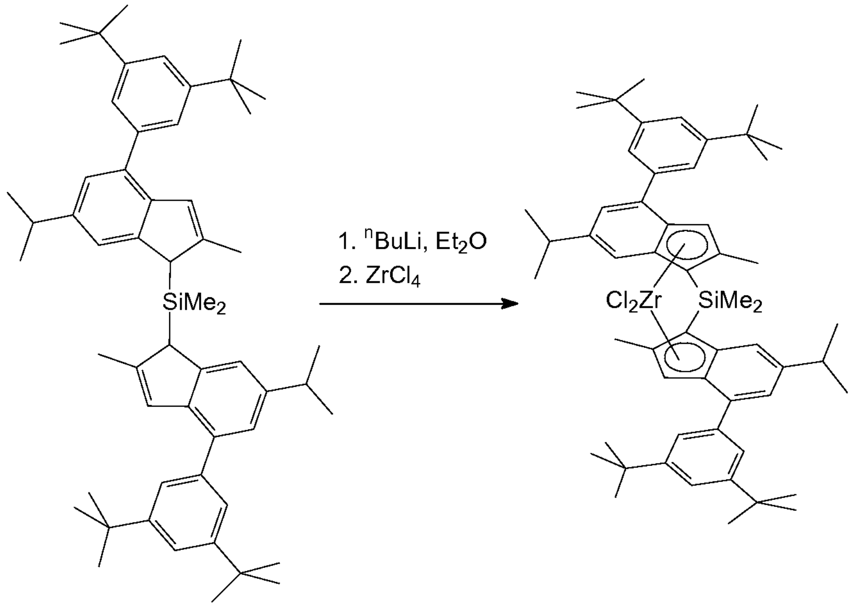


20.0 ml (50.0 mmol) of 2.5 M $^{n}$BuLi in hexanes was added in one portion to a solution of 19.5 g (25.0 mmol) of bis[2-methyl-4-(3,5-di-*tert*-butylphenyl)-6-isopropyl-1*H*-inden-1-yl](dimethyl)silane (as prepared above) in 250 ml of ether cooled to -30 °C. This mixture was stirred overnight at room temperature. The resulting light-orange solution was cooled to -50 °C and 5.83 g (25.0 mmol) of $ZrCl_4$ was added. The reaction mixture was stirred for 24 h giving orange suspension which then was evaporated to dryness. The residue was poured into 200 ml of hot toluene. This hot mixture was filtered through glass frit (G4). On the evidence of NMR spectroscopy the obtained filtrate included a ca. 1 to 1 mixture of *rac*- and *meso*-zirconocenes. This filtrate was evaporated to ca. 60 ml and then heated to dissolve the formed precipitate. Orange crystals precipitated from this solution overnight at room temperature were collected and dried in vacuum. This procedure gave 7.07 g of pure *meso*-zirconocene. The mother liquor was evaporated to ca. 40 ml. Yellow crystals precipitated from this solution for 4 h at room temperature were collected and dried in vacuum. This procedure gave 6.39 g of pure *rac*-zirconocene. The mother liquor was then evaporated to dryness, and 30 ml of n-hexane was added to the residue. Crystals precipitated from this solution overnight at room temperature were collected and dried in vacuum. This procedure gave 3.92 g of a ca. 6 to 4 mixture of *rac*- and *meso*-zirconocenes. Thus, the total yield of *rac*- and *meso*-zirconocenes isolated in this synthesis was 17.4 g (74%).

*Rac*-zirconocene (I-mc3).

Anal. calc. for $C_{56}H_{74}Cl_2SiZr$: C, 71.75; H, 7.96. Found: C, 71.88; H, 8.10.
$^{1}$H NMR ($CDCl_3$): $\delta$ 7.52 (d, *J* = 1.8 Hz, 4H), 7.45 (m, 2H), 7.39 (t, *J* = 1.8 Hz, 2H), 7.30 (m, 2H), 6.89 (m, 2H), 2.94 (sept, *J* = 7.0 Hz, 2H), 2.25 (s, 6H), 1.34 (s, 6H), 1.32 (s, 36H), 1.28 (d, *J* = 7.0 Hz, 3H), 1.26 (d, *J* = 7.0 Hz, 3H). $^{13}C\{^{1}H\}$ NMR ($CDCl_3$): $\delta$ 150.92, 146.39, 139.44, 138.77, 134.74, 131.01, 128.33, 127.30, 123.27, 122.59, 121.45, 119.63, 82.26, 35.05, 34.47, 31.54, 23.59, 23.49, 18.55, 2.87.

*Meso*-zirconocene (*meso*- I-mc3).

Anal. found: C, 72.01; H, 8.15.
$^{1}$H NMR ($CDCl_3$): $\delta$ 7.49 (m, 4H), 7.40 (m, 4H), 7.08 (m, 2H), 6.78 (m, 2H), 2.81 (sept, *J* = 6.5 Hz, 2H), 2.43 (s, 6H), 1.48 (s, 3H), 1.34 (s, 36H), 1.25 (d, *J* = 6.5 Hz, 3H), 1.22 (s, 3H), 1.14 (d, *J* = 6.3 Hz, 3H). $^{13}C\{^{1}H\}$ NMR ($CDCl_3$): $\delta$ 150.77, 145.43, 138.91, 138.63, 135.62, 133.25, 129.23, 125.28, 123.25, 122.17, 121.40, 120.63, 84.23, 35.06, 34.46, 31.57, 23.97, 23.19, 18.60, 3.13, 2.92.

**I-mc4: 4-Methylisobutyrophenone**

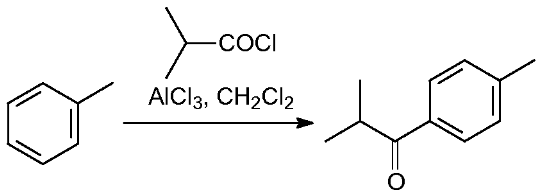


240 g (1.8 mol, 1.2 eq.) of $AlCl_3$ was added in small portions over 2 h to a stirred mixture of 500 ml of toluene and 160 g (1.5 mol) of 2-methylpropanoyl chloride at 0-5 °C. This mixture was stirred overnight at room temperature and then poured into 2000 ml of the crushed ice. The organic layer was separated; the aqueous layer was extracted with 2 x 200 ml of toluene. The combined organic extract was dried over $Na_2SO_4$, passed through a short pad of silica gel 60 (40-63 μm), and then evaporated to dryness. The crude product was distilled under vacuum to give 216 g (89%) of a colorless liquid, b.p. 125-127 °C/20 mm Hg.

Anal. calc. for $C_{11}H_{14}O$: C, 81.44; H, 8.70. Found: C, 81.38; H, 8.79.
$^1H$ NMR ($CDCl_3$): δ 7.86 (d, *J* = 7.6 Hz, 2H), 7.25 (d, *J* = 7.6 Hz, 2H), 3.53 (sep, *J* = 6.8 Hz, 1H), 2.39 (s, 3H), 1.20 (d, *J* = 6.8 Hz, 6H).

**3-Bromo-4-methylisobutyrophenone**

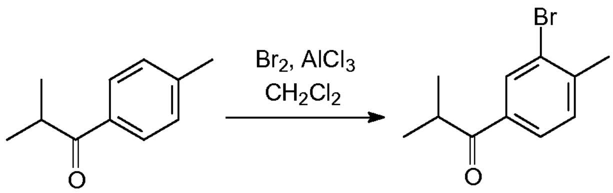


A solution of 216 g (1.33 mol) of 4-methylisobutyrophenone in 700 ml of dichloromethane was added over 30 min to a suspension of 445 g (3.34 mol) of $AlCl_3$ in 1150 ml of dicloromethane at 0 °C. The reaction mixture was stirred for 10 min at this temperature, then 71.5 ml (223 g, 1.4 mol, 5% excess) of bromine was added dropwise over 2 h. The resulting mixture was stirred overnight at room temperature and then poured onto 4000 $cm^3$ of ice. The organic layer was separated, and the aqueous layer was extracted with 3 x 300 ml of dichloromethane. The combined extract was washed with aqueous $K_2CO_3$, dried over $K_2CO_3$, passed through a short pad of silica gel, and, finally, evaporated to dryness. Crude product was distilled under vacuum to give 290 g (90%) of a colorless liquid, b.p. 124-127 °C/6 mm Hg.

Anal. calc. for $C_{11}H_{13}BrO$: C, 54.79; H, 5.43. Found: C, 54.90; H, 5.61.
$^1H$ NMR ($CDCl_3$): δ 8.11 (s, 1H, 2-H), 7.78 (d, *J* = 7.8 Hz, 1H, 6-H), 7.32 (d, *J* = 7.8 Hz, 1H, 5-H), 3.48 (sep, *J* = 6.8 Hz, 1H, C*H*$Me_2$), 2.45 (s, 3H, 4-Me), 1.20 (d, *J* = 6.8 Hz, 6H, CH*$Me_2$*). $^{13}C\{^1H\}$ NMR ($CDCl_3$): δ 202.8, 143.1, 135.5, 132.3, 130.9, 127.1, 125.3, 35.3, 23.1, 19.0.

**3-Bromo-4-methylpivalophenone**

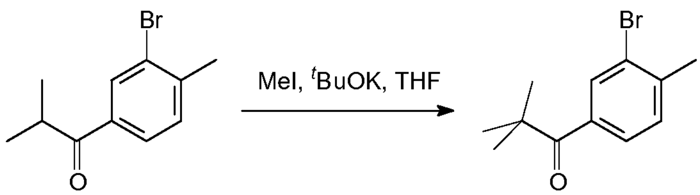


To the mixture of 241 g (1.0 mol) of 3-bromo-4-methylisobutyrophenone and 170 g (1.2 mol) of iodomethane in 200 ml of THF a solution of 157 g (1.4 mol) of potassium *tert*-butoxide in 1000 ml of THF was added dropwise by vigorous stirring for 5 h at 0-5°C. This mixture was stirred overnight at room temperature. Further on, 1000 ml of water and 400 ml of n-hexane were added to the reaction mixture. The organic layer was separated, and the aqueous layer

was extracted with 500 ml of dichloromethane. The combined organic extract was dried over $Na_2SO_4$ and then evaporated to dryness. Crude product was distilled under vacuum to give 248 g (97%) of a colorless liquid, b.p. 159-163 °C/20-21 mm Hg.

Anal. calc. for $C_{12}H_{15}BrO$: C, 56.49; H, 5.93. Found: C, 56.58; H, 6.10.
$^1H$ NMR ($CDCl_3$): $\delta$ 7.91 (d, *J* = 1.5 Hz, 1H, 2-H), 7.59 (dd, *J* = 8.1 Hz, *J* = 1.8 Hz, 1H, 6-H), 7.27 (d, *J* = 8.1 Hz, 1H, 5-H), 2.44 (s, 3H, 4-Me), 1.36 (s, 9H, $^tBu$). $^{13}C\{^1H\}$ NMR ($CDCl_3$): $\delta$ 206.9, 141.1, 137.4, 132.1, 130.3, 126.9, 124.7, 44.2, 28.0, 22.9.

**2-Bromo-4-pivaloylbenzyl bromide**

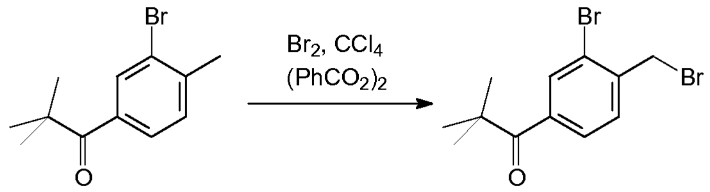


154 g (0.961 mol) of bromine and 1 g of benzoyl peroxide were added to a solution of 245 g (0.961 mol) of 3-bromo-4-methylpivalophenone in 1300 ml of tetrachloromethane. This mixture was refluxed for 2 h (until the disappearance of the red color). The resulting mixture was cooled to room temperature, filtered through glass frit (G3), and the filtrate was evaporated to dryness. Crude product was distilled under vacuum to give 263 g (82%) of a colorless oil, b.p. 135-155 °C/7 mm Hg.

Anal. calc. for $C_{12}H_{14}Br_2O$: C, 43.15; H, 4.22. Found: C, 43.39; H, 4.42.
$^1H$ NMR ($CDCl_3$): $\delta$ 7.87 (d, *J* = 1.8 Hz, 1H, 3-H), 7.61 (dd, *J* = 8.1 Hz, *J* = 1.8 Hz, 1H, 5-H), 7.49 (d, *J* = 8.1 Hz, 1H, 6-H), 4.59 (s, 2H, $CH_2$Br), 1.34 (s, 9H, *$^tBu$*). $^{13}C\{^1H\}$ NMR ($CDCl_3$): $\delta$ 206.9, 140.0, 139.5, 132.7, 130.8, 127.2, 124.2, 44.3, 32.3, 27.8.

**Diethyl (4-pivaloyl-2-bromobenzyl)(methyl)malonate**

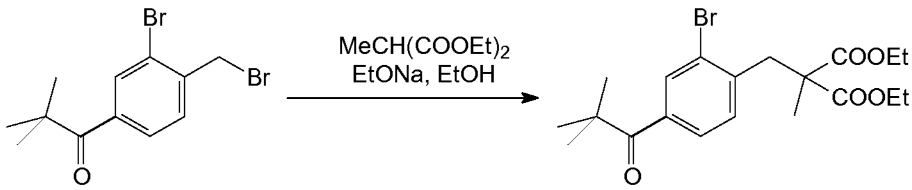


137 g (0.787 mol) of diethyl methylmalonate was added to a solution of sodium ethoxide prepared from 18.1 g (0.787 mol) of sodium metal and 600 ml of anhydrous ethanol. This mixture was stirred for 15 min, then 263 g (0.787 mol) of 2-bromo-4-pivaloylbenzyl bromide was added at such a rate as to maintain a gentle reflux. Additionally, this mixture was refluxed for 3 h, then ethanol was distilled off at atmospheric pressure. The obtained mixture was cooled to room temperature, 1000 ml of water and 500 ml of dichloromethane were added. The organic layer was separated, and the aqueous layer was extracted with 2 x 300 ml of dichloromethane. The combined organic extract was dried over $Na_2SO_4$ and evaporated to dryness. Crude product was distilled under vacuum to give 274 g (81%) of a colorless oil, b.p. 170-176 °C/7 mm Hg.

Anal. calc. for $C_{20}H_{27}BrO_5$: C, 56.21; H, 6.37. Found: C, 56.39; H, 6.41.
$^1H$ NMR ($CDCl_3$): $\delta$ 7.89 (d, *J* = 1.8 Hz, 1H, 3-H), 7.56 (dd, *J* = 8.1 Hz, *J* = 1.8 Hz, 1H, 5-H), 7.24 (d, *J* = 8.1 Hz, 1H, 6-H), 4.17-4.27 (m, 4H, $CH_2$Me), 3.53 (s, 2H, Ar$CH_2$), 1.40 (s, 3H, *Me*C($COOEt)_2$), 1.33 (s, 9H, $^tBu$), 1.26 (t, *J* = 7.1 Hz, 6H, $CH_2$*Me*). $^{13}C\{^1H\}$ NMR ($CDCl_3$): $\delta$ 206.8, 171.5, 139.5, 138.2, 132.6, 130.8, 126.6, 126.1, 61.5, 54.8, 44.2, 39.2, 27.9, 19.4, 13.9.

**3-(2-Bromo-4-pivaloylphenyl)-2-methylpropanoic acid**

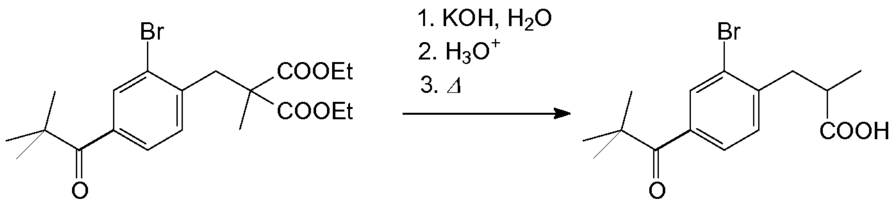


A solution of 160 g of KOH in 420 ml of water was added to a solution of 274 g (0.640 mol) of (4-pivaloyl-2-bromobenzyl)(methyl)malonate in 300 ml of methanol. This mixture was refluxed for 4 h. Methanol and water were distilled off until vapor temperature reached 95 °C, and 3000 ml of water and then 12 M HCl (to pH 1) were added to the residue. The precipitated substituted methylmalonic acid was filtered off, washed with water, and partially dried on the filter. Crude 3-(2-bromo-4-pivaloylphenyl)-2-methylpropanoic acid was obtained as reddish oil after decarboxylation of this substituted methylmalonic acid at 180 °C. Yield 189 g (90%).

Anal. calc. for $C_{15}H_{19}BrO_3$: C, 55.06; H, 5.85. Found: C, 54.83; H, 5.60.
$^1$H NMR ($CDCl_3$): $\delta$ 11.30 (br. s, 1H, COOH), 7.89 (d, *J* = 1.5 Hz, 1H, 3-H), 7.59 (dd, *J* = 7.8 Hz, *J* = 1.8 Hz, 1H, 5-H), 7.27 (d, *J* = 7.8 Hz, 1H, 6-H), 3.21 (dd, *J* = 13.4 Hz, *J* = 7.1 Hz, 1H, ArC*H*H'), 2.88-3.00 (m, 1H, C*H*COOH), 2.84 (dd, *J* = 13.4 Hz, *J* = 7.3 Hz, 1H, ArCH*H'*), 1.34 (s, 9H, $^t$Bu), 1.24 (d, *J* = 7.1 Hz, 3H, Me). $^{13}C\{^1H\}$ NMR ($CDCl_3$): $\delta$ 207.1, 181.4, 141.5, 138.3, 132.6, 130.8, 126.9, 124.6, 44.3, 39.1, 39.1, 27.9, 16.9.

**3-(2-Bromo-4-neopentylphenyl)-2-methylpropanoic acid**

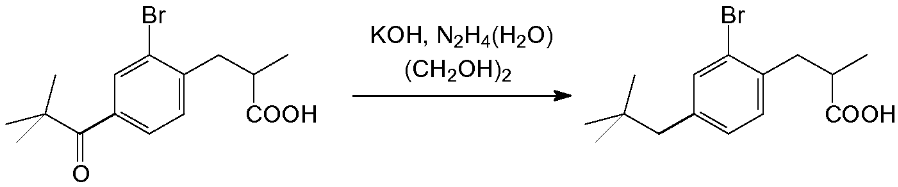


1000 ml of ethylene glycol, and 125 g of KOH were added in one portion to a mixture of 189 g (0.577 mol) of 3-(2-bromo-4-pivaloylphenyl)-2-methylpropanoic acid 100 ml of hydrazine hydrate. The resulting yellowish solution was refluxed for 5 h. Then, a reflux condenser was replaced by a Claisen distillation head with Liebig condenser. A mixture of hydrazine, water and ethylene glycol was slowly distilled off until the distillation temperature reached 198 °C (eventually this reaction mixture was evaporated to about half its initial volume over 5 h). The residue cooled to room temperature was poured into 4 L of water, acidified by 12 N HCl to pH~1, and 700 ml of dichloromethane was added. The organic layer was separated and the aqueous layer was extracted with 2 x 250 ml of dichloromethane. The combined organic extract was washed with 1 L of water and then evaporated to dryness to give 178 g (99%) of a red viscous oil of the title compound which was further used without an additional purification.
$^1$H NMR ($CDCl_3$): $\delta$ 11.6 (br. s, 1H, COOH), 7.31 (d, *J* = 1.5 Hz, 1H, 3-H), 7.11 (d, *J* = 7.6 Hz, 1H, 6-H), 6.98 (dd, *J* = 7.6 Hz, *J* = 1.5 Hz, 1H, 5-H), 3.16 (dd, *J* = 13.5 Hz, *J* = 6.8 Hz, 1H, ArC*H*H'), 2.84-2.98 (m, 1H, C*H*COOH), 2.77 (dd, *J* = 13.5 Hz, *J* = 7.8 Hz, 1H, ArCH*H'*), 2.42 (s, 2H, $CH_2{}^t$Bu), 1.21 (d, *J* = 6.8 Hz, 3H, Me), 0.89 (s, 9H, $^t$Bu). $^{13}C\{^1H\}$ NMR ($CDCl_3$): $\delta$ 182.6, 140.2, 135.6, 134.5, 130.4, 129.5, 124.0, 49.3, 39.5, 38.9, 31.7, 29.3, 16.7.

**3-[2-Bromo-4-neopentyl-phenyl]-2-methylpropanoyl chloride**

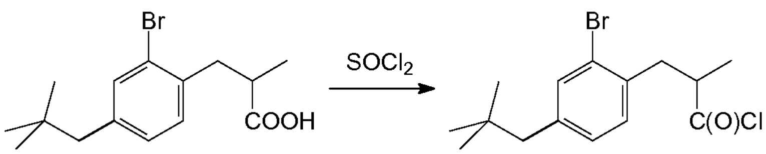


A mixture of 178 g (0.569 mol) of 3-(2-bromo-4-neopentylphenyl)-2-methylpropanoic acid and 145 ml (1.99 mol, 3.5 eq.) ofthionyl chloride was stirred for 24 h at room temperature. Excess thionyl chloride was distilled off. The

residue was distilled under vacuum to give 166 g (88%) of the title product, b.p. 153-157°C/ 7 mm Hg.

Anal. calc. for $C_{15}H_{20}BrClO$: C, 54.32; H, 6.08. Found: C, 54.12; H, 6.35.

$^1$H NMR ($CDCl_3$): $\delta$ 7.33 (d, *J* = 1.5 Hz, 1H, 3-H), 7.11 (d, *J* = 7.8 Hz, 1H, 6-H), 7.00 (dd, *J* = 7.8 Hz, *J* = 1.5 Hz, 1H, 5-H), 3.19-3.37 (m, 2H, ArC*H*H'and C*H*COCl), 2.82 (dd, *J* = 12.9 Hz, *J* = 6.8 Hz, 1H, ArCH*H*'), 2.43 (s, 2H, C$H_2$$^t$Bu), 1.31 (d, *J* = 6.8 Hz, 3H, Me), 0.90 (s, 9H, $^t$Bu). $^{13}$C{$^1$H} NMR ($CDCl_3$): $\delta$ 177.1, 140.8, 134.7, 134.2, 130.6, 129.7, 123.8, 51.4, 49.3, 38.9, 31.8, 29.3, 16.8.

**4-Bromo-6-neopentyl-2-methylindan-1-one**

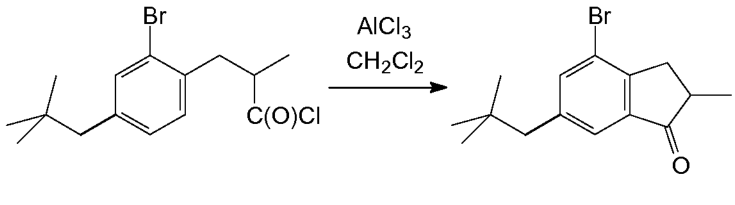


166 g (0.499 mol) of 3-[2-bromo-4-neopentyl-phenyl]-2-methylpropanoyl chloride dissolved in 100 ml of dichloromethane was added dropwise with vigorous stirring to a suspension of 80.0 g (0.600 mol, 1.2 eq.) of $AlCl_3$ in 500 ml of dichloromethane at +5°C. This mixture was stirred overnight at room temperature and then poured on 500 g of crushed ice. The organic layer was separated, and the aqueous layer was extracted with 3 x 100 ml of dichloromethane. The combined organic extract was washed by aqueous $K_2CO_3$, dried over $K_2CO_3$, passed through a short pad of silica gel 60 (40-63 $\mu$m), and then evaporated to dryness. The crude product was distilled under vacuum to give 137 g (93%) of a yellowish oil, b.p. 155-162 °C/7 mm Hg.

Anal. calc. for $C_{15}H_{19}BrO$: C, 61.03; H, 6.49. Found: C, 61.19; H, 6.44.

$^1$H NMR ($CDCl_3$): $\delta$ 7.54 (d, *J* = 1.3 Hz, 1H, 7-H), 7.45 (d, *J* = 1.3 Hz, 1H, 5-H), 3.31 (dd, *J* = 17.4 Hz, *J* = 7.8 Hz, 1H, 3-C*H*H'), 2.67-2.81 (m, 1H, 2-H), 2.62 (dd, *J* = 17.4 Hz, *J* = 4.0 Hz, 1H, 3-CH*H*'), 2.53 (s, 2H, C$H_2$$^t$Bu), 1.33 (d, *J* = 7.6 Hz, 3H, 2-Me), 0.91 (s, 9H, $^t$Bu). $^{13}$C{$^1$H} NMR ($CDCl_3$): $\delta$ 208.6, 150.6, 141.5, 139.3, 137.8, 124.2, 121.0, 49.3, 42.2, 35.5, 31.7, 29.1, 16.1.

**1-methoxy-2-methyl-4-Bromo-6-neopentyl-indane**

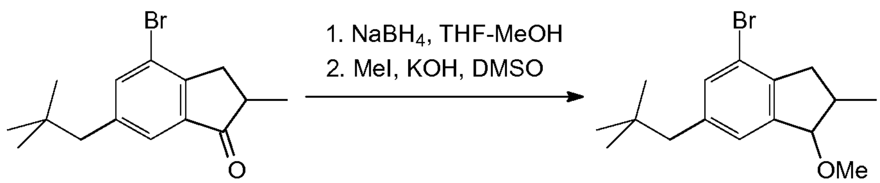


235 ml of methanol was added dropwise with vigorous stirring to a mixture of 137 g (0.464 mol) of 4-bromo-6-neopentyl-2-methylindan-1-one and 26.3 g (0.695 mmol) of $NaBH_4$ in 470 ml of THF for 5 h at 0-5 °C. This mixture was stirred overnight at room temperature and then evaporated to dryness under vacuum. The residue was acidified by 2 M HCl to pH~5, and the crude product was extracted with 3 x 250 ml of dichloromethane. The combined organic extract was dried over $Na_2SO_4$ and evaporated to dryness. The resulting yellowish oil was immediately dissolved in 450 ml of DMSO, then 104 g (1.86 mol, 4 eq.) of KOH and 132 g (0.930 mol, 2 eq.) of iodomethane were added. The obtained mixture was stirred for 5 h at ambient temperature, and then 2 L of water was added. The organic layer was separated, and the aqueous layer was extracted with 3 x 100 ml of dichloromethane. The combined organic extract was additionally washed with 5 x 1000 ml of water, dried over $Na_2SO_4$, and then evaporated to dryness. Fractional distillation of the residue gave 131 g (91%) of 1-methoxy-2-methyl-4-bromo-6-neopentyl-indane as a mixture of two stereoisomers, b.p. 137-139°C/ 5-6 mm Hg.

Anal. calc. for $C_{16}H_{23}BrO$: C, 61.74; H, 7.45. Found: C, 61.96; H, 7.61.

$^1$H NMR ($CDCl_3$): $\delta$ 7.19 (s, 1H, 5-H of *anti*-isomer), 7.18 (s, 1H, 5-H of *syn*-isomer), 7.05 (s, 1H, 7-H of *anti*-isomer), 7.05 (s, 1H, 7-H of *syn*-isomer), 4.53 (d, *J* = 5.6 Hz, 1H, 1-H of *syn*-isomer), 4.43 (d, *J* = 4.3 Hz, 1H, 1-H of *anti*-isomer), 3.42 (s, 3H, OMe of *anti*-isomer), 3.38 (s, 3H, OMe of *syn*-isomer), 3.19 (dd, *J* = 16.2 Hz, *J* = 7.6 Hz, 1H, ArC*H*H' of *anti*-isomer), 2.91 (dd, *J* = 15.7 Hz, *J* = 6.8 Hz, 1H, ArC*H*H' of *syn*-isomer), 2.34-2.71 (m, 8H, ArCH*H*', 2-H and C$H_2$$^t$Bu

of both isomers), 1.17 (d, *J* = 7.1 Hz, 3H, 2-Me of *anti*-isomer), 1.10 (d, *J* = 6.8 Hz, 3H, 2-Me of *syn*-isomer), 0.90 (s, 18H, $^{t}$Bu of both isomers). $^{13}C\{^{1}H\}$ NMR ($CDCl_3$): $\delta$ 143.8, 143.3, 141.2, 140.7, 140.3, 139.9, 133.2, 133.0, 126.3, 126.2, 119.5, 119.5, 91.9, 86.7, 56.7, 56.2, 49.6 (two resonances), 39.4, 39.16, 39.15, 38.2, 31.8, 31.8, 29.3 (two resonances), 19.3, 13.5.

**2-methylMethyl-7-(3,5-Di-*tert*-butylphenyl)-5-neopentyl-1*H*-indene**

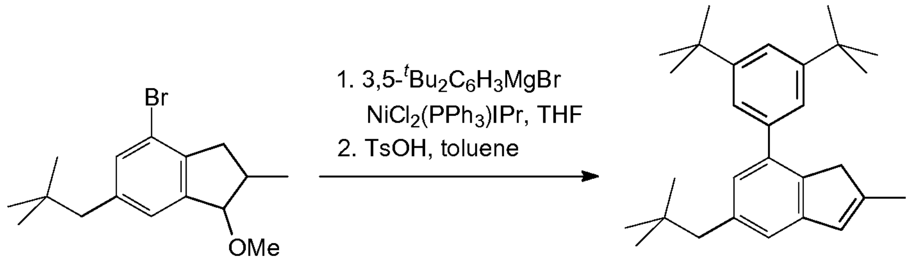


220 ml (110 mmol, 1.25 eq.) of 0.5 M 3,5-di-*tert*-butylphenylmagnesium bromide in THF was added to a mixture of 1.38 g (1.77 mmol, 2 mol. %) of $NiCl_2(PPh_3)$IPr and 27.5 g (88.2 mmol) of 1-methoxy-2-methyl-4-bromo-6-neopentylindane, at such a rate as to maintain a gentle reflux. The resulting solution was refluxed for 0.5 h, then cooled to room temperature, and 50 ml of water was added. The major part of THF was distilled off in a rotary evaporator, and then 500 ml of dichloromethane and 500 ml of 1 M HCl were added to the residue. The organic layer was separated, and

the aqueous layer was extracted with 150 ml of dichloromethane. The combined organic extract was evaporated to dryness to give a greenish-yellow oil. A mixture of this product, 1.5 g of TsOH, and 600 ml of toluene was refluxed using Dean-Stark head for 15 min. The obtained mixture was washed by 200 ml of 10% aqueous $NaHCO_3$. The organic layer was separated, and the aqueous layer was extracted with 150 ml of dichloromethane. The combined organic extract was evaporated to dryness, and the product was isolated by flash-chromatography on silica gel 60 (40-63 μm; eluent: hexanes-dichloromethane = 10:1, vol.). This procedure gave 31.7 g (93%) of a colorless oil which solidified on standing at room temperature.

Anal. calc. for $C_{29}H_{40}$: C, 89.63; H, 10.37. Found: C, 89.86; H, 10.59.
$^{1}H$ NMR ($CDCl_3$): $\delta$ 7.41 (t, *J* = 1.8 Hz, 1H, 4-H in 3,5-$^{t}Bu_2C_6H_3$), 7.37 (d, *J* = 1.8 Hz, 2H, 2,6-H in 3,5-$^{t}Bu_2C_6H_3$), 7.02 (s, 1H, 6-H in indenyl), 6.92 (s, 1H, 4-H in indenyl), 6.51 (q, *J* = 1.3 Hz, 1H, 3-H in indenyl), 3.35 (s, 2H, $CH_2$ in indenyl), 2.57 (s, 2H, $CH_2{}^{t}Bu$), 2.13 (s, 3H, 2-Me in indenyl), 1.38 (s, 18H, 3,5-$^{t}Bu_2C_6H_3$), 0.95 (s, 9H, $CH_2{}^{t}Bu$). $^{13}C\{^{1}H\}$ NMR ($CDCl_3$): $\delta$ 150.5, 146.2, 146.0, 140.7, 138.5, 138.3, 137.6, 127.2, 126.8, 122.9, 121.0, 120.8, 50.3, 42.6, 34.9, 31.8, 31.6, 29.5, 16.8.

**Bis[2-methyl-4-(3,5-di-*tert*-butylphenyl)-6-neopentyl-1*H*-inden-1-yl] (dimethyl)silane**

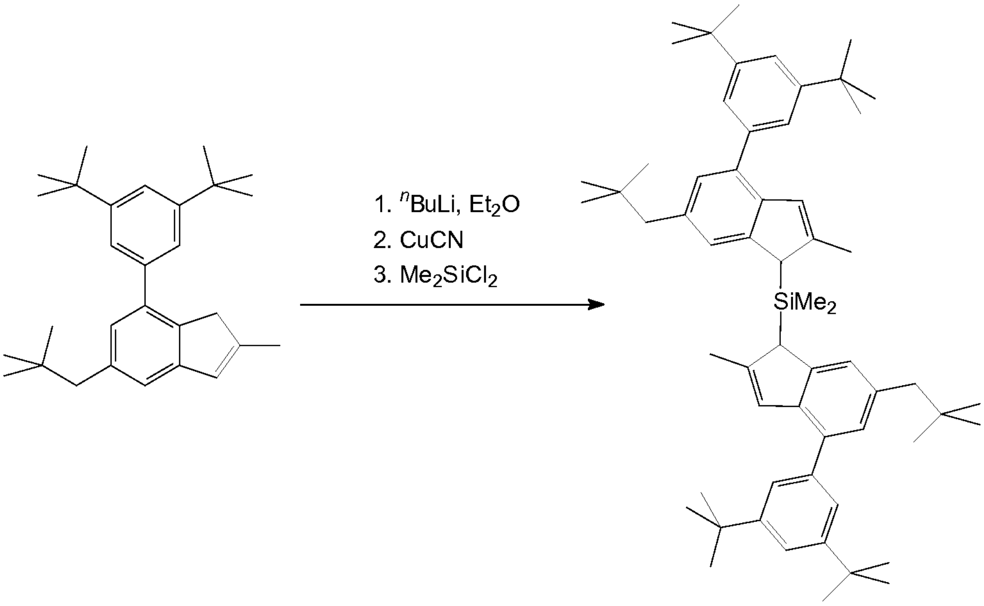


16.0 ml (40.0 mmol) of 2.5 M $^nBuLi$ in hexanes was added in one portion to a solution of 15.8 g (40.6 mmol) of 2-methyl-7-(3,5-di-*tert*-butylphenyl)-5-neopentyl-1*H*-indene in 250 ml of ether at -50 °C. This mixture was stirred overnight at room temperature, then the resulting mixture was cooled to -40 °C, and 200 mg of CuCN was added. The obtained mixture was stirred for 30 min at -25 °C, then 2.58 g (20.0 mmol) of dichlorodimethylsilane was added in one portion. Then, this mixture was stirred overnight at ambient temperature, filtered through a pad of silica gel 60 (40-63 μm) which was additionally washed by 2x50 ml of dichloromethane. The combined filtrate was evaporated under vacuum, and the title product was isolated by flash-chromatography on silica gel 60 (40-63 μm; eluent: hexanes-dichloromethane = 10:1, vol.). This procedure gave 13.9 g (82%) of a yellowish glassy solid which on the evidence of NMR spectroscopy has >95% purity in a ca. 3 to 2 mixture of the stereoisomers.
$^1H$ NMR ($CDCl_3$): $\delta$7.45-7.56 (m), 7.26 (m), 7.20 (m), 7.18 (m), 6.91 (m), 4.02 (s), 3.95 (s), 2.56-2.79 (m), 2.37 (s), 2.28 (s), 1.50 (s), 1.05 (s), 1.04 (s), -0.12 (s),-0.13 (s), -0.19 (s).

**R*ac*-dimethylsilanediylbis[$\eta^5$-4-(3,5-di-*tert*-butylphenyl)-6-neopentyl-2-methyl-1*H*-inden-1-yl]zirconium dichloride (I-mc4)**

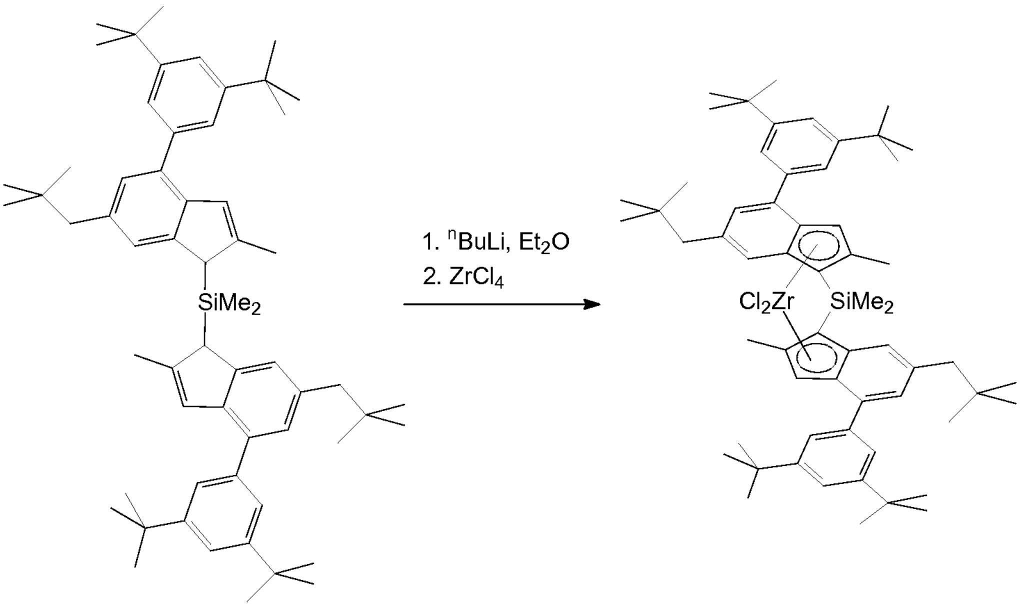

13.4 ml (33.5 mmol) of 2.5 M $^{n}$BuLi in hexanes was added in one portion to a solution of 13.9 g (16.7 mmol) of bis[2-methyl-4-(3,5-di-*tert*-butylphenyl)-6-neopentyl-1*H*-inden-1-yl](dimethyl)silane (prepared above) in 200 ml of ether cooled to -30 °C. This mixture was stirred overnight at room temperature, then cooled to -50 °C, and 3.90 g (16.7 mmol) of $ZrCl_4$ was added. The reaction mixture was stirred for 24 h, and then the formed orange suspension was evaporated to dryness. The residue was poured into 200 ml of hot toluene. The obtained hot mixture was filtered through glass frit (G4). On the evidence of NMR spectroscopy the obtained filtrate included a ca. 1 to 1 mixture of *rac*- and *meso*-zirconocenes. This filtrate was evaporated to ca. 60 ml and then heated to dissolve the precipitate. Crystals precipitated from this solution for 1.5 h at room temperature were collected and dried in vacuum. This procedure gave 4.94 g of pure *meso*-zirconocene. The mother liquor was evaporated to ca. 40 ml. Crystals precipitated from this solution for 1.5 h at room temperature were collected and dried in vacuum. This procedure gave 1.53 g of pure *rac*-zirconocene. The obtained mother liquor was evaporated to dryness, and 35 ml of n-hexane was added. Crystals precipitated from this solution for 1.5 h at room temperature were collected and dried in vacuum. This procedure gave 3.31 g of pure *rac*-zirconocene. Again, the obtained mother liquor was evaporated to dryness, and 25 ml of n-hexane was added. Crystals precipitated from this solution overnight at room temperature were collected and dried in vacuum. This procedure gave 0.64 g of a ca. 6 to 1 mixture of *meso*- and *rac*-zirconocenes. Thus, the overall yield of *rac*- and *meso*-zirconocenes isolated in this synthesis was 10.4 g (63%).

*Rac*-zirconocene (I-mc4).

Anal. calc. for $C_{60}H_{82}Cl_2SiZr$: C, 72.54; H, 8.32. Found: C, 72.56; H, 8.44.
$^{1}$H NMR ($CDCl_3$): $\delta$ 7.52 (m, 4H), 7.40 (m, 2H), 7.38 (m, 2H), 7.23 (m, 2H), 6.90 (m, 2H), 2.53 (d, *J* = 18.2 Hz, 2H), 2.50 (d, *J* = 18.2 Hz, 2H), 2.25 (s, 6H), 1.32 (2s, 42H), 1.00 (s, 18H). $^{13}C\{^{1}H\}$ NMR ($CDCl_3$): $\delta$ 150.87, 138.72, 138.44, 137.90, 134.85, 131.07, 130.37, 128.45, 124.31, 123.31, 122.42, 121.41, 82.48, 50.93, 35.02, 32.43, 31.52, 29.71, 18.86, 2.69.

*Meso*-zirconocene *(meso* I-mc4).

Anal. found: C, 72.67; H, 8.49.
$^{1}$H NMR ($CDCl_3$): $\delta$ 7.46 (m, 4H), 7.38 (m, 2H), 7.32 (m, 2H), 6.96 (m, 2H), 6.76 (m, 2H), 2.44 (s, 6H), 2.42 (d, *J* = 13.2 Hz, 2H), 2.32 (d, *J* = 13.2 Hz, 2H), 1.44 (s, 3H), 1.33 (s, 36H), 1.22 (s, 3H), 0.96 (s, 18H). $^{13}C\{^{1}H\}$ NMR ($CDCl_3$): $\delta$ 150.60, 138.77, 137.39, 136.46, 135.93, 133.10, 129.61, 129.04, 125.02, 123.29, 121.22, 121.16, 83.88, 50.84, 34.97, 32.08, 31.49, 29.60, 18.77, 3.03, 2.50.

Table 1 summarises the 6-position substituents in the examples:

| **Metallocene** | **$R^6$** |
|---|---|
| C-mc1 | H |
| C-mc2 | H * |
| I-mc1 | methyl |
| I-mc2 | ethyl |
| I-mc3 | *iso*-propyl |
| I-mc4 | *neo*-pentyl |
| * with 7-methoxy, | |

***Comparative catalysts***

**CE1 - *Catalyst synthesis with C-mc1***

Inside the glovebox, 80 µL of dry and degassed surfactant solution were mixed with 2 mL of MAO in a septum bottle and left to react overnight. The following day, 64.9 mg of the metallocene C-mc1 was dissolved with 4 mL of the MAO solution in another septum bottle and left to stir inside the glovebox.

After 60 minutes, 1 mL of the surfactant solution and the 4 mL of the MAO-metallocene solution were successively added into a 50mL emulsification glass reactor containing 40mL of PFC at -10 °C and equipped with an overhead stirrer (stirring speed = 600 rpm). Total amount of MAO is 5 mL (300 equivalents). A red-orange emulsion formed immediately and stirred during 15 minutes at 0 °C / 600rpm. Then the emulsion was transferred via a 2/4 teflon tube to 100mL of hot PFC at 90 °C, and stirred at 600rpm until the transfer is completed, then the speed was reduced to 300 rpm. After 15 minutes stirring, the oil bath was removed and the stirrer turned off. The catalyst was left to settle up on top of the PFC and after 45 minutes the solvent was siphoned off. The remaining red catalyst was dried during 2 hours at 50 °C over an argon flow. 0.45 g of a red free flowing powder was obtained.

**CE2 - *Catalyst synthesis with C-mc2***

Inside the glovebox, 80 μL of dry and degassed surfactant solution were mixed with 2 mL of MAO in a septum bottle and left to react overnight. The following day, 69.4 mg of the metallocene *C-mc2* was dissolved with 4 mL of the MAO solution in another septum bottle and left to stir inside the glovebox.

After 60 minutes, 1 mL of the surfactant solution and the 4 mL of the MAO-metallocene solution were successively added into a 50mL emulsification glass reactor containing 40mL of PFC at -10 °C and equipped with an overhead stirrer (stirring speed = 600 rpm). Total amount of MAO is 5 mL (300 equivalents). A red-orange emulsion formed immediately and stirred during 15 minutes at 0 °C / 600rpm. Then the emulsion was transferred via a 2/4 teflon tube to 100mL of hot PFC at 90 °C, and stirred at 600rpm until the transfer is completed, then the speed was reduced to 300 rpm. After 15 minutes stirring, the oil bath was removed and the stirrer turned off. The catalyst was left to settle up on top of the PFC and after 45 minutes the solvent was siphoned off. The remaining red catalyst was dried during 2 hours at 50 °C over an argon flow. 0.74 g of a red free flowing powder was obtained.

**IE1 - *Catalyst synthesis with I-mc1***

Inside the glovebox, 80 μL of dry and degassed surfactant solution were mixed with 2 mL of MAO in a septum bottle and left to react overnight. The following day, 67.0 mg of the metallocene *I-mc1* was dissolved with 4 mL of the MAO solution in another septum bottle and left to stir inside the glovebox.

After 60 minutes, 1 mL of the surfactant solution and the 4 mL of the MAO-metallocene solution were successively added into a 50mL emulsification glass reactor containing 40mL of PFC at -10 °C and equipped with an overhead stirrer (stirring speed = 600 rpm). Total amount of MAO is 5 mL (300 equivalents). A red emulsion formed immediately and stirred during 15 minutes at 0 °C / 600rpm. Then the emulsion was transferred via a 2/4 teflon tube to 100mL of hot PFC at 90 °C, and stirred at 600rpm until the transfer is completed, then the speed was reduced to 300 rpm. After 15 minutes stirring, the oil bath was removed and the stirrer turned off. The catalyst was left to settle up on top of the PFC and after 35 minutes the solvent was siphoned off. The remaining red catalyst was dried during 2 hours at 50 °C over an argon flow. 0.50 g of a red free flowing powder was obtained.

**IE2 - *Catalyst synthesis with I-mc2***

Inside the glovebox, 80 μL of dry and degassed surfactant solution were mixed with 2 mL of MAO in a septum bottle and left to react overnight. The following day, 69.1 mg of the metallocene *I-mc2* was dissolved with 4 mL of the MAO solution in another septum bottle and left to stir inside the glovebox.

After 60 minutes, 1 mL of the surfactant solution and the 4 mL of the MAO-metallocene solution were successively added into a 50mL emulsification glass reactor containing 40mL of PFC at -10 °C and equipped with an overhead stirrer (stirring speed = 600 rpm). Total amount of MAO is 5 mL (300 equivalents). A red-orange emulsion formed immediately and stirred during 15 minutes at 0 °C / 600rpm. Then the emulsion was transferred via a 2/4 teflon tube to 100mL of hot PFC at 90 °C, and stirred at 600 rpm until the transfer is completed, then the speed was reduced to 300 rpm. After 15 minutes stirring, the oil bath was removed and the stirrer turned off. The catalyst was left to settle up on top of the PFC and after 35 minutes the solvent was siphoned off. The remaining red catalyst was dried during 2 hours at 50 °C over an argon flow. 0.51 g of a red free flowing powder was obtained.

**IE3 - *Catalyst synthesis with I-mc3***

Inside the glovebox, 80 μL of dry and degassed surfactant solution were mixed with 2 mL of MAO in a septum bottle and left to react overnight. The following day, 71.2 mg of the metallocene I-mc3 was dissolved with 4 mL of the MAO solution in another septum bottle and left to stir inside the glovebox.

After 60 minutes, 1 mL of the surfactant solution and the 4 mL of the MAO-metallocene solution were successively added into a 50mL emulsification glass reactor containing 40mL of PFC at -10 °C and equipped with an overhead stirrer (stirring speed = 600 rpm). Total amount of MAO is 5 mL (300 equivalents). A red-orange emulsion formed immediately and stirred during 15 minutes at 0 °C / 600rpm. Then the emulsion was transferred via a 2/4 teflon tube to 100mL of hot PFC at 90 °C, and stirred at 600rpm until the transfer is completed, then the speed was reduced to 300 rpm. After 15 minutes stirring, the oil bath was removed and the stirrer turned off. The catalyst was left to settle up on top of the PFC and after 35 minutes the solvent was siphoned off. The remaining red catalyst was dried during 2 hours at 50 °C over an argon flow. 0.60 g of a red free flowing powder was obtained.

**IE4 - *Catalyst synthesis with I-mc4***

Inside the glovebox, 80 μL of dry and degassed surfactant solution were mixed with 2 mL of MAO in a septum bottle and left to react overnight. The following day, 76.8 mg of the metallocene I-mc4 was dissolved with 4 mL of the MAO solution in another septum bottle and left to stir inside the glovebox.

After 60 minutes, 1 mL of the surfactant solution and the 4 mL of the MAO-metallocene solution were successively added into a 50mL emulsification glass reactor containing 40mL of PFC at -10 °C and equipped with an overhead stirrer (stirring speed = 600 rpm). Total amount of MAO is 5 mL (300 equivalents). A red-orange emulsion formed immediately and stirred during 15 minutes at 0 °C / 600rpm. Then the emulsion was transferred via a 2/4 teflon tube to 100mL of hot PFC at 90 °C, and stirred at 600rpm until the transfer is completed, then the speed was reduced to 300 rpm. After 15 minutes stirring, the oil bath was removed and the stirrer turned off. The catalyst was left to settle up on top of the PFC and after 35 minutes the solvent was siphoned off. The remaining red catalyst was dried during 2 hours at 50 °C over an argon flow. 0.59 g of a red free flowing powder was obtained.

**Table 2:** Catalyst composition (ICP)

| Catalyst | Al/Zr (molar) | Zr content (wt%) | MC content (wt%) |
|---|---|---|---|
| CE1 | 280 | 0.36 | 2.71 |
| CE2 | 276 | 0.37 | 2.90 |
| IE1 | 316 | 0.38 | 3.67 |
| IE2 | 280 | 0.43 | 4.29 |
| IE3 | 323 | 0.33 | 3.39 |
| IE4 | 279 | 0.38 | 4.14 |

Polymerisations

The polymerisations were performed in a 5 L reactor. 200 μl of triethylaluminum was fed as a scavenger in 5 mL of dry and degassed pentane. The desired amount of hydrogen was then loaded (measured in mmol) and 1100 g of liquid propylene was fed into the reactor.

Procedure A: The temperature was set to 30 °C. The desired amount of catalyst (3 to 30 mg) in 5mL of PFC is flushed into the reactor with a nitrogen overpressure. The temperature is then raised to 70 °C over a period of 15 minutes. The polymerisation is stopped after 30 minutes by venting the reactor and flushing with nitrogen before the polymer is collected. (Polymerisations CP1-CP3)

Procedure B: The temperature was set to 20 °C. The desired amount of catalyst (3 to 30 mg) in 5mL of PFC is flushed into the reactor with a nitrogen overpressure. After 5 minutes of the temperature is raised to 70 °C over a period of 15 minutes. The polymerisation is stopped after 60 minutes by venting the reactor and flushing with nitrogen before the polymer is collected. (Polymerisations CP4-CP6, IP1-IP12)

The catalyst activity was calculated on the basis of the 30 (or 60) minutes period according to the following formula:

$$\textbf{Act Cat:}\quad \text{Catalyst Activity (kg/(g(cat)} * \text{h))} = \frac{\text{amount of polymer produced (kg)}}{\text{catalyst loading (g)} \times \text{polymerisation time (h)}}$$

**Act**

**Met:** $$\text{Catalyst Metal Activity } (\text{kg}/(\text{g}(\text{cat}) * \text{h})) = \frac{\text{amount of polymer produced (kg)}}{\text{catalyst metal loading (g)} \times \text{polymerisation time (h)}}$$

**Table 3:** Polymerization results with CE1, CE2, IE1-IE4

| Catalyst | Ex | Cat. (mg) | $H_2$ mmol | Pol. Yield, g | Act cat kg/gcat/h | Act Metal kg/gmetal/h | $MFR_2$ g/10min | $M_w$ kg/mol | $M_w/M_n$ | $T_m$ (°C) | $T_c$ (°C) |
|---|---|---|---|---|---|---|---|---|---|---|---|
| CE2 | CP1 | 18.3 | 1 | 50 | 5.5 | 1480 | 1.9 | 347 | 2.0 | 159.4 | 113.5 |
| | CP2 | 16.1 | 6 | 120 | 15.0 | 4042 | 19.0 | 190 | 2.1 | 156.2 | 113.8 |
| | CP3 | 13.6 | 15 | 114 | 16.8 | 4531 | 120 | 106 | 2.2 | 156.6 | 116.5 |
| CE1 | CP4 | 11.1 | 1 | 99 | 9.0 | 2487 | 9.4** | 574.0 | 2.5 | 156.5 | 111.9 |
| | CP5 | 5.5 | 6 | 100 | 18.2 | 5051 | 1.9 | 302.0 | 2.4 | 157.5 | 112.9 |
| | CP6 | 10.7 | 15 | 218 | 20.4 | 5659 | 21.0 | 179.0 | 2.4 | 156.6 | 112.7 |
| IE1 | IP1 | 14.9 | 1 | 406 | 27.2 | 7165 | 30.0 ** | 495.0 | 2.6 | 155.6 | 112.7 |
| | IP2 | 12.5 | 6 | 547 | 43.7 | 11509 | 3.2 | 289.0 | 2.5 | 156.7 | 111.4 |
| | IP3 | 8.5 | 15 | 466 | 54.8 | 14421 | 37.3 | 157.0 | 2.5 | 156.9 | 114.5 |
| IE2 | IP4 | 10.0 | 1 | 245 | 24.5 | 5688 | 32.6** | 611.0 | 2.4 | 156.4 | 109.7 |
| | IP5 | 10.0 | 6 | 398 | 39.8 | 9244 | 2.9 | 329.0 | 2.3 | 157.5 | 111.6 |
| | IP6 | 8.2 | 15 | 437 | 53.3 | 12391 | 26.9 | 175.0 | 2.8 | 156.3 | 113.4 |
| IE3 | IP7 | 10.8 | 1 | 142 | 13.2 | 3990 | 20.1** | 640.0 | 2.6 | 155.6 | 109.2 |
| | IP8 | 12.6 | 6 | 333 | 26.4 | 7997 | 2.0 | 324.0 | 2.5 | 156.3 | 110.5 |
| | IP9 | 9.2 | 15 | 311 | 33.8 | 10231 | 24.1 | 189.0 | 2.6 | 155.0 | 111.5 |

| Catalyst | Ex | Cat. (mg) | $H_2$ mmol | Pol. Yield, g | Act cat kg/gcat/h | Act Metal kg/gmetal/h | $MFR_2$ g/10min | $M_w$ kg/mol | $M_w/M_n$ | $T_m$ (°C) | $T_c$ (°C) |
|---|---|---|---|---|---|---|---|---|---|---|---|
| IE4 | IP10 | 14.5 | 1 | 265 | 18.3 | 4809 | 25.0** | 560.0 | 2.4 | 155.3 | 111.6 |
| | IP11 | 12.7 | 6 | 423 | 33.3 | 8771 | 120.0** | 344.0 | 2.4 | 156.0 | 111.0 |
| | IP12 | 10.8 | 15 | 468 | 43.4 | 11408 | 16.0 | 186.0 | 2.6 | 155.6 | 112.7 |

* Procedure A for (Polymerisations CP1-CP3) and Procedure B for (Polymerisations CP4-CP6, IP1-IP12)

** $MFR_{21}$ (g/10min)

It can be seen from table 3 above that using catalysts of the invention the activity is clearly higher using the same amount of $H_2$ in the polymerisation when inventive catalysts are compared to the results obtained using comparative catalysts. The polymers still have higher Mw (lower MFR) and possess high melting points.

Table 4 summarises the important results herein succinctly.

Table 4

| **MC/ catalyst** | **R$^6$** | Polymerisation example | **Activity** (kg-PP/g-cat/h) | **Activity** (kg-PP/g-Zr/h) | **Mw** (kg/mol) | **$M_W/M_n$** | **Tm** (°C) |
|---|---|---|---|---|---|---|---|
| C-mc1/ CE1 | - | CP5 | 18.2 | 5051 | 302 | 2.4 | 157.5 |
| C-mc2 CE2 | - * | CP1 | 5.5 | 1480 | 347 | 2.0 | 159.4 |
| I-mc1/ IE1 | methyl | IP2 | 43.7 | 11509 | 289 | 2.5 | 156.7 |
| I-mc2/ IE2 | ethyl | IP5 | 39.8 | 9244 | 329 | 2.3 | 157.5 |
| I-mc3/ IE3 | *iso*-propyl | IP8 | 26.4 | 7997 | 324 | 2.5 | 156.3 |
| I-mc4/ IE4 | *neo*-pentyl | IP11 | 33.3 | 8771 | 344 | 2.4 | 156.0 |
| * with 7-methoxy | | | | | | | |

**Claims**

**1.** A catalyst in solid particulate form free from an external carrier material comprising

(i) a complex of formula (I)

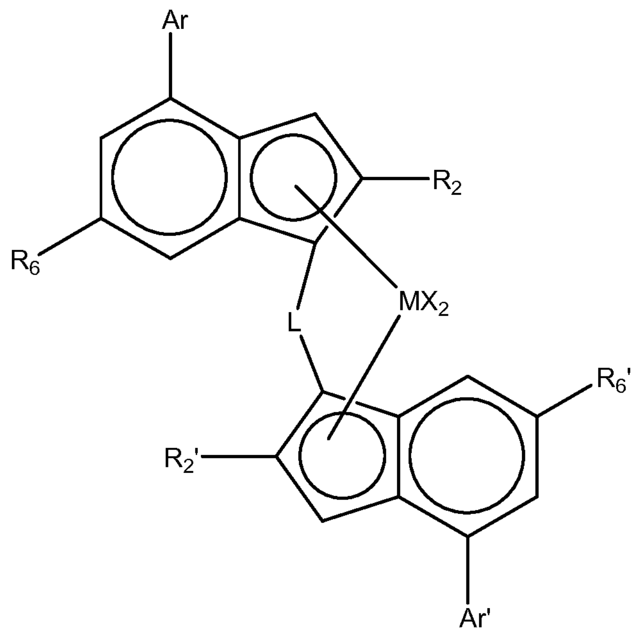

wherein

M is zirconium or hafnium;
each X is a sigma ligand;
L is a divalent bridge selected from $-R'_2C-$, $-R'_2C-CR'_2-$, $-R'_2Si-$, $-R'_2Si-SiR'_2-$,$-R'_2Ge-$, wherein each R' is independently a hydrogen atom, $C_{1-20}$-alkyl, tri($C_{1-20}$-alkyl)silyl, $C_{6-20}$-aryl, $C_{7-20}$-arylalkyl or $C_{7-20}$-alkylaryl;
$R_2$ is a $C_{1-20}$-hydrocarbyl radical;
$R_{2'}$ is a $C_{1-20}$-hydrocarbyl radical;
$R_6$ is a linear or branched aliphatic $C_{1-20}$-hydrocarbyl group, $SR_9$ or $OR_9$;

$R_{6'}$ is a linear or branched aliphatic $C_{1-20}$-hydrocarbyl group, $SR_9$, or $OR_{9'}$;
with the proviso that neither $R_6$ or $R_{6'}$ represents a group having a quaternary carbon atom directly attached to the indenyl ring;
$R_9$ is a $C_{1-20}$-hydrocarbyl group;
$R_{9'}$ is a $C_{1-20}$-hydrocarbyl group;
Ar is a $C_{6-12}$-aryl or $C_{5-12}$-heteroaryl group optionally carrying one or more substituents $R_8$;
Ar' is a $C_{6-12}$-aryl or $C_{5-12}$-heteroaryl group optionally carrying one or more substituents $R_{8'}$;
each $R_8$ is a $C_{1-20}$-hydrocarbyl group;
each $R_{8'}$, is a $C_{1-20}$-hydrocarbyl group;
wherein at least two of $R_2$ and $R_{2'}$; $R_6$ and $R_{6'}$; or Ar and Ar' are the same;

and (ii) a cocatalyst comprising a compound of a group 13 metal, e.g. Al or boron.

**2.** The catalyst of claim 1 which is obtainable by a process in which

(a) a liquid/liquid emulsion system is formed, said liquid/liquid emulsion system comprising a solution of the catalyst components (i) and (ii) dispersed in a solvent so as to form dispersed droplets; and
(b) solid particles are formed by solidifying said dispersed droplets.

**3.** A catalyst as claimed in any preceding claim comprising a symmetrical complex of formula (II)

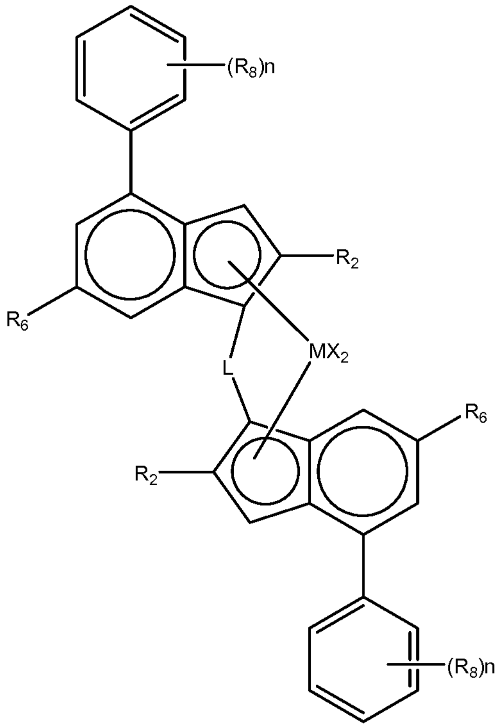

(II)

wherein

M is zirconium or hafnium;
each X is a sigma ligand;
L is a divalent bridge selected from $-R'_2C-$, $-R'_2C-CR'_2-$, $-R'_2Si-$, $-R'_2Si-SiR'_2-$,$-R'_2Ge-$, wherein each R' is independently a hydrogen atom, $C_{1-20}$-alkyl, tri($C_{1-20}$-alkyl)silyl, $C_{6-20}$-aryl, $C_{7-20}$-arylalkyl or $C_{7-20}$-alkylaryl;
$R_2$ is a $C_{1-20}$-hydrocarbyl radical;
$R_6$ is a linear or branched aliphatic $C_{1-20}$-hydrocarbyl group, $SR_9$ or $OR_9$;
with the proviso that $R_6$ does not represent a group having a quaternary carbon atom directly attached to the indenyl ring;
$R_9$ is a $C_{1-20}$-hydrocarbyl group;
n is independently 1, 2 or 3; and

each $R_8$ is a $C_{1-20}$-hydrocarbyl group.

**4.** A catalyst as claimed in any preceding claim comprising a symmetrical complex of formula (III)

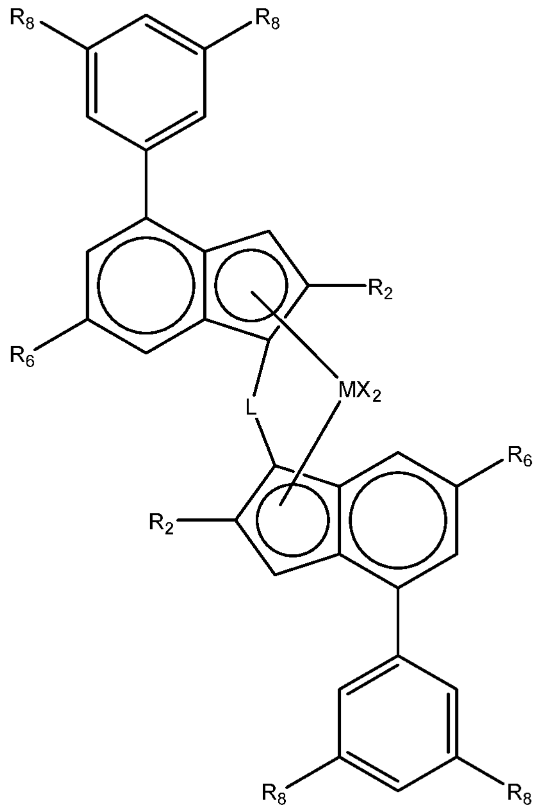

(III)

wherein

M is zirconium or hafnium;
each X is a sigma ligand, preferably each X is independently a hydrogen atom, a halogen atom, $C_{1-6}$-alkoxy group, $C_{1-6}$-alkyl, phenyl or benzyl group;
L is a divalent bridge selected from $-R'_2C-$, $-R'_2C-CR'_2-$, $-R'_2Si-$, $-R'_2Si-SiR'_2-$,$-R'_2Ge-$, wherein each R' is independently a hydrogen atom, $C_{1-20}$-alkyl, tri($C_{1-20}$-alkyl)silyl, $C_{6-20}$-aryl, $C_{7-20}$-arylalkyl or $C_{7-20}$-alkylaryl; preferably dimethylsilyl, methylene or ethylene;
$R_2$ is a $C_{1-10}$-alkyl group;
$R_6$ is a $C_{1-10}$-alkyl group with the proviso that $R_6$ does not represent a group having a quaternary carbon atom directly attached the indenyl ring;
and each $R^8$ is a $C_{1-20}$-hydrocarbyl group.

**5.** A catalyst as claimed in any preceding claim comprising a symmetrical complex of formula (IV):

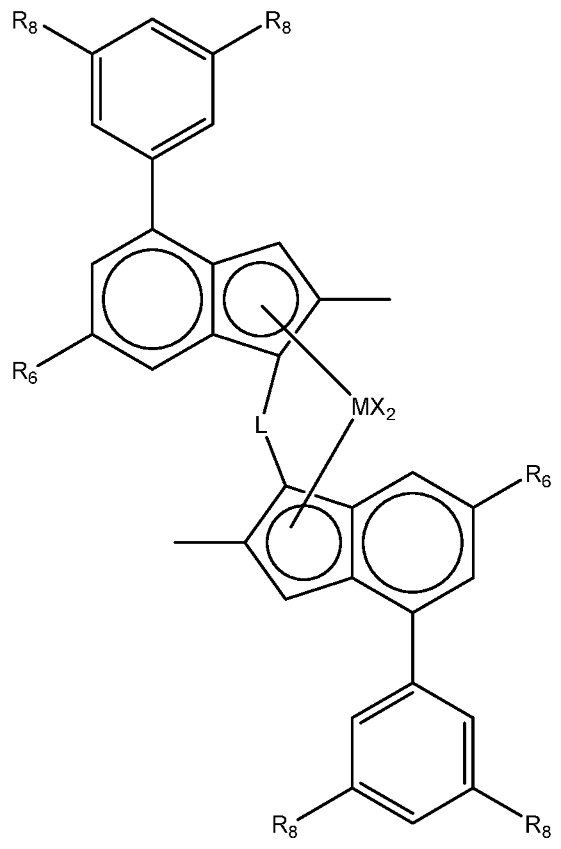

(IV)

wherein

M is zirconium or hafnium;
each X is a sigma ligand, preferably each X is independently a hydrogen atom, a halogen atom, $C_{1-6}$-alkoxy group, $C_{1-6}$-alkyl, phenyl or benzyl group;
L is a divalent bridge selected from $-R'_2C-$, $-R'_2C-CR'_2-$, $-R'_2Si-$, $-R'_2Si-SiR'_2-$,$-R'_2Ge-$, wherein each R' is independently a hydrogen atom, $C_{1-20}$-hydrocarbyl, tri($C_{1-20}$-alkyl)silyl, $C_{6-20}$-aryl, $C_{7-20}$-arylalkyl or $C_{7-20}$-alkylaryl; preferably dimethylsilyl;
$R_6$ is a linear $C_{1-10}$-alkyl group, $-CH(C_{1-4}\text{-alkyl})_2$ group or $CH_2(C_{1-9}\text{-alkyl})$ group, where said $C_{1-4}$-alkyl and $C_{1-9}$-alkyl groups can be linear or branched; and
each $R^8$ is a $C_{1-10}$-alkyl group.

**6.** A catalyst as claimed in any preceding claim comprising a symmetrical complex of formula (V)

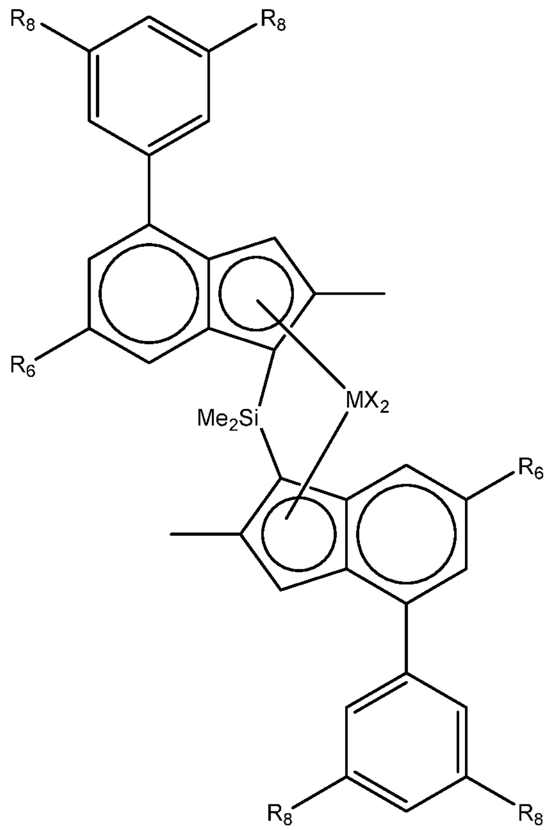

(V)

wherein M is Zr/Hf;
X is a hydrogen atom, a halogen atom, $C_{1-6}$-alkoxy group, $C_{1-6}$-alkyl, phenyl or benzyl group;
$R_6$ is linear $C_{1-6}$-alkyl, -CH($C_{1-4}$-alkyl)$_2$ or -$CH_2$($C_{1-6}$-alkyl), where said $C_{1-4}$-alkyl and $C_{1-6}$-alkyl groups can be linear or branched; and
$R_8$ is $C_{3-8}$-alkyl, such as branched $C_{3-8}$-alkyl.

**7.** A catalyst as claimed in any preceding claim comprising a symmetrical complex of formula (VI):

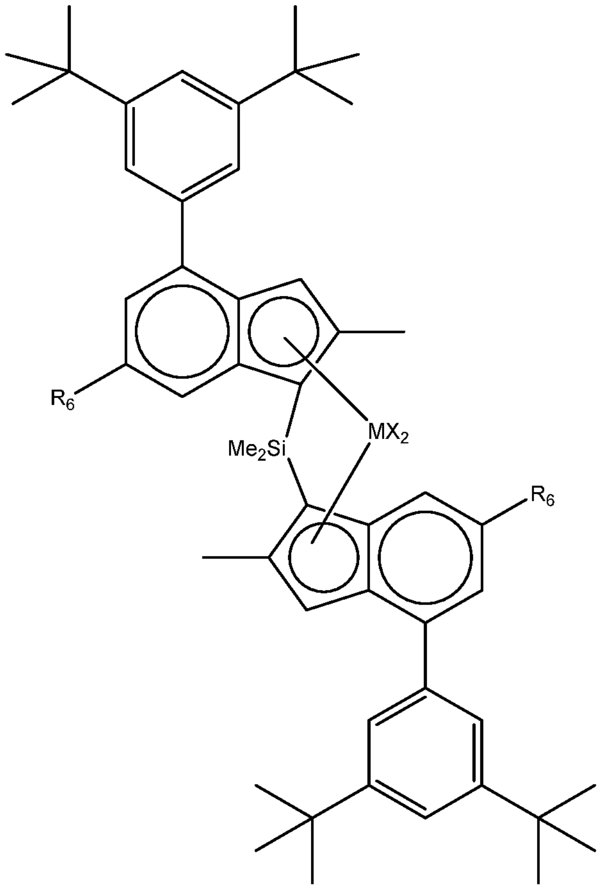

(VI)

wherein M is Zr/Hf
X is a hydrogen atom, a halogen atom, $C_{1-6}$-alkoxy group, $C_{1-6}$-alkyl, phenyl or benzyl group;
$R_6$ is linear $C_{1-6}$-alkyl, -CH($C_{1-4}$-alkyl)$_2$ or -$CH_2$($C_{1-6}$-alkyl) where said $C_{1-4}$-alkyl and $C_{1-6}$-alkyl groups can be linear or branched.

**8.** A catalyst as claimed in any preceding claim comprising the complex *Rac*-dimethylsilanediylbis[2,6-dimethyl-4-(3,5-di-*tert*-butylphenyl)-inden-1-yl]zirconium dichloride *Rac*-dimethylsilanediylbis[2-methyl-4-(3,5-di-*tert*-butylphenyl)-6-ethyl-inden-1-yl]zirconium dichloride *Rac*-dimethylsilanediylbis[2-methyl-4-(3,5-di-*tert*-butylphenyl)-6-isopropyl-inden-1-yl]zirconium dichloride *Rac*-dimethylsilanediylbis[2-methyl-4-(3,5-di-*tert*-butylphenyl)-6-neopentyl-inden-1-yl]zirconium dichloride
or their Hf analogues.

**9.** A catalyst as claimed in any preceding claim wherein said cocatalyst is MAO.

**10.** A catalyst as claimed in any preceding claim wherein the two ligands forming the complex are identical.

**11.** A complex as claimed in any of claims 3 to 10 being a symmetrical complex of formula (II)

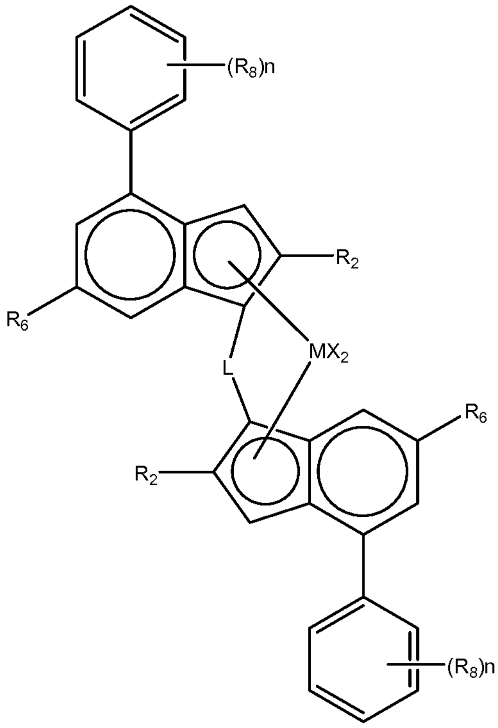

(II)

wherein

M is zirconium or hafnium;
each X is a sigma ligand;
L is a divalent bridge selected from $-R'_2C-$, $-R'_2C-CR'_2-$, $-R'_2Si-$, $-R'_2Si-SiR'_2-$,$-R'_2Ge-$, wherein each R' is independently a hydrogen atom, $C_{1-20}$-alkyl, tri($C_{1-20}$-alkyl)silyl, $C_{6-20}$-aryl, $C_{7-20}$-arylalkyl or $C_{7-20}$-alkylaryl;
$R_2$ is a $C_{1-20}$-hydrocarbyl radical;
$R_6$ is a linear or branched aliphatic $C_{1-20}$-hydrocarbyl group, $SR_9$ or $OR_9$;
with the proviso that $R_6$ does not represent a group having a quaternary carbon atom directly attached to the indenyl ring;
$R_9$ is a $C_{1-20}$-hydrocarbyl group;
n is independently 1, 2 or 3; and
each $R_8$ is a $C_{1-20}$-hydrocarbyl group.

**12.** A process for the manufacture of a catalyst as claimed in any of claims 1 to 10 comprising obtaining a (i) complex of formula (I) and (ii) a cocatalyst comprising a compound of a group 13 metal, e.g. Al or boron;
forming a liquid/liquid emulsion system, which comprises a solution of catalyst components (i) and (ii) dispersed in a solvent, and solidifying said dispersed droplets to form solid particles.

**13.** A process for the polymerisation of propylene comprising polymerising propylene with a catalyst as claimed in any of claims 1 to 10.

**14.** A process as claimed in claim 13 wherein said process forms an isotactic polypropylene.

**15.** A process as claimed in claim 14 wherein the catalyst activity in said process is at least 10.0 kg/g(cat)/h.

**Patentansprüche**

**1.** Katalysator in fester partikulärer Form und frei von einem externen Trägermaterial, umfassend

(i) einen Komplex mit der Formel (I):

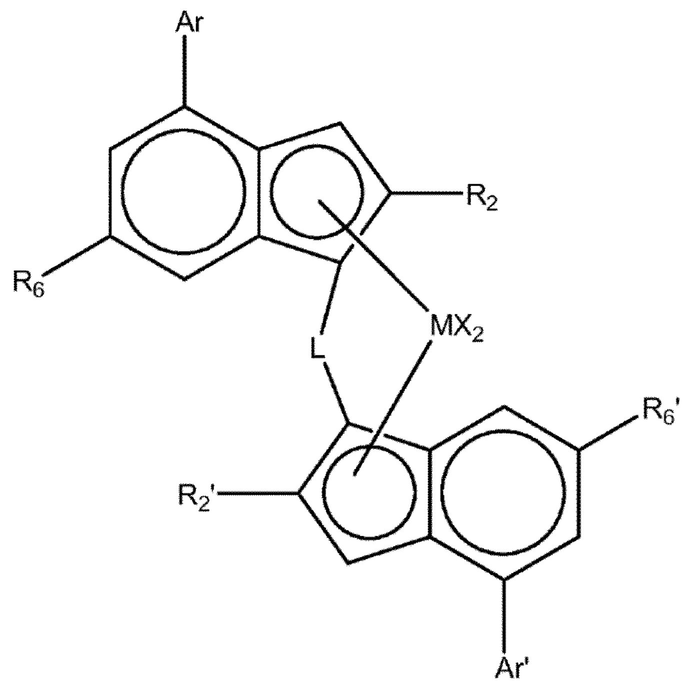

wobei

M Zirconium oder Hafnium ist;
jeder X ein Sigma-Ligand ist;
L eine divalente Brücke ist, die aus $-R'_2C-$, $-R'_2C-CR'_2-$, $-R'_2Si-$, $-R'_2Si-SiR'_2-$, $-R'_2Ge-$ ausgewählt ist, wobei jeder R' unabhängig ein Wasserstoffatom, $C_{1-20}$-Alkyl, Tri($C_{1-20}$-alkyl)silyl, $C_{6-20}$-Aryl, $C_{7-20}$-Arylalkyl oder $C_{7-20}$-Alkylaryl ist;
$R_2$ ein $C_{1-20}$-Hydrocarbylradikal ist;
$R_2'$ ein $C_{1-20}$-Hydrocarbylradikal ist;
$R_6$ eine lineare oder verzweigte aliphatische $C_{1-20}$-Hydrocarbylgruppe, $SR_9$ oder $OR_9$ ist;
$R_6'$ eine lineare oder verzweigte aliphatische $C_{1-20}$-Hydrocarbylgruppe, $SR_9'$ oder $OR_9'$ ist;
unter der Voraussetzung, dass weder $R_6$ noch $R_6'$ eine Gruppe repräsentiert, die ein quartäres Kohlenstoffatom aufweist, das direkt an den Indenylring gebunden ist;
$R_9$ eine $C_{1-20}$-Hydrocarbylgruppe ist;
$R_9'$ eine $C_{1-20}$-Hydrocarbylgruppe ist;
Ar eine $C_{6-12}$-Aryl- oder $C_{5-12}$-Heteroarylgruppe ist, die optional ein oder mehrere Substituenten $R_8$ trägt;
Ar' eine $C_{6-12}$-Aryl- oder $C_{5-12}$-Heteroarylgruppe ist, die optional ein oder mehrere Substituenten $R_8'$ trägt;
jeder $R_8$ eine $C_{1-20}$-Hydrocarbylgruppe ist;
jeder $R_8'$ eine $C_{1-20}$-Hydrocarbylgruppe ist;
wobei mindestens zwei von $R_2$ und $R_2'$; $R_6$ und $R_6'$, oder Ar und Ar' gleich sind;

und (ii) einen Cokatalysator, der eine Verbindung von einem Metall der Gruppe 13, z.B. Al oder Bor, umfasst.

**2.** Katalysator nach Anspruch 1, der durch ein Verfahren erhältlich ist, in dem

(a) ein Flüssig/Flüssig-Emulsionssystem gebildet wird, wobei das Flüssig/Flüssig-Emulsionssystem eine Lösung aus den Katalysatorkomponenten (i) und (ii) umfasst, die in einem Lösungsmittel dispergiert sind, um so dispergierte Tröpfchen zu bilden; und
(b) feste Partikel gebildet werden, indem die dispergierten Tröpfchen verfestigt werden.

**3.** Katalysator nach einem der vorhergehenden Ansprüche, umfassend einen symmetrischen Komplex mit der Formel (II):

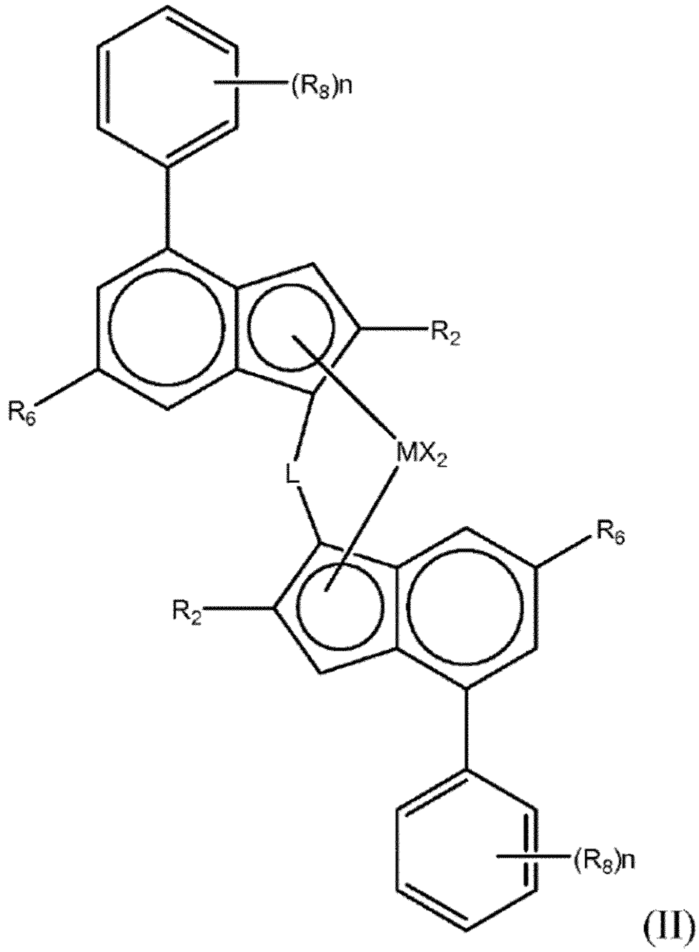

(II)

wobei

M Zirconium oder Hafnium ist;
jeder X ein Sigma-Ligand ist;
L eine divalente Brücke ist, die aus $-R'_2C-$, $-R'_2C-CR'_2-$, $-R'_2Si-$, $-R'_2Si-SiR'_2-$, $-R'_2Ge-$ ausgewählt ist, wobei jeder R' unabhängig ein Wasserstoffatom, $C_{1-20}$-Alkyl, Tri($C_{1-20}$-alkyl)silyl, $C_{6-20}$-Aryl, $C_{7-20}$-Arylalkyl oder $C_{7-20}$-Alkylaryl ist;
$R_2$ ein $C_{1-20}$-Hydrocarbylradikal ist;
$R_6$ eine lineare oder verzweigte aliphatische $C_{1-20}$-Hydrocarbylgruppe, $SR_9$ oder $OR_9$ ist;
unter der Voraussetzung, dass $R_6$ keine Gruppe repräsentiert, die ein quartäres Kohlenstoffatom aufweist, das an den Indenylring gebunden ist;
$R_9$ eine $C_{1-20}$-Hydrocarbylgruppe ist;
n unabhängig 1, 2 oder 3 ist; und
jeder $R_8$ eine $C_{1-20}$-Hydrocarbylgruppe ist.

**4.** Katalysator nach einem der vorhergehenden Ansprüche, umfassend einen symmetrischen Komplex mit der Formel (III):

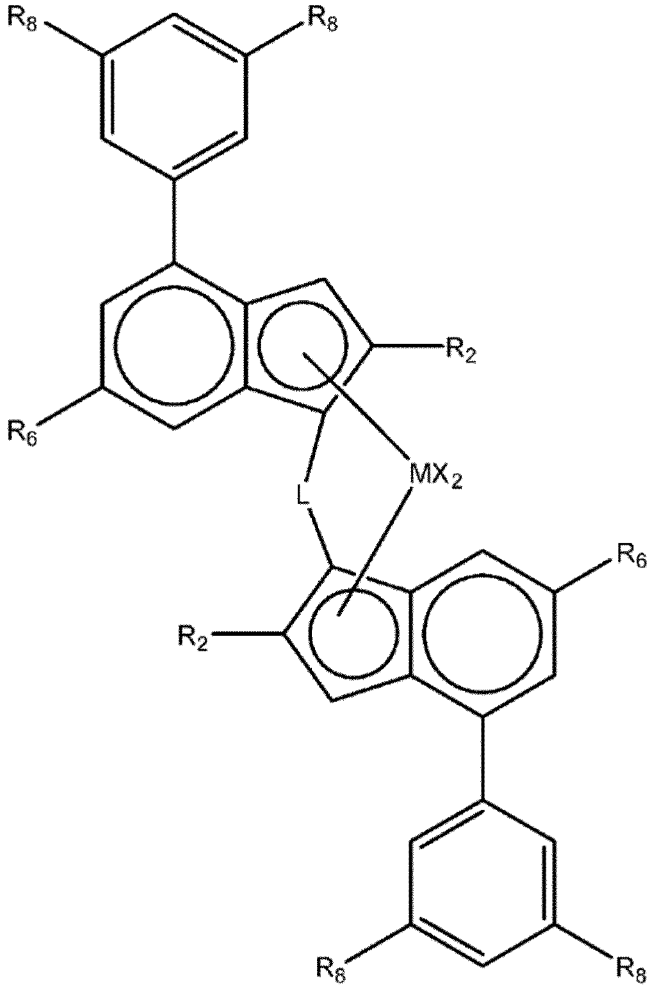

(III)

wobei

M Zirconium oder Hafnium ist;
jeder X ein Sigma-Ligand ist, vorzugsweise jeder X unabhängig ein Wasserstoffatom, ein Halogenatom, eine $C_{1-6}$-Alkoxygruppe, $C_{1-6}$-Alkylgruppe, Phenylgruppe oder Benzyl-Gruppe ist;
L eine divalente Brücke ist, die aus $-R'_2C-$, $-R'_2C-CR'_2-$, $-R'_2Si-$, $-R'_2Si-SiR'_2-$, $-R'_2Ge-$ ausgewählt ist, wobei jeder R' unabhängig ein Wasserstoffatom, $C_{1-20}$-Alkyl, Tri($C_{1-20}$-alkyl)silyl, $C_{6-20}$-Aryl, $C_{7-20}$-Arylalkyl oder $C_{7-20}$-Alkylaryl ist; vorzugsweise Dimethylsilyl, Methylen oder Ethylen;
$R_2$ eine $C_{1-10}$-Alkylgruppe ist;
$R_6$ eine $C_{1-10}$-Alkylgruppe ist, unter der Voraussetzung, dass $R_6$ keine Gruppe repräsentiert, die ein quartäres Kohlenstoffatom aufweist, das direkt an den Indenylring gebunden ist;
und jeder $R^8$ eine $C_{1-20}$-Hydrocarbylgruppe ist.

**5.** Katalysator nach einem der vorhergehenden Ansprüche, umfassend einen symmetrischen Komplex mit der Formel (IV):

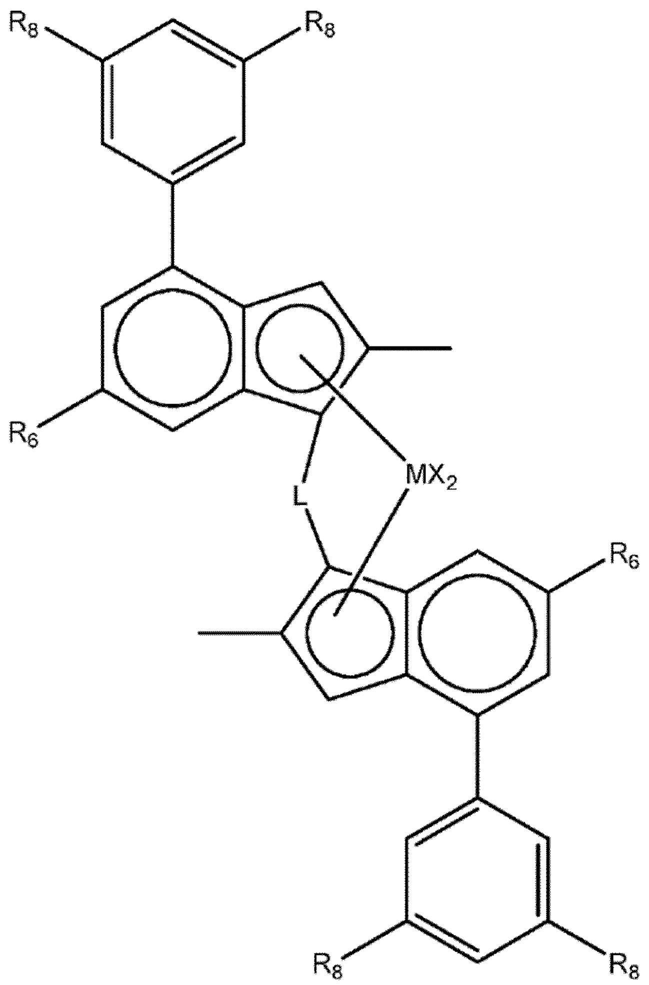

(IV)

wobei

M Zirconium oder Hafnium ist;
jeder X ein Sigma-Ligand ist, vorzugsweise jeder X unabhängig ein Wasserstoffatom, ein Halogenatom, eine $C_{1-6}$-Alkoxygruppe, $C_{1-6}$-Alkylgruppe, Phenylgruppe oder Benzyl-Gruppe ist;
L eine divalente Brücke ist, die aus $-R'_2C-$, $-R'_2C-CR'_2-$, $-R'_2Si-$, $-R'_2Si-SiR'_2-$, $-R'_2Ge-$ ausgewählt ist, wobei jeder R' unabhängig ein Wasserstoffatom, $C_{1-20}$-Hydrocarbyl, Tri($C_{1-20}$-alkyl)silyl, $C_{6-20}$-Aryl, $C_{7-20}$-Arylalkyl oder $C_{7-20}$-Alkylaryl ist; vorzugsweise Dimethylsilyl;
$R_6$ eine lineare $C_{1-10}$-Alkylgruppe, $-CH(C_{1-4}\text{-alkyl})_2$-Gruppe oder $CH_2(C_{1-9}\text{-alkyl})$-Gruppe ist, wobei die $C_{1-4}$-Alkyl- und $C_{1-9}$-Alkylgruppen linear oder verzweigt sein können; und
jeder $R^8$ eine $C_{1-10}$-Alkylgruppe ist.

**6.** Katalysator nach einem der vorhergehenden Ansprüche, umfassend einen symmetrischen Komplex mit der Formel (V):

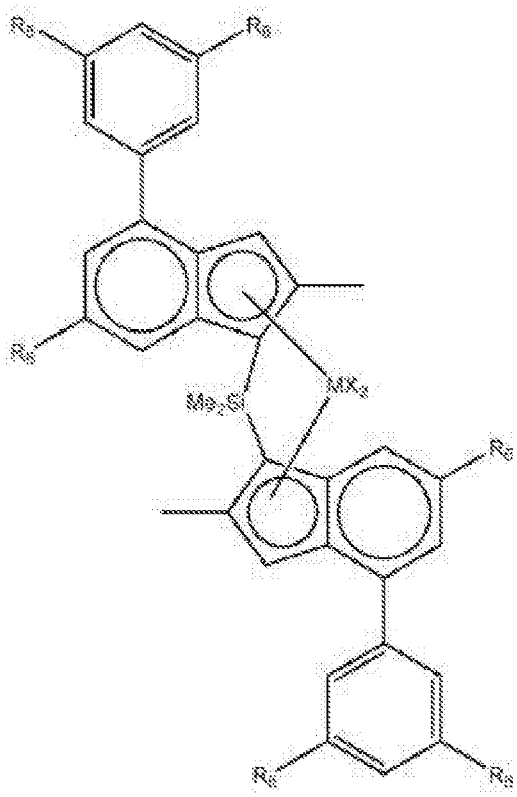

(V)

wobei

M Zr/Hf ist;
X ein Wasserstoffatom, ein Halogenatom, eine $C_{1-6}$-Alkoxygruppe, $C_{1-6}$-Alkylgruppe, Phenylgruppe oder Benzyl-Gruppe ist;
$R_6$ ein lineares $C_{1-6}$-Alkyl, $-CH(C_{1-4}\text{-alkyl})_2$ oder $-CH_2(C_{1-6}\text{-alkyl})$ ist, wobei die $C_{1-4}$-Alkyl- und $C_{1-6}$-Alkylgruppen linear oder verzweigt sein können; und
$R^8$ ein $C_{3-8}$-Alkyl ist, wie ein verzweigtes $C_{3-8}$-Alkyl.

**7.** Katalysator nach einem der vorhergehenden Ansprüche, umfassend einen symmetrischen Komplex mit der Formel (VI):

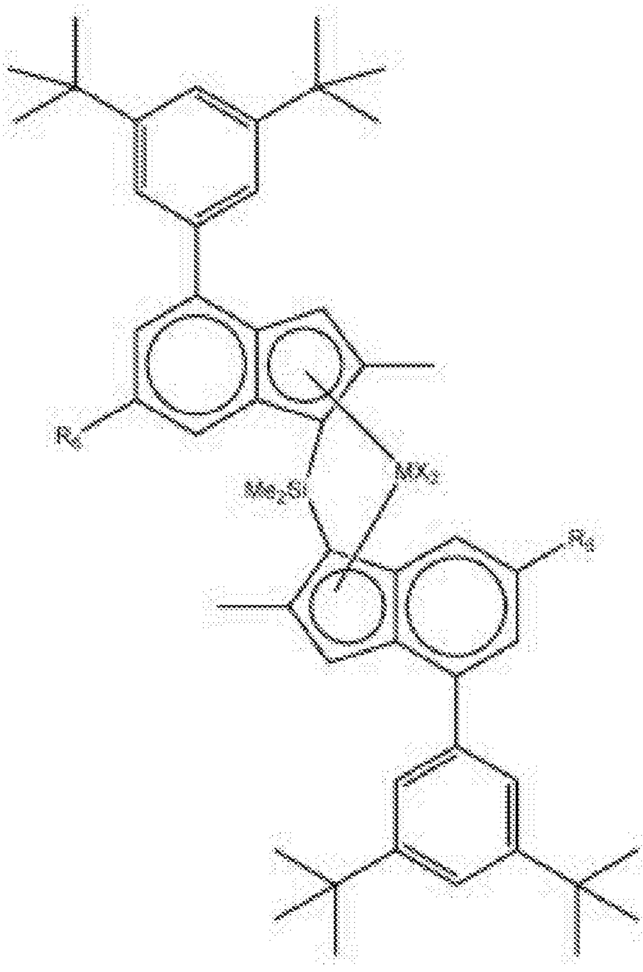

(VI)

wobei

M Zr/Hf ist;
X ein Wasserstoffatom, ein Halogenatom, eine $C_{1-6}$-Alkoxygruppe, $C_{1-6}$-Alkylgruppe, Phenylgruppe oder Benzyl-Gruppe ist;
$R_6$ ein lineares $C_{1-6}$-Alkyl, -CH($C_{1-4}$-alkyl)$_2$ oder -$CH_2$($C_{1-9}$-alkyl) ist, wobei die $C_{1-4}$-Alkyl- und $C_{1-9}$-Alkylgruppen linear oder verzweigt sein können.

**8.** Katalysator nach einem der vorhergehenden Ansprüche, umfassend den Komplex *Rac*-Dimethylsilandiylbis[2,6-dimethyl-4-(3,5-di-*tert*-butylphenyl)-inden-1-yl]zirconiumdichlorid, *Rac*-Dimethylsilandiylbis[2-methyl-4-(3,5-di-*tert*-butylphenyl)-6-ethyl-inden-1-yl]zirconiumdichlorid, *Rac*-Dimethylsilandiylbis[2-methyl-4-(3,5-di-*tert*-butylphenyl)-6-isopropyl-inden-1-yl]zirconiumdichlorid, *Rac*-Dimethylsilandiylbis[2-methyl-4-(3,5-di-*tert*-butylphenyl)-6-neopenthyl-inden-1-yl]zirconiumdichlorid
oder deren Hf-Analoga.

**9.** Katalysator nach einem der vorhergehenden Ansprüche, wobei der Cokatalysator MAO ist.

**10.** Katalysator nach einem der vorhergehenden Ansprüche, wobei die zwei Liganden, die den Komplex bilden, identisch sind.

**11.** Katalysator nach einem der Ansprüche 3 bis 10, umfassend einen symmetrischen Komplex mit der Formel (II)

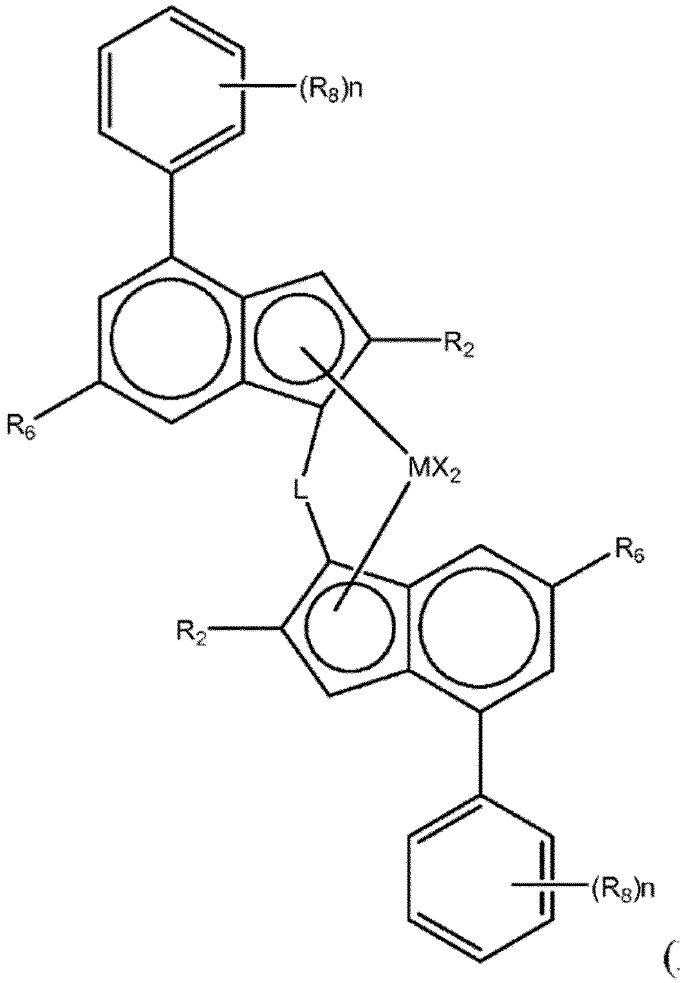

(II)

wobei

M Zirconium oder Hafnium ist;
jeder X ein Sigma-Ligand ist;
L eine divalente Brücke ist, die aus $-R'_2C-$, $-R'_2C-CR'_2-$, $-R'_2Si-$, $-R'_2Si-SiR'_2-$, $-R'_2Ge-$ ausgewählt ist, wobei jeder R' unabhängig ein Wasserstoffatom, $C_{1-20}$-Alkyl, Tri($C_{1-20}$-alkyl)silyl, $C_{6-20}$-Aryl, $C_{7-20}$-Arylalkyl oder $C_{7-20}$-Alkylaryl ist;
$R_2$ ein $C_{1-20}$-Hydrocarbylradikal ist;
$R_6$ eine lineare oder verzweigte aliphatische $C_{1-20}$-Hydrocarbylgruppe, $SR_9$ oder $OR_9$ ist;
unter der Voraussetzung, dass $R_6$ keine Gruppe repräsentiert, die ein quartäres Kohlenstoffatom aufweist, das direkt an den Indenylring gebunden ist;
$R_9$ eine $C_{1-20}$-Hydrocarbylgruppe ist;
n unabhängig 1, 2 oder 3 ist; und
jeder $R_8$ eine $C_{1-20}$-Hydrocarbylgruppe ist.

**12.** Verfahren für die Herstellung von einem Katalysator nach einem der Ansprüche 1 bis 10, umfassend das Erhalten von (i) einem Komplex mit der Formel (I) und (ii) einem Cokatalysator, der eine Verbindung von einem Metall der Gruppe 13, z.B. Al oder Bor, umfasst;
Bilden von einem Flüssig/Flüssig-Emulsionssystem, das eine Lösung aus den Katalysatorkomponenten (i) und (ii), dispergiert in einem Lösungsmittel, umfasst, und Verfestigen von den dispergierten Tröpfchen, um feste Partikel zu bilden.

**13.** Verfahren für die Polymerisation von Propylen, umfassend das Polymerisieren von Propylen mit einem Katalysator nach einem der Ansprüche 1 bis 10.

**14.** Verfahren nach Anspruch 13, wobei das Verfahren ein isotaktisches Polypropylen bildet.

**15.** Verfahren nach Anspruch 14, wobei die Katalysatoraktivität in dem Verfahren mindestens 10,0 kg/g(kat)/h beträgt.

**Revendications**

**1.** Catalyseur sous forme particulaire solide exempt d'un matériau de support externe comprenant

(i) un complexe de formule (I)

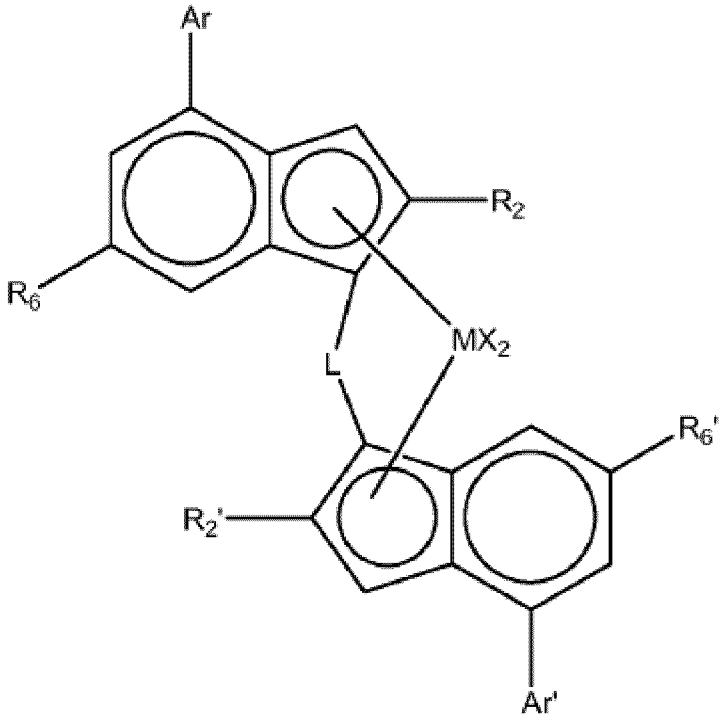

dans lequel

M est un zirconium ou un hafnium ;
chaque X est un ligand sigma ;
L est un pont divalent sélectionné parmi -R'$_2$C-, -R'$_2$C-CR'$_2$-, -R'$_2$Si-, -R'$_2$Si-SiR'$_2$-, -R'$_2$Ge-, dans lesquels chaque R' est indépendamment un atome d'hydrogène, un alkyle en $C_1$ à $C_{20}$, un tri(alkyle en $C_1$ à $C_{20}$)silyle, un aryle en $C_6$ à $C_{20}$, un arylalkyle en $C_7$ à $C_{20}$ ou un alkylaryle en $C_7$ à $C_{20}$ ;
$R_2$ est un radical hydrocarbyle en $C_1$ à $C_{20}$ ;
$R_{2'}$ est un radical hydrocarbyle en $C_1$ à $C_{20}$ ;
$R_6$ est un groupe hydrocarbyle en $C_1$ à $C_{20}$ aliphatique linéaire ou ramifié, $SR_9$ ou $OR_9$ ;
$R_{6'}$ est un groupe hydrocarbyle en $C_1$ à $C_{20}$ aliphatique linéaire ou ramifié, $SR_{9'}$ ou $OR_{9'}$ ;
à condition que ni $R_6$, ni $R_6$, ne représentent un groupe contenant un atome de carbone quaternaire directement lié au cycle indényle ;
$R_9$ est un groupe hydrocarbyle en $C_1$ à $C_{20}$ ;
$R_{9'}$ est un groupe hydrocarbyle en $C_1$ à $C_{20}$ ;
Ar est un groupe aryle en $C_6$ à $C_{12}$ ou hétéroaryle en $C_5$ à $C_{12}$ portant facultativement un ou plusieurs substituants $R_8$ ;
Ar' est un groupe aryle en $C_6$ à $C_{12}$ ou hétéroaryle en $C_5$ à $C_{12}$ portant facultativement un ou plusieurs substituants $R_{8'}$ ;
chaque $R_8$ est un groupe hydrocarbyle en $C_1$ à $C_{20}$ ;
chaque $R_{8'}$ est un groupe hydrocarbyle en $C_1$ à $C_{20}$ ;
dans lequel au moins deux parmi $R_2$ et $R_{2'}$ ; $R_6$ et $R_{6'}$ ; ou Ar et Ar' sont identiques ;

et (ii) un cocatalyseur comprenant un composé d'un métal du groupe 13, par exemple Al ou bore.

**2.** Catalyseur selon la revendication 1 pouvant être obtenu par un procédé dans lequel

(a) un système d'émulsion liquide/liquide est formé, ledit système d'émulsion liquide/liquide comprenant une solution des composants (i) et (ii) du catalyseur dispersés dans un solvant afin de former des gouttelettes dispersées ; et
(b) des particules solides sont formées par la solidification desdites gouttelettes dispersées.

**3.** Catalyseur selon n'importe quelle revendication précédente comprenant un complexe symétrique de formule (II)

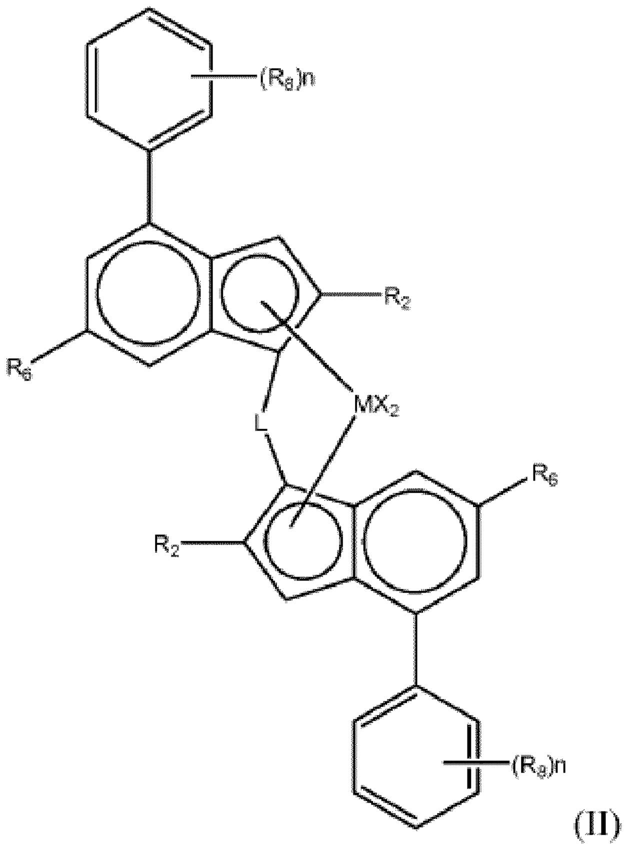

(II)

dans lequel

M est un zirconium ou un hafnium ;
chaque X est un ligand sigma ;
L est un pont divalent sélectionné parmi $-R'_2C-$, $-R'_2C-CR'_2-$, $-R'_2Si-$, $-R'_2Si-SiR'_2-$, $-R'_2Ge-$, dans lesquels chaque R' est indépendamment un atome d'hydrogène, un alkyle en $C_1$ à $C_{20}$, un tri(alkyle en $C_1$ à $C_{20}$)silyle, un aryle en $C_6$ à $C_{20}$, un arylalkyle en $C_7$ à $C_{20}$ ou un alkylaryle en $C_7$ à $C_{20}$ ;
$R_2$ est un radical hydrocarbyle en $C_1$ à $C_{20}$ ;
$R_6$ est un groupe hydrocarbyle en $C_1$ à $C_{20}$ aliphatique linéaire ou ramifié, $SR_9$ ou $OR_9$ ;
à condition que $R_6$ ne représente pas un groupe contenant un atome de carbone quaternaire directement lié au cycle indényle ;
$R_9$ est un groupe' hydrocarbyle en $C_1$ à $C_{20}$ ;
n est indépendamment 1, 2 ou 3 ; et
chaque $R_8$ est un groupe hydrocarbyle en $C_1$ à $C_{20}$.

**4.** Catalyseur selon n'importe quelle revendication précédente comprenant un complexe symétrique de formule (III)

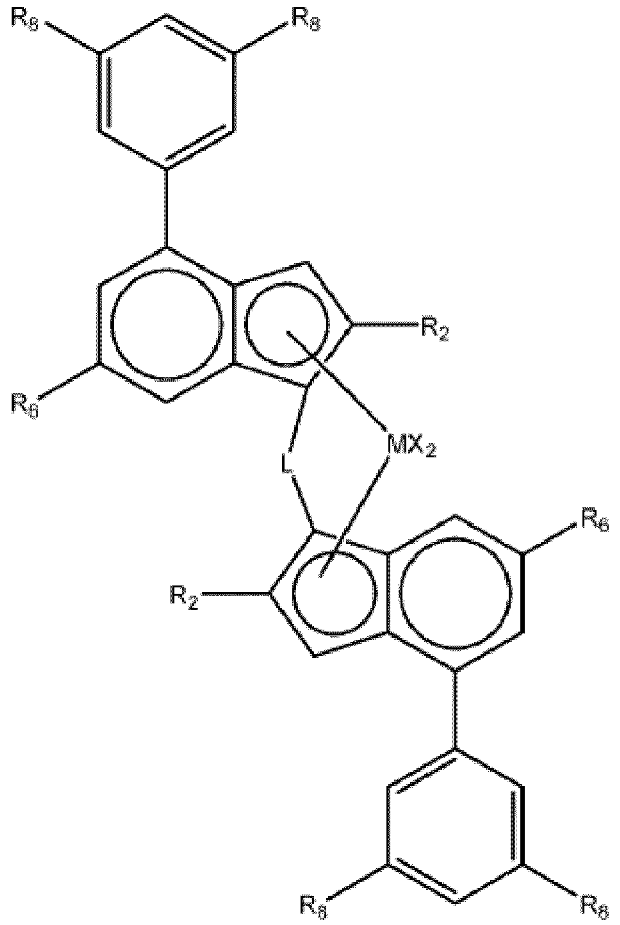

(III)

dans lequel

M est un zirconium ou un hafnium ;
chaque X est un ligand sigma, de préférence chaque X est indépendamment un atome d'hydrogène, un atome d'halogène, un groupe alcoxy en $C_1$ à $C_6$, un groupe alkyle en $C_1$ à $C_6$, un phényle ou un groupe benzyle ;
L est un pont divalent sélectionné parmi $-R'_2C-$, $-R'_2C-CR'_2-$, $-R'_2Si-$, $-R'_2Si-SiR'_2-$, $-R'_2Ge-$, dans lesquels chaque R' est indépendamment un atome d'hydrogène, un alkyle en $C_1$ à $C_{20}$, un tri(alkyle en $C_1$ à $C_{20}$)silyle, un aryle en $C_6$ à $C_{20}$, un arylalkyle en $C_7$ à $C_{20}$ ou un alkylaryle en $C_7$ à $C_{20}$ ; de préférence un diméthylsilyle, un méthylène ou un éthylène ;
$R_2$ est un groupe alkyle en $C_1$ à $C_{10}$ ;
$R_6$ est un groupe alkyle en $C_1$ à $C_{10}$ à condition que $R_6$ ne représente pas un groupe contenant un atome de carbone quaternaire directement lié au cycle indényle ;
et chaque $R^8$ est un groupe hydrocarbyle en $C_1$ à $C_{20}$.

**5.** Catalyseur selon n'importe quelle revendication précédente comprenant un complexe symétrique de formule (IV) :

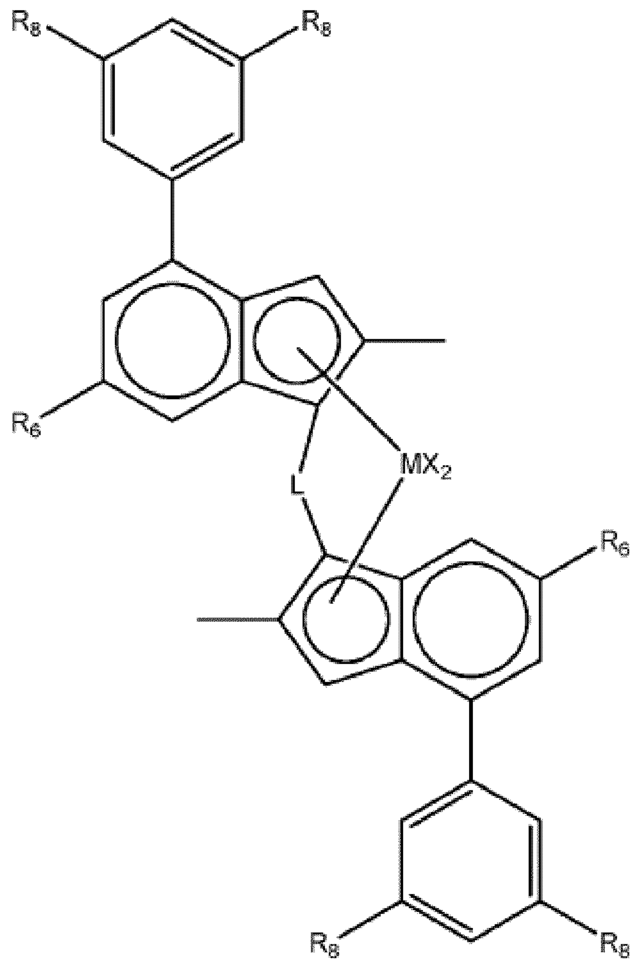

(IV)

dans lequel

M est un zirconium ou un hafnium ;
chaque X est un ligand sigma, de préférence chaque X est indépendamment un atome d'hydrogène, un atome d'halogène, un groupe alcoxy en $C_1$ à $C_6$, un alkyle en $C_1$ à $C_6$, un groupe phényle ou benzyle ;
L est un pont divalent sélectionné parmi $-R'_2C-$, $-R'_2C-CR'_2-$, $-R'_2Si-$, $-R'_2Si-SiR'_2-$, $-R'_2Ge-$, dans lesquels chaque R' est indépendamment un atome d'hydrogène, un hydrocarbyle en $C_1$ à $C_{20}$, un tri(alkyle en $C_1$ à $C_{20}$)silyle, un aryle en $C_6$ à $C_{20}$, un arylalkyle en $C_7$ à $C_{20}$ ou un alkylaryle en $C_7$ à $C_{20}$ ; de préférence un diméthylsilyle ;
$R_6$ est un groupe alkyle en $C_1$ à $C_{10}$ linéaire, un groupe -CH(alkyle en $C_1$ à $C_4$)$_2$ ou un groupe $CH_2$(alkyle en $C_1$ à $C_9$), où lesdits groupes alkyle en $C_1$ à $C_4$ et alkyle en $C_1$ à $C_9$ peuvent être linéaires ou ramifiés ; et
chaque $R^8$ est un groupe alkyle en $C_1$ à $C_{10}$.

**6.** Catalyseur selon n'importe quelle revendication précédente comprenant un complexe symétrique de formule (V)

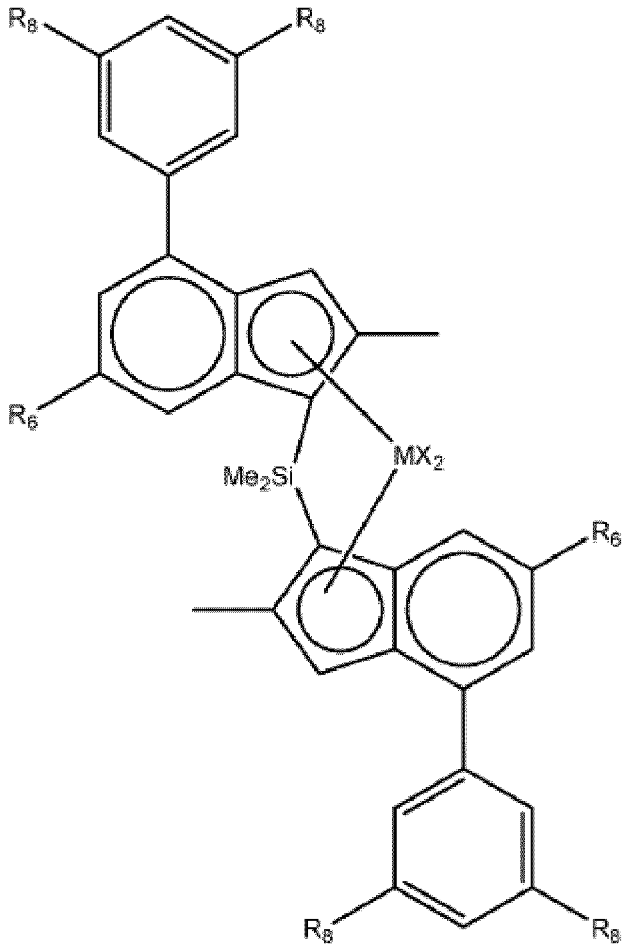

(V)

dans lequel M est un Zr/Hf ;

X est un atome d'hydrogène, un atome d'halogène, un groupe alcoxy en $C_1$ à $C_6$, un alkyle en $C_1$ à $C_6$, un phényle ou un groupe benzyle ;

$R_6$ est un alkyle en $C_1$ à $C_6$ linéaire, un -CH(alkyle en $C_1$ à $C_4$)$_2$ ou un -CH$_2$(alkyle en $C_1$ à $C_6$), où lesdits groupes alkyle en $C_1$ à $C_4$ et alkyle en $C_1$ à $C_6$ peuvent être linéaires ou ramifiés ; et

$R_8$ est un alkyle en $C_3$ à $C_8$, tel qu'un alkyle en $C_3$ à $C_8$ ramifié.

**7.** Catalyseur selon n'importe quelle revendication précédente comprenant un complexe symétrique de formule (VI)

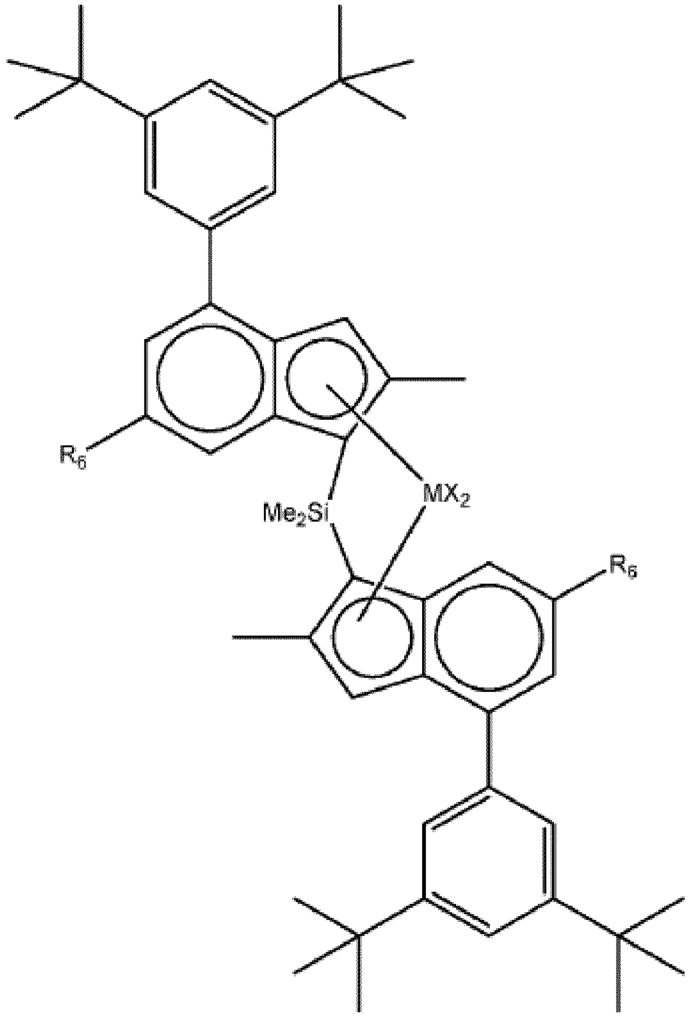

(VI)

dans lequel M est un Zr/Hf ;
X est un atome d'hydrogène, un atome d'halogène, un groupe alcoxy en $C_1$ à $C_6$, un alkyle en $C_1$ à $C_6$, un groupe phényle ou benzyle ;
$R_6$ est un alkyle en $C_1$ à $C_6$ linéaire, un -CH(alkyle en $C_1$ à $C_4$)$_2$ ou un -$CH_2$(alkyle en $C_1$ à $C_6$), où lesdits groupes alkyle en $C_1$ à $C_4$ et alkyle en $C_1$ à $C_6$ peuvent être linéaires ou ramifiés.

**8.** Catalyseur selon n'importe quelle revendication précédente comprenant le complexe dichlorure de *Rac*-diméthylsilanediylbis[2,6-diméthyl-4-(3,5-di-*tert*-butylphényl)-indèn-1-yl]zirconium dichlorure de *Rac*-diméthylsilanediylbis[2-méthyl-4-(3,5-di-*tert*-butylphényl)-6-éthyl-indèn-1-yl]zirconium dichlorure de *Rac*-diméthylsilane-diylbis[2-méthyl-4-(3,5-di-*tert*-butylphényl)-6-isopropyl-indèn-1-yl]zirconium dichlorure de *Rac*-diméthylsilanediylbis[2-méthyl-4-(3,5-di-*tert*-butylphényl)-6-néopentyl-indèn-1-yl]zirconium
ou leurs analogues avec Hf.

**9.** Catalyseur selon n'importe quelle revendication précédente dans lequel ledit cocatalyseur est une MAO.

**10.** Catalyseur selon n'importe quelle revendication précédente dans lequel les deux ligands formant le complexe sont identiques.

**11.** Complexe selon l'une quelconque des revendications 3 à 10 qui est un complexe symétrique de formule (II)

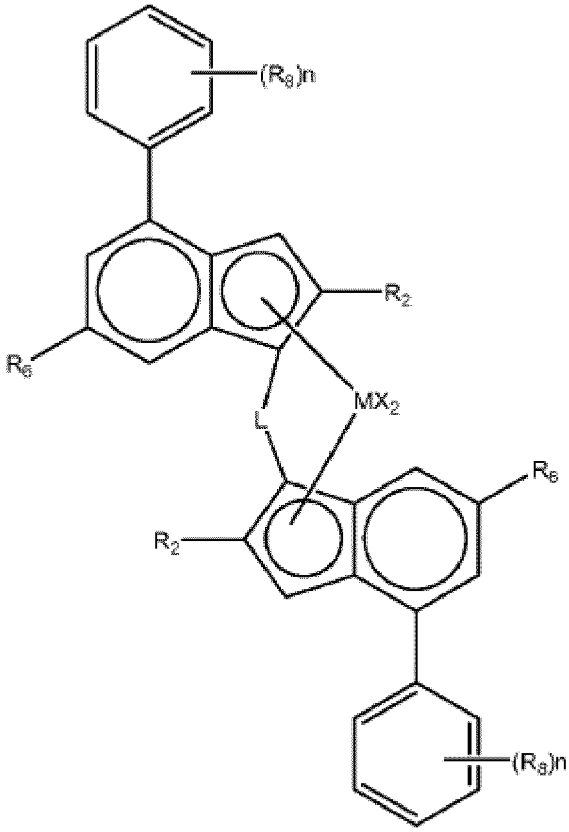

(II)

dans lequel

M est un zirconium ou un hafnium ;
chaque X est un ligand sigma ;
L est un pont divalent sélectionné parmi -R'$_2$C-, -R'$_2$C-CR'$_2$-, -R'$_2$Si-, -R'$_2$Si-SiR'$_2$-, -R'$_2$Ge-, dans lesquels chaque R' est indépendamment un atome d'hydrogène, un alkyle en $C_1$ à $C_{20}$, un tri(alkyle en $C_1$ à $C_{20}$)silyle, un aryle en $C_6$ à $C_{20}$, un arylalkyle en $C_7$ à $C_{20}$ ou un alkylaryle en $C_7$ à $C_{20}$ ;
$R_2$ est un radical hydrocarbyle en $C_1$ à $C_{20}$ ;
$R_6$ est un groupe hydrocarbyle en $C_1$ à $C_{20}$ aliphatique linéaire ou ramifié, $SR_9$ ou $OR_9$ ;
à condition que $R_6$ ne représente pas un groupe contenant un atome de carbone quaternaire directement lié au cycle indényle ;
$R_9$ est un groupe hydrocarbyle en $C_1$ à $C_{20}$ ;
n est indépendamment 1, 2 ou 3 ; et
chaque $R_8$ est un groupe hydrocarbyle en $C_1$ à $C_{20}$.

**12.** Procédé pour la préparation d'un catalyseur selon l'une quelconque des revendications 1 à 10 comprenant l'obtention d'un (i) complexe de formule (I) et (ii) d'un cocatalyseur comprenant un composé d'un métal du groupe 13, par exemple Al ou bore ;
la formation d'un système d'émulsion liquide/liquide, qui comprend une solution des composants (i) et (ii) du catalyseur dispersés dans un solvant, et la solidification desdites gouttelettes dispersées pour former des particules solides.

**13.** Procédé pour la polymérisation du propylène comprenant la polymérisation du propylène avec un catalyseur selon l'une quelconque des revendications 1 à 10.

**14.** Procédé selon la revendication 13 dans lequel ledit procédé forme un polypropylène isotactique.

**15.** Procédé selon la revendication 14 dans lequel l'activité du catalyseur dans ledit procédé est au moins de 10,0 kg/g(cat)/h.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 537686 A
- EP 1070729 A
- US 7405261 B
- WO 2009054831 A
- WO 03051934 A
- WO 0202576 A
- WO 0202575 A
- EP 2532687 A
- WO 2006069733 A
- EP 12199251 A


### Non-patent literature cited in the description


- *Deng Macromolecules,* 1996, vol. 29, 6371
- *Spaleck Organometallics,* 1994, vol. 13, 954
- **J. EWEN et al.** *Macromol. Rapid Commun.,* 1998, vol. 19, 71
- **A. ENDERS ; G. MAAS.** *Chemie in unserer Zeit,* 2000, vol. 34 (6
- **PIERANDREA LO NOSTRO.** Advances in Colloid and Interface Science. Elsevier Science, 1995, vol. 56, 245-287
- **HINTERMANN, L.** *Beilstein J. Org. Chem.,* 2007, vol. 3, 1
- **MATSUBARA, K. ; UENO, K. ; SHIBATA, Y.** *Organometallics,* 2006, vol. 25, 3422
- **IZMER, V.V. ; LEBEDEV, A.Y. ; NIKULIN, M.V. ; RYABOV, A.N. ; ASACHENKO, A.F. ; LYGIN, A.V. ; SOROKIN, D.A. ; VOSKOBOYNIKOV, A.Z.** *Organometallics,* 2006, vol. 25, 1217
- **BUSICO, V. ; CIPULLO, R.** *Prog. Polym. Sci.,* 2001, vol. 26, 443
- **BUSICO, V. ; CIPULLO, R. ; MONACO, G. ; VACATELLO, M. ; SEGRE, A.L.** *Macromolecules,* 1997, vol. 30, 6251
- **ZHOU, Z. ; KUEMMERLE, R. ; QIU, X. ; REDWINE, D. ; CONG, R. ; TAHA, A. ; BAUGH, D. ; WINNIFORD, B.** *J. Mag. Reson.,* 2007, vol. 187, 225
- **BUSICO, V. ; CARBONNIERE, P. ; CIPULLO, R. ; PELLECCHIA, R. ; SEVERN, J. ; TALARICO, G.** *Macromol. Rapid Commun.,* 2007, vol. 28, 11289
- **RESCONI, L. ; CAVALLO, L. ; FAIT, A. ; PIEMONTESI, F.** *Chem. Rev.,* 2000, vol. 100, 1253